# EUROPEAN PATENT SPECIFICATION

(11) **EP 3 469 077 B1**
(45) Date of publication and mention of the grant of the patent: **13.05.2026**
(21) Application number: 17730618.0
(22) Date of filing: 31.05.2017
(51) Int. Cl.: C12N 9/22, C12N 15/10, C12N 15/82

(54) **USE OF CPF1 ENDONUCLEASE FOR PLANT GENOME MODIFICATIONS**
VERWENDUNG VON CPF1-ENDONUKLEASE FÜR PFLANZENGENOMMODIFIKATIONEN
UTILISATION DE L'ENDONUCLÉASE CPF1 POUR MODIFIER LE GÉNOME DES PLANTES

(30) Priority: 14.06.2016 US 201662349826 P; 07.03.2017 US 201762468013 P
(43) Date of publication of application: 17.04.2019
(73) Proprietor: Pioneer Hi-Bred International, Inc., Johnston, Iowa 50131-1014 (US)
(72) Inventor: CIGAN, Andrew Mark, Madison Wisconsin 53704 (US); DJUKANOVIC, Vesna, Johnston Iowa 50131-0552 (US); YOUNG, Joshua K., Johnston Iowa 50131-0552 (US)
(74) Representative: J A Kemp LLP
(86) International application number: PCT/US2017/035175
(87) International publication number: WO 2017/218185

(56) References cited:
- BERND ZETSCHE ET AL: "Cpf1 Is a Single RNA-Guided Endonuclease of a Class 2 CRISPR-Cas System", CELL, vol. 163, no. 3, 1 October 2015 (2015-10-01), US, pages 759 - 771, XP055267511, ISSN: 0092-8674, DOI: 10.1016/j.cell.2015.09.038
- INES FONFARA ET AL: "The CRISPR-associated DNA-cleaving enzyme Cpf1 also processes precursor CRISPR RNA", NATURE, vol. 532, 20 April 2016 (2016-04-20), pages 517 - 521, XP055349049, ISSN: 0028-0836, DOI: 10.1038/nature17945
- HYERAN KIM ET AL: "Targeted Genome Editing for Crop Improvement", PLANT BREEDING AND BIOTECHNOLOGY, vol. 3, no. 4, 30 December 2015 (2015-12-30), pages 283 - 290, XP055397678, ISSN: 2287-9358, DOI: 10.9787/PBB.2015.3.4.283
- ROBERT D. FAGERLUND ET AL: "The Cpf1 CRISPR-Cas protein expands genome-editing tools", GENOME BIOLOGY, vol. 523, no. 1, 17 December 2015 (2015-12-17), GB, pages 481, XP055271439, ISSN: 1465-6906, DOI: 10.1186/s13059-015-0824-9
- ANONYMOUS: "Using Cpf1 for CRISPR . Benchling", 1 January 2015 (2015-01-01), XP055396832, Retrieved from the Internet <URL:https://benchling.com/pub/cpf1> [retrieved on 20170808]

## Description

### FIELD

The disclosure relates to the field of plant molecular biology, in particular to compositions of guide polynucleotide/Cpf1 endonuclease systems and compositions and methods for altering the genome of a plant cell.

### REFERENCE TO SEQUENCE LISTING SUBMITTED ELECTRONICALLY

The official copy of the sequence listing is submitted electronically via EFS-Web as an ASCII formatted sequence listing with a file named 20170518_7128PCT_ST25.txt created on May 18, 2017 and having a size of 94 kilobytes and is filed concurrently with the specification. The sequence listing contained in this ASCII formatted document is part of the specification and is herein incorporated by reference in its entirety.

### BACKGROUND

Recombinant DNA technology has made it possible to insert DNA sequences at targeted genomic locations and/or modify specific endogenous chromosomal sequences. Site-specific integration techniques, which employ site-specific recombination systems, as well as other types of recombination technologies, have been used to generate targeted insertions of genes of interest in a variety of organism. Genome-editing techniques such as designer zinc finger nucleases (ZFNs) or transcription activator-like effector nucleases (TALENs), or homing meganucleases, are available for producing targeted genome perturbations, but these systems tends to have a low specificity and employ designed nucleases that need to be redesigned for each target site, which renders them costly and time-consuming to prepare.

Although several approaches have been developed to target a specific site for modification in the genome of a plant, there still remains a need for more effective genome engineering technologies that are affordable, easy to set up, scalable, and amenable to targeting multiple positions within the plant genome.

### SUMMARY OF THE INVENTION

The invention provides a method for modifying a target sequence in the genome of a plant cell, the method comprising:
a) introducing into a plant cell a Cpf1 endonuclease protein or a plant-optimized polynucleotide encoding said Cpf1 endonuclease protein, and a guide polynucleotide comprising a variable targeting domain that is substantially complementary to a target sequence in the plant genome or a recombinant DNA expressing said guide polynucleotide; and
b) incubating said plant cell at a temperature of 28°C to 37°C for a period of at least about 4 hrs.;

wherein said guide polynucleotide and Cpf1 endonuclease are capable of forming a complex that can recognize, bind to, and optionally nick or cleave said target sequence;
wherein said method does not comprise an essentially biological process for the production of plants.

The invention further provides a method for editing a nucleotide sequence in the genome of a plant cell, the method comprising;
a) introducing into a plant cell a Cpf1 endonuclease protein or a plant-optimized polynucleotide encoding said Cpf1 endonuclease protein, a guide polynucleotide comprising a variable targeting domain that is substantially complementary to a target sequence in the plant genome, and a polynucleotide modification template,
   wherein said polynucleotide modification template comprises at least one nucleotide modification of said nucleotide sequence; and,
b) incubating said plant cell at a temperature of 28°C to 37°C for a period of at least about 4 hrs.;

wherein said guide polynucleotide and Cpf1 endonuclease protein are capable of forming a complex that can recognize, bind to, and optionally nick or cleave all or part of said target sequence;
wherein said method does not comprise an essentially biological process for the production of plants.

Yet further provided by the invention is a method of simultaneously modifying multiple target sequences in the genome of a plant cell, the method comprising:
a) introducing into said plant cell a Cpf1 endonuclease protein, or a plant-optimized polynucleotide encoding said Cpf1 endonuclease protein, and a precursor guide RNA transcriptional initiation cassette capable of expressing a single precursor RNA that is processed into a multitude of single guide RNAs, wherein each single guide RNA comprises a variable targeting domain 3' of a Protospacer Adjacent Motif (PAM), wherein said variable targeting domain is complementary to a single target sequence in the plant genome; and
b) incubating the plant cell of (a) at a temperature of 28°C to 37°C for a period of at least about 4 hrs.;

wherein each of said single guide RNA and said Cpf1 endonuclease protein is capable of forming a ribonucleotide complex that can recognize, bind to, and optionally nick or cleave a target sequence;
wherein said method does not comprise an essentially biological process for the production of plants.

The invention further provides a method for modifying a DNA target sequence in the genome of a plant cell, the method comprising:
a) introducing into a plant cell a Cpf1 endonuclease protein or a plant-optimized polynucleotide encoding said Cpf1 endonuclease protein, and a guide polynucleotide or a recombinant DNA expressing said guide polynucleotide, capable of forming a Cpf1 complex with the Cpf1 endonuclease protein, the guide polynucleotide comprising a variable targeting domain that is substantially complementary to a target sequence in the plant genome; and
b) introducing a polynucleotide modification template comprising at least one region that corresponds to a DNA target sequence adjacent to a PAM sequence recognized by the Cpf1 complex,
wherein said method further comprises incubating said plant cell at a temperature of 28°C to 37°C for a period of at least about 4 hrs.;
further wherein said Cpf1 complex can recognize, bind to, and optionally nick or cleave said target sequence;
wherein said method does not comprise an essentially biological process for the production of plants.

Further aspects and preferred embodiments are defined in the dependent claims. Any aspects, embodiments and examples of the present disclosure which do not fall under the scope of the appended claims do not form part of the invention and are merely provided for illustrative purposes.

### BRIEF SUMMARY OF TECHNICAL TEACHINGS

Compositions and methods are disclosed for guide polynucleotide/Cpf1 endonuclease systems and elements comprising such systems, including, but not limited to, guide polynucleotide/Cpf1 endonuclease complexes, guide polynucleotides, polynucleotide modification templates, Cpf1 endonuclease proteins, recombinant DNA constructs comprising plant-optimized Cpf1 endonuclease genes, and combinations thereof. Compositions and methods are also disclosed for use of Cpf1 endonuclease systems in plant genome modifications.

In one aspect of the disclosure, a Cpf1 endonuclease is used in a plant cell or plant, where the Cpf1 endonuclease is capable of forming a complex with a guide polynucleotide, where the complex can recognize, bind to, and optionally nick or cleave a target sequence in the plant cell or plant.

In one aspect of the disclosure, the method comprises a method for modifying a target sequence in the genome of a plant cell, the method comprising: a) introducing into a plant cell a Cpf1 endonuclease or a plant-optimized polynucleotide encoding the Cpf1 endonuclease, and a guide polynucleotide comprising a variable targeting domain that is substantially complementary to a target sequence in the plant genome or a recombinant DNA expressing the guide polynucleotide; and, b) incubating the plant cell at a temperature greater than 28°C for a period of at least about 4 hrs., where the guide polynucleotide and Cpf1 endonuclease are capable of forming a complex that can recognize, bind to, and optionally nick or cleave the target sequence.

In one aspect of the disclosure, the method comprises a method for modifying a target sequence in the genome of a plant cell, the method comprising: a) introducing a guide polynucleotide comprising a variable targeting domain that is substantially complementary to a target sequence in the plant genome or a recombinant DNA expressing the guide polynucleotide, into a plant cell comprising in its genome a recombinant DNA construct comprising a promoter operably linked to a plant-optimized polynucleotide encoding a Cpf1 endonuclease; and, b) incubating the plant cell at a temperature greater than 28°C for a period of at least about 4 hrs., where the guide polynucleotide and Cpf1 endonuclease are capable of forming a complex that can recognize, bind to, and optionally nick or cleave the target sequence.

In one aspect of the disclosure, the method comprises a method for modifying a target sequence in the genome of a plant or plant cell, the method comprising: a) obtaining a plant or plant cell comprising in its genome a first recombinant DNA construct, the first recombinant DNA construct comprising a promoter operably linked to plant-optimized polynucleotide encoding a Cpf1 endonuclease, and a second recombinant DNA construct, the second recombinant DNA construct comprising a promoter operably linked to a nucleotide sequence encoding a guide RNA, where the guide RNA and Cpf1 endonuclease are capable of forming a complex that can recognize, bind to, and optionally nick or cleave the target sequence; and, b) incubating the plant or plant cell at a temperature greater than 28°C for a period of at least about 4 hrs., where the guide RNA and Cpf1 endonuclease are capable of forming a complex that can recognize, bind to, and optionally nick or cleave the target sequence.

The method can further comprise identifying at least one plant cell, plant or progeny plant that has a modification at the target sequence, where the modification at the target sequence is selected from the group consisting of (i) a replacement of at least one nucleotide, (ii) a deletion of at least one nucleotide, (iii) an insertion of at least one nucleotide, and (iv) any combination of (i) - (iii). The method can further provide a donor DNA to the plant cell, where the donor DNA comprises a polynucleotide of interest. This can produce a plant cell or plant having a detectable targeted genome modification.

In one aspect of the disclosure, the method comprises a method for editing a nucleotide sequence in the genome of a plant cell, the method comprising: a) introducing into a plant cell a Cpf1 endonuclease or a plant-optimized polynucleotide encoding the Cpf1 endonuclease, a guide polynucleotide comprising a variable targeting domain that is substantially complementary to a target sequence in the plant genome or a recombinant DNA expressing the guide polynucleotide, and a polynucleotide modification template, where the polynucleotide modification template comprises at least one nucleotide modification of the nucleotide sequence; and, b) incubating the plant cell at a temperature greater than 28°C for a period of at least about 4 hrs., where the guide polynucleotide and Cpf1 endonuclease are capable of forming a complex that can recognize, bind to, and optionally nick or cleave all or part of the target sequence.

The plant cell, plant or progeny plant can be incubated at a temperature greater than 28°C. Exposure of the plant cell to the temperature greater than 28°C can be at any developmental stage of the plant cell, plant embryo and/or plant. Progeny of plants comprising a Cpf1 endonuclease (or a recombinant construct expressing the Cpf1 endonuclease) and a guide RNA (and/or a recombinant construct expressing the guide RNA) can also be exposed a temperature greater than 28°C, thereby activating the guide RNA/ Cpf1 complex within the progeny plant (at a desired stage during development) which allows for the progeny plant to be modified at a specific target site that is recognized by the guide RNA/Cpf1 endonuclease complex.

In one aspect of the disclosure, the recombinant DNA construct is a recombinant DNA construct comprising a promoter operably linked to a plant-optimized nucleotide sequence encoding a Cpf1 endonuclease.

In one aspect of the disclosure, the method comprises a method of simultaneously modifying multiple target sequences in the genome of a plant cell, the method comprising : a) introducing into the plant cell a Cpf1 endonuclease protein, or a plant-optimized polynucleotide encoding the Cpf1 endonuclease, and a precursor guide RNA transcriptional cassette capable of expressing a single precursor RNA that is processed into a multitude of single guide RNAs, where each single guide RNA comprises a variable targeting domain 3' of a Protospacer Adjacent Motif (PAM) that is substantially complementary to a single target sequence in the plant genome; and, b) incubating the plant cell of (a) at a temperature greater than 28°C for a period of at least about 4 hrs., where each of single guide RNA and the Cpf1 endonuclease protein is capable of forming a ribonucleotide complex that can recognize, bind to, and optionally nick or cleave a target sequence. The multiple target sequences can consist of at least 2, 3, 4, 5, 6, 7, 8, 9, 10, 11, 12, 13, 14, 15, 16, 17, 18, 19, 20 target sequences up to 100 target sequences.

In one aspect of the disclosure, the kit is a kit for binding, cleaving or nicking a target sequence in a plant cell or plant, the kit comprising a guide DNA specific for the target sequence, and plant-optimized nucleotide sequence encoding a Cpf1 endonuclease, where the guide DNA is capable of forming a guide DNA/Cpf1 endonuclease complex, where the complex can recognize, bind to, and optionally nick or cleave the target sequence.

In one aspect of the disclosure, the method is a method for modifying a DNA target sequence in the genome of a plant cell, the method comprising: a) introducing into a plant cell a Cpf1 endonuclease, and a guide polynucleotide capable of forming a complex with the Cpf1 endonuclease, the guide polynucleotide comprising a variable targeting domain that is substantially complementary to a target sequence in the plant genome; and b)introducing a polynucleotide modification template comprising at least one region that corresponds to a DNA target sequence adjacent to a PAM sequence recognized by the Cpf1 complex, where the at least one region that corresponds to a DNA target sequence comprises at least one nucleotide mismatch compared to the DNA target sequence in a position from +1 to +19, 3' to the PAM sequence. The method can further comprise incubating the plant cell at a temperature greater than 28°C for a period of at least about 4 hrs, and/or where the Cpf1 complex can recognize, bind to, and optionally nick or cleave the target sequence.

In one aspect of the disclosure, the polynucleotide modification template is a polynucleotide modification template comprising at least one region that is complementary to a DNA target sequence adjacent to a PAM sequence recognized by a Cpf1 complex, where the at least one region that corresponds to a DNA target sequence comprises at least one nucleotide mismatch compared to the DNA target sequence in a position from +1 to +19, 3' to the PAM sequence. The polynucleotide modification template can further comprise a modification in a region that corresponds to the target DNA sequence in a position from +20 to +24, 3' to the PAM sequence.

Also disclosed are nucleic acid constructs, plants, plant cells, explants, seeds and grain having a modified target sequence or having a modification at a nucleotide sequence in the genome of the plant, produced by the methods described herein. Additional aspects of the methods and compositions of the present disclosure are shown herein.

### BRIEF DESCRIPTION OF THE DRAWINGS AND THE SEQUENCE LISTING

The disclosure can be more fully understood from the following detailed description and the accompanying drawings and Sequence Listing, which form a part of this application. The sequence descriptions and sequence listing attached hereto comply with the rules governing nucleotide and amino acid sequence disclosures in patent applications as set forth in 37 C.F.R. §§1.821-1.825. The sequence descriptions contain the three letter codes for amino acids as defined in 37 C.F.R. §§ 1.821-1.825.

### Figures

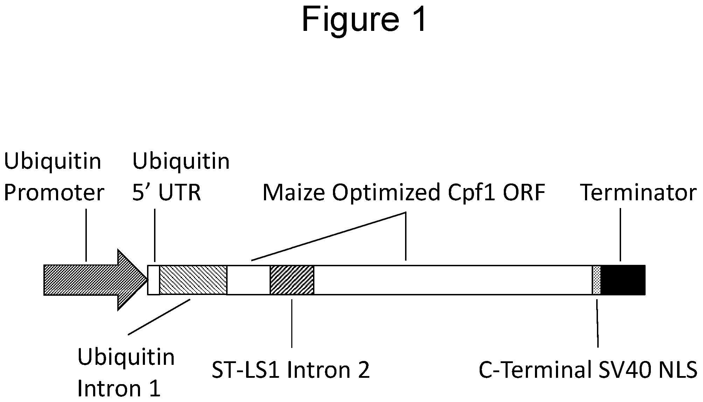

Figure 1 depicts an expression cassette comprising a maize optimized Cpf1 endonuclease gene (Maize Optimized Cpf1 ORF) encoding a Cpf1 endonuclease protein. Components include a promoter (such as for example, a Ubiquitin Promoter) operably linked to a Ubiquitin 5 prime untranslated region (5'UTR), Ubiquitin intron 1 and a maize codon optimized Cpf1 open-reading frame (ORF), ST-LS1 intron-2, 3 prime or C-terminal SV40 nuclear localization signal (NLS), and terminator.

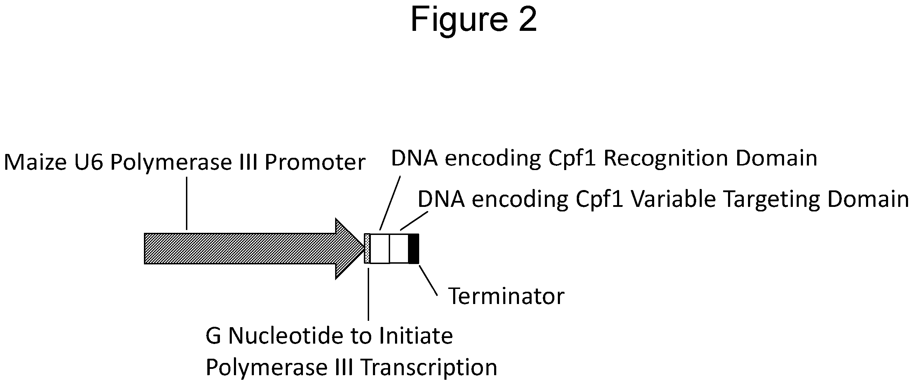

Figure 2 depicts an expression cassette comprising a maize small RNA transcription initiation sequence (Maize U6 Polymerase III Promoter) operably linked to a DNA sequence encoding a guide polynucleotide (comprising a Cpf1 recognition domain and a Cpf1 variable targeting domain) that can form a functional complex with a Cpf1 endonuclease capable of binding, nicking or cleaving a single stranded or double stranded target site. Components include a maize U6 Polymerase III Promoter operably linked to a G nucleotide to initiate transcription, a 5 prime about 20 nucleotide (nt) region encoding a sequence that can be recognized by Cpf1 (Cpf1 recognition domain), an about 23 nt sequence that guides Cpf1 cleavage activity (Cpf1 variable targeting domain), and a terminator.

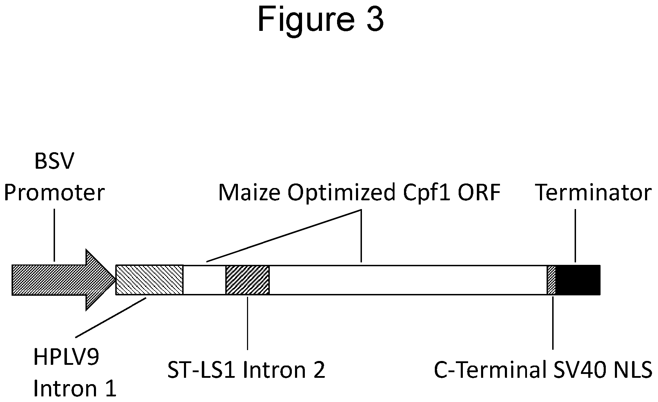

Figure 3 depicts an expression cassette comprising a BSV Promoter operably linked to a DNA sequence encoding a maize optimized Cpf1 endonuclease and a terminator. Components include a BSV promoter, HPLV9 Intron 1, a Maize Optimized Cpf1 Open Reading Frame (ORF), a ST-LS1 intron 2, a C-terminal SV40 nuclear localization signal (NLS) and a terminator.

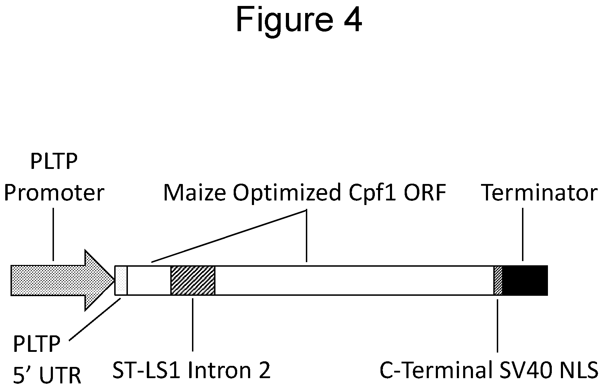

Figure 4 depicts an expression cassette comprising a PLTP Promoter operably linked to a DNA sequence encoding a maize optimized Cpf1 endonuclease and a terminator. Components include a PLTP promoter, PLTP 5 prime UTR, a ST-LS1 intron-2, a Maize Optimized Cpf1 Open Reading Frame (ORF), a C-terminal SV40 nuclear localization signal (NLS) and a terminator.

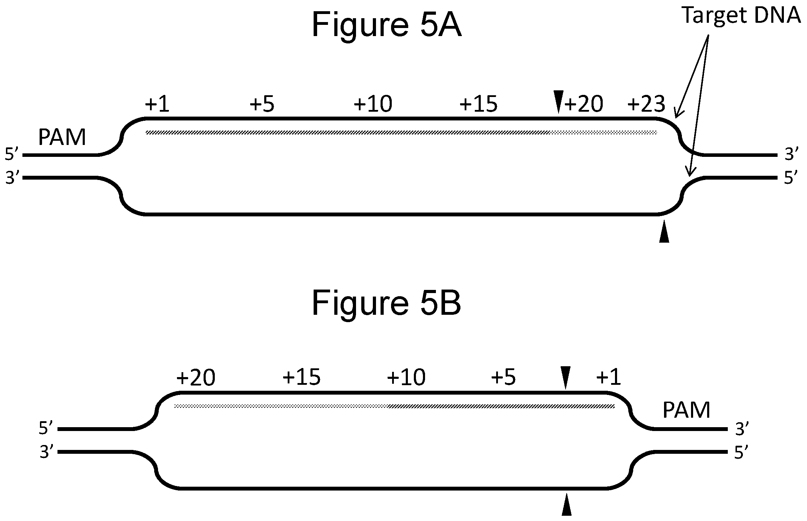

Figures 5A-5B illustrates a DNA target site that can be cleaved by a Cpf1 endonuclease (Figure 5A) or a Cas endonuclease (Figure 5B). Figure 5A illustrates a DNA target site for a Cpf1 endonuclease. Sense (5' to 3' top strand) and anti-sense (5' to 3' bottom strand) DNA strands are shown in black and the regions of the Cpf1 DNA target site are indicated, including the PAM (protospacer adjacent motif) and the DNA target site region (+1 to +23) that is base paired with the variable targeting domain of the Cpf1 guide RNA (dark and light gray lines). The region of the Cpf1 guide RNA that exhibits low tolerance for mismatches is depicted by a dark gray line while the light gray line shows the region with high tolerance for mismatches (positions +20 to +23, 3 prime of the PAM). The Cpf1 DNA cut sites are indicated with triangles. Figure 5B illustrates a DNA target site for a Cas9 endonuclease. Sense (5' to 3' top strand) and anti-sense (5' to 3' bottom strand) DNA strands are shown in black and the regions of the Cas9 DNA target site are indicated, including the PAM (protospacer adjacent motif) and the DNA target site region (+1 to +20) that is base paired with the variable targeting domain of the Cas9 guide RNA (dark and light gray lines). The region of the Cas9 guide RNA that exhibits low tolerance for mismatches is depicted by a dark gray line while the light gray line shows the region with a higher tolerance for mismatches (positions ~+10 to +20, 5 prime of the PAM). The Cas9 DNA cut sites are indicated with triangles.

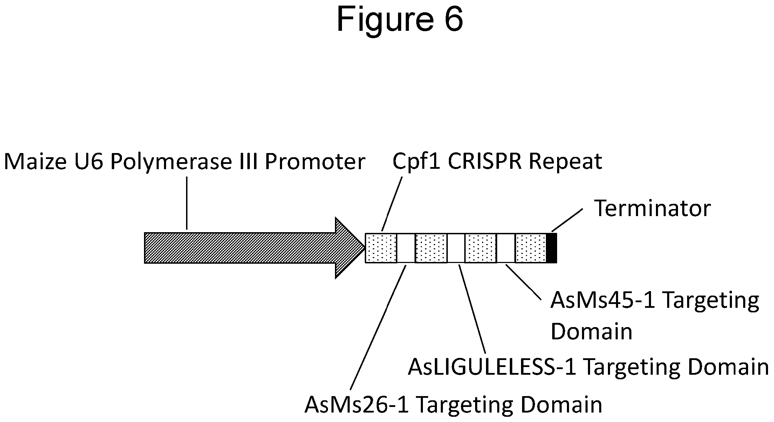

Figure 6 depicts an expression cassette comprising a maize small RNA transcription initiation sequence (Maize U6 Polymerase III Promoter) operably linked to a DNA sequence encoding a guide polynucleotide that can form a functional complex with a Cpf1 endonuclease capable of binding, nicking or cleaving a single stranded or double stranded target site. Components include a maize U6 Polymerase III Promoter, Cpf1 CRISPR repeats, a AsMs26-1 targeting domain, a AsLIGULELESS-1 targeting domain, a AsMs45-1 targeting domain, and a terminator.

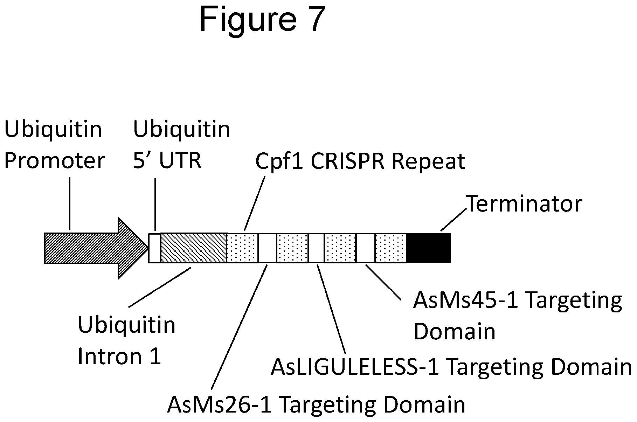

Figure 7 depicts an expression cassette comprising a Pol-II promoter (Ubiquitin Promoter) operably linked to a DNA sequence encoding a guide polynucleotide that can form a functional complex with a Cpf1 endonuclease capable of binding, nicking or cleaving a single stranded or double stranded target site. Components include a Ubiquitin Pol-II Promoter, a ubiquitin 5'UTR, a ubiquitin Intron -1, Cpf1 CRISPR repeats, a AsMs26-1 targeting domain, a AsLIGULELESS-1 targeting domain, a AsMs45-1 targeting domain, and a terminator.

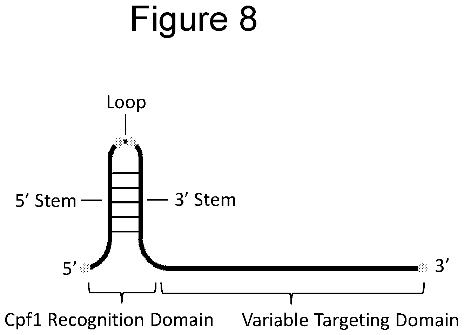

Figure 8 depicts one example of a hybrid DNA-RNA guide polynucleotide (hDRNA) comprising at least one DNA nucleotide (grey dot) in the Cpf1 recognition domain and/or at least one DNA nucleotide (grey dot) in the variable targeting domain.

### Sequences

**Table 1. Summary of Nucleic Acid and Protein SEQ ID Numbers**

| Description | Nucleic acid SEQ ID NO. | Protein SEQ ID NO. |
|---|---|---|
| CpfI endonuclease gene from *Acidaminococcus* sp. BV3L6 | 1 | |
| potato ST-LS1 intron | 2 | |
| *Simian virus 40* (SV40) monopartite nuclear localization signal | | 3 |
| Maize optimized CpfI endonuclease gene with Intron and NLS | 4 | |
| 5 prime region of Cpf1 guide RNA (Cpf1 recognition domain) | 5 | |
| U6 polymerase III promoter of maize | 6 | |
| AsMS26-1 (maize target site including 5' PAM sequence) | 7 | |
| AsLIGULELESS-1 (maize target site including 5' PAM sequence) | 8 | |
| AsMS45-1 (maize target site including 5' PAM sequence) | 9 | |
| gi\|545612232\|ref\|WP_021736722.1\| type V CRISPR-associated protein Cpf1 [Acidaminococcus sp. BV3L6] | | 10 |
| maize optimized Cpf1 endonuclease with 3 prime NLS; | | 11 |
| maize Ubiquitin Promoter | 12 | |
| maize Ubiquitin 5 Prime UTR | 13 | |
| maize Ubiquitin Intron 1 | 14 | |
| AsCpf1 Maize Optimized Guide RNA | 15 | |
| Guide RNA for AsMS26-1 | 16 | |
| Guide RNA for AsLIGULELESS-1 | 17 | |
| Guide RNA for AsMS45-1 | 18 | |
| Banana Streak Virus Promoter (BSV promoter) | 19 | |
| Phospholipid Transfer Protein Promoter (PLTP promoter) | 20 | |
| Maize HPLV9 Intron 1 | 21 | |
| 5 prime UTR from the Phospholipid Transfer Protein gene | 22 | |
| AsLIGULELESS1 Homology Directed DNA Repair Template 1 | 23 | |
| AsLIGULELESS1 Homology Directed DNA Repair Template 2 | 24 | |
| AsLIGULELESS1 Homology Directed DNA Repair Template 3 | 25 | |
| SpyLIGULELESS1 Maize Genomic Target Sequence | 26 | |
| AsCpf1 CRISPR Array Repeat | 27 | |
| Polymerase III Multiplex Transcriptional expression Cassette | 28 | |
| Polymerase II Multiplex Transcriptional expression Cassette | 29 | |
| CpfI Gene Homolog 1, Francisella novicida U112 | 30 | |
| pfI Gene Homolog 2, Lachnospiraceae bacterium ND2006 | 31 | |
| Translation of CpfI Gene Homolog 1, Francisella novicida U112 | | 32 |
| Translation of CpfI Gene Homolog 2, Lachnospiraceae bacterium ND2006 | | 33 |
| SpyMS26-1 Maize Genomic Target Sequence | 34 | |
| SpyMS45-1 Maize Genomic Target Sequence | 35 | |
| Maize Optimized *Fransincella novicida* U112 Cpf1 Gene with Intron and NLS | 36 | |
| Maize Optimized *Lachnospiraceae* bacterium ND2006 Cpf1 Gene with Intron and NLS | 37 | |
| Recognition Sequence for Cpf1 from *Fransincella novicida* U112 | 38 | |
| Recognition Sequence for Cpf1 from *Lachnospiraceae bacterium* ND2006 | 39 | |
| Synthesized *Fransincella novicida* (Fn) Cpf1 Maize Optimized Guide RNA | 40 | |
| Synthesized *Lachnospiraceae bacterium* (Lb) Cpf1 Maize Optimized Guide RNA | 41 | |
| Synthesized Fn Cpf1 Maize Optimized Guide RNA Containing AsMS26-1 Variable Targeting Domain | 42 | |
| Synthesized Lb Cpf1 Maize Optimized Guide RNA Containing AsMS26-1 Variable Targeting Domain | 43 | |
| hybrid DNA-RNA guide polynucleotide 1 | 44 | |
| hybrid DNA-RNA guide polynucleotide 2 | 45 | |
| hybrid DNA-RNA guide polynucleotide 3 | 46 | |
| hybrid DNA-RNA guide polynucleotide 4 | 47 | |
| T7 Transcribed Streptococcus pyogenes Cas9 Single Guide RNA | 48 | |
| SpyMS45-2 Maize Genomic Target Sequence | 49 | |
| Variable targeting domain | 50 | |

### DETAILED DESCRIPTION

The invention is defined in the appended claims. Any aspects, embodiments and examples of the present disclosure which do not fall under the scope of the appended claims do not form part of the invention and are merely provided for illustrative purposes.

Compositions and methods are disclosed for guide polynucleotide/Cpf1 endonuclease systems and elements comprising such system for use in plant genome modifications. Methods are disclosed for modifying a target nucleotide sequence in the genome of a plant cell or plant. The present disclosure also describes methods for editing a nucleotide sequence in the genome of a plant cell, for targeted mutagenesis, and for inserting a polynucleotide of interest into the genome of a plant cell.

The term "Cas gene" herein refers to one or more genes that are generally coupled, associated or close to, or in the vicinity of flanking CRISPR loci. The terms "Cas gene", "CRISPR-associated (Cas) gene" and "Clustered Regularly Interspaced Short Palindromic Repeats-associated gene" are used interchangeably herein.

CRISPR (clustered regularly interspaced short palindromic repeats) loci refers to certain genetic loci encoding components of DNA cleavage systems, for example, used by bacterial and archaeal cells to destroy foreign DNA (Horvath and Barrangou, 2010, Science 327:167-170; WO2007/025097, published March 1, 2007). A CRISPR locus can consist of a CRISPR array, comprising short direct repeats (CRISPR repeats) separated by short variable DNA sequences (called 'spacers'), which can be flanked by diverse Cas (CRISPR-associated) genes. The number of CRISPR-associated genes at a given CRISPR locus can vary between species. Multiple CRISPR/Cas systems have been described including Class 1 systems, with multisubunit effector complexes (comprising type I, type III and type IV subtypes), and Class 2 systems, with single protein effectors (comprising type II and type V subtypes, such as but not limiting to Cas9, Cpf1, C2c1, C2c2, C2c3). Class 1systems (Makarova et al. 2015, Nature Reviews; Microbiology Vol. 13:1-15; Zetsche et al., 2015, Cell 163, 1-13; Shmakov et al., 2015, Molecular_Cell 60, 1-13; Haft et al., 2005, Computational Biology, PLoS Comput Biol 1(6): e60. doi:10.1371 /journal .pcbi. 0010060 and WO 2013/176772 A1 published on November 23, 2013). The type II CRISPR/Cas system from bacteria employs a crRNA (CRISPR RNA) and tracrRNA (trans-activating CRISPR RNA) to guide the Cas endonuclease to its DNA target. The crRNA contains a spacer region complementary to one strand of the double strand DNA target and a region that base pairs with the tracrRNA (trans-activating CRISPR RNA) forming a RNA duplex that directs the Cas endonuclease to cleave the DNA target. Spacers are acquired through a not fully understood process involving Cas1 and Cas2 proteins. All type II CRISPR/Cas loci contain cas1 and cas2 genes in addition to the cas9 gene (Chylinski et al., 2013, RNA Biology 10:726-737; Makarova et al. 2015, Nature Reviews Microbiology Vol. 13:1-15). Type II CRISPR-Cas loci can encode a tracrRNA, which is partially complementary to the repeats within the respective CRISPR array, and can comprise other proteins such as Csn1 and Csn2. The presence of cas9 in the vicinity of Cas 1 and cas2 genes is the hallmark of type II loci (Makarova et al. 2015, Nature Reviews Microbiology Vol. 13:1-15).

The term "Cas protein" refers to a protein encoded by a Cas (CRISPR-associated) gene. A Cas protein includes a Cas9 protein, a Cpf1 protein, a C2c1 protein, a C2c2 protein, a C2c3 protein, Cas3, Cas3-HD, Cas 5, Cas7, Cas8, Cas10, or combinations or complexes of these (Makarova et al. 2015, Nature Reviews Microbiology Vol. 13:1-15).

Cas proteins include Cas endonucleases. Cas endonucleases, when in complex with a suitable polynucleotide component, are capable of recognizing, binding to, and optionally nicking or cleaving all or part of a specific DNA target sequence. A Cas endonuclease comprises one or more nuclease domains.

"Cas9" (formerly referred to as Cas5, Csn1, or Csx12) herein refers to a Cas endonuclease that when in complex with a suitable polynucleotide component (such as crNucleotide and a tracrNucleotide, or a single guide polynucleotide) is capable of recognizing, binding to, and optionally nicking or cleaving all or part of a DNA target sequence. A Cas9 protein comprises a RuvC nuclease domain and an HNH (H-N-H) nuclease domain, each of which can cleave a single DNA strand at a target sequence (the concerted action of both domains leads to DNA double-strand cleavage, whereas activity of one domain leads to a nick). In general, the RuvC domain comprises subdomains I, II and III, where domain I is located near the N-terminus of Cas9 and subdomains II and III are located in the middle of the protein, flanking the HNH domain (Hsu et al., 2013, Cell 157:1262-1278). Cas9 endonucleases are typically derived from a type II CRISPR system, which includes a DNA cleavage system utilizing a Cas9 endonuclease in complex with at least one polynucleotide component. For example, a Cas9 can be in complex with a CRISPR RNA (crRNA) and a trans-activating CRISPR RNA (tracrRNA). In another example, a Cas9 can be in complex with a single guide RNA (Makarova et al. 2015, Nature Reviews Microbiology Vol. 13:1-15).

"Cpf1" as used herein refers to a Cas endonuclease of a class 2- type V system, that comprises a RuvC-like nuclease domain and a putative second nuclease domain, that coordinate cleavage of a double-stranded DNA (Yamane et al., 2016, Cell 165:949-962). The absence of an obvious trans-acting RNA (tracrRNA) is another characteristic feature of a Cpf1 endonuclease system, as Cpf1 only requires a crNucleotide to form a polynucleotide guided endonuclease (PGEN) complex capable of cleaving a DNA target sequence. Cleavage of the target sequence by Cpf1 is distant from the PAM (about 19 base pairs removed on the crRNA-non complementary strands) and produces a double-strand break with an approximately 4 nucleotides 5' overhang. (Zetsche et al., 2015, Cell 163, 1-13; Fagerlund et al., 2015, Genome Biology 16:251-253). In one aspect, the Cpf1 endonuclease is an *Acidaminococcus* sp. BV3L6 Cpf1 endonuclease. (Zetsche et al., 2015, Cell 163, 1-13; Fagerlund et al., 2015, Genome Biology 16:251-253; Yamane et al., 2016, Cell 165:949-962)

The term "Cpf1 gene" herein refers to a gene that encodes a Cpf1 endonuclease.

Unexpectedly, as described herein, the Cpf1 endonuclease activity, when in complex with a guide polynucleotide (such as a guide polynucleotide/Cpf1 endonuclease complex) can be regulated by temperature. A temperature greater than 28°C resulted in increased Cpf1 cleavage activity compared to the activity at or below 28°C. This unexpected temperature sensitivity can allow for a regulated activation of the Cpf1 endonuclease and thus a regulated activity of a guide RNA/ Cpf1 endonuclease complex. Exposing the guide RNA/ Cpf1 endonuclease complex (or host cell comprising the complex) to an increased temperature allows for an increased frequency of target site mutagenesis indicative of an increased frequency of cleavage activity by the Cpf1 protein. Furthermore, it is described herein that this increased frequency in target site mutagenesis is independent from what promoter is used (Pol-II) to drive the Cpf1 expression.

The guide polynucleotide/Cpf1 endonuclease system and methods described herein may be used to target DNA in plant cells for cleavage and be utilized as a tool to bind, nick, or cleave plant genomic DNA (including chromosomal DNA, mitochondrial DNA, plastidial DNA) for the purposes of plant genomic and gene manipulation. Optimal activity of the Cpf1 endonuclease in plant cells can be obtained by incubating the guide polynucleotide/Cpf1 endonuclease complex (or the host cell comprising the guide polynucleotide/Cpf1 endonuclease complex) at temperatures greater than 28°C, such as but not limited to temperatures of at least 29°C, 30°C, 31°C, 32°C, 33°C, 34°C, 35°C, 36°C, 37°C, 38°C, 39°C, 40°C, 41°C, 42°C, 43°C, 44°C or 45°C up to 50°C or up to and including any temperature that results in the Cpf1 endonuclease being functional. Incubation time at these temperatures greater than 28°C can be for a period of at least about 10 min., 20 min., 30 min., 40 min., 50 min., 1 hr., 2 hrs., 3 hrs., 4 hrs., 5 hrs., 6 hrs., 7 hrs., 8 hrs., 9 hrs., 10 hrs., 11 hrs., 12 hrs., 13 hrs., 14 hrs., 15 hrs., 16 hrs., 17 hrs., 18 hrs., 19 hrs., 20 hrs., 21 hrs., 22 hrs., 23 hrs., 24 hrs., 25 hrs., 26 hrs., 27 hrs., 28 hrs., 29 hrs., 30 hrs., 31 hrs., 32 hrs., 33 hrs., 34 hrs., 35 hrs., 36 hrs., 37 hrs., 38 hrs., 39 hrs., 40 hrs., 41 hrs., 42 hrs., 43 hrs., 44 hrs., 45 hrs., 46 hrs., 47 hrs., 48 hrs. to at least 3, 4, 5, 6, 7 days, to at least 1, 2, 3, 4, 5 weeks, to at least 1, 2, 3 months, up to the entire life span of the organism.

Optimal activity of the Cpf1 endonuclease in plant cells can be obtained by incubating the guide polynucleotide/Cpf1 endonuclease complex (or the plant cell or plant comprising the guide polynucleotide/Cpf1 endonuclease complex) at temperatures between 29°C and 30°C, between 29°C and 35°C, between 29°C and 40°C, between 29°C and 45°C, between 29°C and 50°C, between 30°C and 35°C, between 35°C and 40°C, between 40°C and 45°C and between 45°C and 50°C.

In some aspects of the disclosure, the Cpf1 endonuclease activity can be activated by increasing the temperature environment of the guide polynucleotide/Cpf1 endonuclease complex, or host cell comprising such complex, from a temperature below or equal to 28°C (ranging from 20°C, 21°C, 22°C, 23°C, 24°C, 25°C, 26°C, 26 up to 28°C, a temperature regime where the Cpf1 endonuclease is nearly inactive) to a temperature of at least 29°C, 30°C, 31°C, 32°C, 33°C, 34°C, 35°C, 36°C, 37°C, 38°C, 39°C, 40°C, 41°C, 42°C, 43°C, 44°C or 45°C or up to 50°C or up to and including any temperature that results in the Cpf1 endonuclease being functional. The period of temperature increase that the guide polynucleotide/Cpf1 endonuclease complex, or host cell comprising such complex, is exposed to can be, for a period of at least about 10 min., 20 min., 30 min. , 40 min., 50 min., 1 hr., 2 hrs., 3 hr., 4 hrs., 5 hrs., 6 hrs., 7 hrs., 8 hrs., 9 hrs., 10 hrs., 11 hrs., 12 hrs., 13 hrs., 14 hrs., 15 hrs., 16 hrs., 17 hrs., 18 hrs,19 hrs., 20 hrs., 21 hrs., 22 hrs., 23 hrs., 24 hrs., 25 hrs., 26 hrs., 27 hrs., 28 hrs., 29 hrs., 30 hrs., 31 hrs., 32 hrs., 33 hrs., 34 hrs., 35 hrs., 36 hrs., 37 hrs., 38 hrs., 39 hrs., 40 hrs., 41 hrs., 42 hrs., 43 hrs., 44 hrs., 45 hrs., 46 hrs., 47 hrs., 48 hrs. 3 days, 4 days, 5 days , 6 days, 7 days, to at least 1, 2, 3, 4, 5 weeks, or to at least multiple months, up to 3 months, or up to and including any time period that results in the Cpf1 endonuclease being functional. Transient induction of the guide polynucleotide/Cpf1 endonuclease activity such as, but not limited to, exposure of a plant cell comprising the Cpf1 endonuclease to a temperature greater than 28°C can be at any developmental stage of the plant cell, plant embryo and/or plant and can be induced in any plant cell type, including plant pollen. Progeny of plants comprising a Cpf1 endonuclease (or a recombinant construct expressing the Cpf1 endonuclease) and a guide RNA (and/or a recombinant construct expressing the guide RNA) can also be exposed to the increased temperature (greater than 28°C and up to 50°C), thereby activating the guide RNA/ Cpf1 complex within the progeny plant (at a desired stage during development) which allows for the progeny plant to be modified at a specific target site that is recognized by the guide RNA/Cpf1 endonuclease complex. For example, transient induction of the guide polynucleotide/Cpf1 endonuclease system during meiosis of a plant cell, may increase the change of a DNA cleavage in genomic regions that are only accessible during meiosis such as regions known to have a low recombination frequency or centromere regions, while minimizing or eliminating activity during other plant cell developmental stages.

The regulated (temperature inducible) guide RNA/Cpf1 system described herein is especially useful for genome engineering, for example in plant genome engineering, in circumstances where nuclease off-target cutting can be toxic to the targeted cells, or otherwise undesired. Transient induction of the Cpf1 endonuclease activity allows for a reduction in off-target cleavage as the Cpf1 endonuclease will cleave DNA most efficiently during the increased temperature treatment (greater than 28°C and up to 50°C) and be inactive or minimally active when the temperature of the plant cell or Cpf1 environment is reduced back to a temperature below 29°C. In one aspect the reduction in off-target cleavage can be at least 5%, 10%, 20%, 30%, 40%, 50%, 60%, 70%, 80%, 90%, 99%, 5-10%, 10-20%, 20-30%, 30-40%, 40-50%, 50-60%, 60-70%, 70-80%, 80-90%, 90-99% up to 100%. The percent (%) off-target cleavage can be measured by any method known in the art such as, but not limiting to, Koo et al. Molecules and Cells 2015, 38(6):475-481 and Cho et al. Genome Res 2014, 24(1):132-141.

In some aspects, herein disclosed is a method for reducing off-target modification using a Cpf1 system comprising: contacting a target nucleic acid molecule having a target sequence with: a single guide polynucleotide comprising a variable targeting domain comprising deoxyribonucleic acid (DNA) and configured to hybridize with a target sequence in a nucleic acid; an Cpf1 recognition domain adjacent to the variable targeting domain comprising a ribonucleic acid (RNA); and a Cpf1 polypeptide, where the single guide polynucleotide forms a complex with the Cpf1 polypeptide and where the target nucleic acid molecule is cleaved or edited at the target sequence more preferentially than at other sequences in the target nucleic acid, thereby reducing off-target modification. In some aspects of the disclosure, the target nucleic acid is DNA, in some aspects of the disclosure, the target nucleic acid is RNA, in some aspects of the disclosure, the target nucleic acid is a mixture of RNA and DNA. In some aspects of the disclosure, the Cpf1 recognition domain is downstream of the targeting region. In some aspects of the disclosure, the Cpf1 recognition domain is upstream of the targeting region. In some aspects of the disclosure, the Cpf1 recognition domain comprises a structure selected from the group consisting of a lower stem, a loop, an upper stem, a hairpin, or any combination thereof. In some aspects of the disclosure, the Cpf1 recognition domain comprises a stem loop structure. In some aspects of the disclosure, the Cpf1 recognition domain interacts with the Cpf1 polypeptide. In some aspects of the disclosure, the Cpf1 recognition domain comprises a mixture of DNA and RNA. In some aspects of the disclosure, the variable targeting domain comprises a mixture of DNA and RNA. In some aspects of the disclosure, the variable targeting domain does not contain uracil.

The Cpf1 endonuclease described herein can be used for targeted genome editing (via simplex and multiplex double-strand breaks and nicks) and targeted genome regulation (via tethering of epigenetic effector domains to either the Cpf1 protein or single guide RNA (sgRNA). A Cpf1 endonuclease can also be engineered to function as an RNA-guided recombinase, and via RNA tethers could serve as a scaffold for the assembly of multiprotein and nucleic acid complexes (Mali et al., 2013, Nature Methods Vol. 10: 957-963).

A Cpf1 protein herein can comprise one or more heterologous nuclear localization sequences (NLS). A heterologous NLS amino acid sequence herein may be of sufficient strength to drive accumulation of a protein in a detectable amount in the nucleus of a eukaryotic cell herein, for example a plant cell. An NLS may comprise one (monopartite) or more (e.g., bipartite) short sequences (e.g., 2 to 20 residues) of basic, positively charged residues (e.g., lysine and/or arginine), and can be located anywhere in a Cpf1 amino acid sequence but such that it is exposed on the protein surface. An NLS may be operably linked to the N-terminus or C-terminus of a Cpf1 protein herein, for example. Two or more NLS sequences can be linked to a Cpf1 protein, for example, such as on both the N- and C-termini of a Cpf1 protein. The Cpf1 endonuclease gene can be operably linked to a SV40 nuclear targeting signal upstream of the Cpf1 codon region and a bipartite VirD2 nuclear localization signal (Tinland et al. (1992) Proc. Natl. Acad. Sci. USA 89:7442-6) downstream of the Cpf1 codon region. Non-limiting examples of suitable NLS sequences herein include those disclosed in U.S. Patent Nos. 6660830 and 7309576.

The term "plant-optimized Cpf1 endonuclease" herein refers to a Cpf1 protein encoded by a nucleotide sequence that has been optimized for expression in a plant cell or plant.

A "plant-optimized nucleotide sequence encoding a Cpf1 endonuclease ", "plant-optimized construct encoding a Cpf1 endonuclease" and a "plant-optimized polynucleotide encoding a Cpf1" are used interchangeably herein and refer to a nucleotide sequence encoding a Cpf1 endonuclease protein, or a variant or functional fragment thereof, that has been optimized for expression in a plant cell or plant. A plant comprising a plant-optimized Cpf1 endonuclease includes a plant comprising the nucleotide sequence encoding for the Cpf1 sequence and/or a plant comprising the Cpf1 endonuclease protein. In one aspect, the plant-optimized Cpf1 endonuclease nucleotide sequence results in increased Cpf1 protein expression when compared to the wild-type sequence of which is was optimized from. In one aspect, the plant-optimized nucleotide sequence encoding Cpf1 endonuclease is a maize-optimized, canola-optimized, sunflower-optimized, rice-optimized, wheat-optimized, or soybean-optimized Cpf1 endonuclease. In one aspect, the plant-optimized nucleotide sequence encodes a protein of SEQ ID NO: 10 or SEQ ID NO: 11, or a functional fragment thereof. A plant-optimized nucleotide sequence includes a codon-optimized gene. A plant-optimized nucleotide sequence can be synthesized by modifying a nucleotide sequence encoding a protein such as, for example, a Cpf1 endonuclease as disclosed herein, using one or more plant-preferred codons for improved expression. See, for example, Campbell and Gowri (1990) Plant Physiol. 92:1-11 for a discussion of host-preferred codon usage.

Any Cpf1 protein (or any DNA sequence encoding a Cpf1 protein) can be used for the methods described herein and include, but is not limited to the Cpf1 proteins of SEQ ID NOs: 10, 11, 32 or 33, or functional variants of SEQ ID NOs: 10, 11, 32 or 33, or functional fragments of SEQ ID NOs: 10, 11, 32 or 33. Other Cpf1 proteins for use in the compositions and methods disclosed herein include those described, for example, in U.S. Patent Publication No. 2016/0208243.

The terms "functional fragment ", "fragment that is functionally equivalent" and "functionally equivalent fragment" of a Cpf1 endonuclease are used interchangeably herein, and refer to a portion or subsequence of the Cpf1 endonuclease protein sequence in which the ability to recognize, bind to, and optionally nick or cleave (introduce a single or double-strand break in) the target site is retained.

The terms "functional variant ", "variant that is functionally equivalent" and "functionally equivalent variant" of a Cpf1 endonuclease are used interchangeably herein, and refer to a variant of the Cpf1 endonuclease protein sequence in which the ability to recognize, bind to, and optionally nick or cleave (introduce a single or double-strand break in) the target site is retained. Fragments and variants can be obtained via methods such as site-directed mutagenesis and synthetic construction.

Functional fragments of a Cpf1 endonucleases include fragments comprising 50-100, 100-200, 100-300, 100-400, 100-500, 100-600, 100-700, 100-800, 100-900, 100-1000, 200-300, 200-400, 200-500, 200-600, 200-700, 200-800, 200-900, 200-1000, 300-400, 300-500, 300-600, 300-700, 300-800, 300-900, 300-1000, 400-500, 400-600, 400-700, 400-800, 400-900, 400-1000, 500-600, 500-700, 500-800, 500-900, 500-1000, 600-700, 600-800, 600-900, 600-1000, 700-800, 700-900, 700-1000, 800-900, 800-1000, 900-1000, 1000-1100, 1100-1200 or 1200-1300 amino acids of a reference Cpf1 protein, such as the reference Cpf1 endonucleases of the present disclosure of SEQ ID NOs: 10 or 11.

Functional fragments of a Cpf1 endonuclease include proteins comprising at least one domain selected from the group consisting of a guide polynucleotide binding domain (an amino acid domain that can bind to or hybridize to a guide RNA), a crRNA binding domain (an amino acid domain that can bind to or hybridize to a crRNA), a DNA binding domain (an amino acid domain that can bind to a DNA target sequence), a DNA cleavage domain (an amino acid domain that can cleave a DNA target sequence), and any combination thereof.

The Cpf1 protein, or functional fragment thereof, for use in the disclosed methods, can be isolated from a recombinant source where the genetically modified host cell (e.g. an insect cell or a yeast cell or human-derived cell line) is modified to express the nucleic acid sequence encoding the Cpf1 protein. Alternatively, the Cpf1 protein can be produced using cell free protein expression systems or be synthetically produced.

A functional variant of the Cpf1 endonuclease can comprise a modified form of the Cpf1 polypeptide. The modified form of the Cpf1 polypeptide can include an amino acid change (e.g., deletion, insertion, or substitution) that reduces the naturally-occurring nuclease activity of the Cpf1 protein. For example, in some instances, the modified form of the Cpf1 protein has less than 50%, less than 40%, less than 30%, less than 20%, less than 10%, less than 5%, or less than 1% of the nuclease activity of the corresponding wild-type Cpf1 polypeptide. In some cases, the modified form of the Cpf1 polypeptide has no substantial nuclease activity and is referred to as catalytically "inactivated Cpf1" or "deactivated Cpf1 (dCpf1)."

A functional variant of a Cpf1 protein sequence may be used, but should have specific binding activity, and optionally endonucleolytic activity, toward DNA when associated with an RNA component herein. Such a functional variant may comprise an amino acid sequence that is at least about 20%, 30%, 40%, 50%, 60%, 70%, 80%, 81%, 82%, 83%, 84%, 85%, 86%, 87%, 88%, 89%, 90%, 91%, 92%, 93%, 94%, 95%, 96%, 97%, 98%, or 99% identical to the amino acid sequence of the reference Cpf1. Such a variant Cpf1 protein should have specific binding activity, and optionally cleavage or nicking activity, toward DNA when associated with an RNA component herein.

The temperature induction of the guide polynucleotide Cpf1 system described herein may be utilized to regulate the cleavage activity of the Cpf1 endonuclease. In one aspect, this can provide a transient pulse of Cpf1 endonuclease activity from plasmid DNA expression cassettes which would reduce the potential for off-target cleavage activity. Additionally, the temperature induction of guide RNA/Cpf1 activity from plasmid DNA expression cassettes could be utilized to time cleavage activity with the desired cellular, tissue culture or plant developmental stage long after it has been introduced into the cell. The guide polynucleotide Cpf1 system described herein may also be combined with inducible (e.g. heat shock, chemical or cell cycle) or tissue specific promoters to provide additional control over Cpf1 endonuclease activity.

Cpf1 endonuclease fragments and variants can be obtained via methods such as site-directed mutagenesis and synthetic construction.

Methods for determining if fragments and/or variants of a Cpf1 endonuclease of the present disclosure are functional include methods that measure the endonuclease activity of the fragment or variant when in complex with a suitable polynucleotide. Methods that measure endonuclease activity are well known in the art such as, but not limiting to, PCT/US13/39011, filed May 1, 2013, PCT/US16/32073 filed May 12, 2016, PCT/US16/32028 filed May 12, 2016). Methods for measuring Cpf1 endonuclease activity include methods that measure the mutation frequency at a target site after a double strand break has occurred.

Methods for measuring Cpf1 endonuclease activity include methods that measure the mutation frequency at a target site after a double strand break has occurred. Methods for measuring if a functional fragment or functional variant of a Cpf1 endonuclease of the present disclosure can make a double strand break include the following method: briefly, appropriate CRISPR-Cpf1 maize genomic DNA target sites can be selected, a guide RNA transcriptional cassette (recombinant DNA that expresses a guide RNA) and a DNA recombinant construct expressing the Cpf1 endonuclease of the present disclosure (or a functional fragment of the Cpf1 endonuclease of the present disclosure, or a functional variant of the Cpf1 endonuclease variant of the present disclosure endonuclease can be constructed and can be co-delivered by biolistic transformation into Hi-Type II 10-day-old immature maize embryos (IMEs) in the presence of BBM and WUS2 genes as described in Svitashev *et al.* (2015). A visual marker DNA expression cassette encoding a yellow fluorescent protein can also be co-delivered with the guide RNA transcriptional cassette and the Cpf1 endonuclease expression cassette (recombinant DNA construct) to aid in the selection of evenly transformed IMEs. After 2 days, the 20-30 most evenly transformed IMEs can be harvested based on their fluorescence. Total genomic DNA is extracted and the DNA region surrounding the intended target site is PCR amplified with Phusion^{®} HighFidelity PCR Master Mix (New England Biolabs, M0531L) adding on the sequences necessary for amplicon-specific barcodes and Illumnia sequencing and deep sequenced. The resulting reads are then examined for the presence of mutations at the expected site of cleavage by comparison to control experiments where the guide RNA transcriptional cassette was omitted from the transformation. If mutations are observed at the intended target sites when using a fragment or variant of the Cpf1 endonuclease of the present disclosure, in complex with a suitable guide polynucleotide, the fragments or variants are functional.

Methods for measuring if a functional fragment of functional variant of a Cpf1 endonuclease of the present disclosure can make a single strand break (also referred to as a nick; hence acts as a nickase) in the double stranded DNA target site include the following method: The cellular repair of chromosomal single-strand breaks (SSBs) in a double-stranded DNA target may be typically repaired seamlessly in plant cells such as maize. Therefore, to examine a functional Cpf1 fragment or functional variant of a Cpf1 for nicking activity, two chromosomal DNA target sites in close proximity (0-200 bp), each targeting a different strand (sense and anti-sense DNA strands) of the double-stranded DNA, can be targeted. If SSB activity is present, the SSB activity from both target sites will result in a DNA double-strand break (DSB) that will result in the production of insertion or deletion (indel) mutagenesis in maize cells. This outcome can then be used to detect and monitor the activity of the Cpf1 nickase similar to that described in Karvelis et al. (2015). Briefly, appropriate CRISPR-Cpf1 maize genomic DNA target sites are selected, guide RNA transcription cassettes and functional fragment Cpf1 nicking expression cassettes are constructed and co-delivered by biolistic transformation into Hi-Type II 10-day-old immature maize embryos (IMEs) in the presence of BBM and WUS2 genes as described in Svitashev et al. (2015). Since particle gun transformation can be highly variable, a visual marker DNA expression cassette encoding a yellow fluorescent protein can also be co-delivered to aid in the selection of evenly transformed IMEs [immature maize embryos]. After 2 days, the 20-30 most evenly transformed IMEs are harvested based on their fluorescence, total genomic DNA extracted, the region surrounding the intended target site PCR amplified with Phusion^{®} HighFidelity PCR Master Mix (New England Biolabs, M0531L) adding on the sequences necessary for amplicon-specific barcodes and Illumnia sequencing and deep sequenced. The resulting reads are then examined for the presence of mutations at the expected site of cleavage by comparison to control experiments where the small RNA transcriptional cassette was omitted from the transformation.

Methods for measuring if a functional fragment of functional variant of a Cpf1 endonuclease of the present disclosure can bind to the intended DNA target site include the following method: The binding of a maize chromosomal DNA target site does not result in either a single-stranded break (SSB) or a double-stranded break (DSB) in the double-stranded DNA target site. Therefore, to examine a functional Cpf1 fragment for binding activity in maize cells, another nuclease domain (e.g. Fokl) may be attached to the functional Cpf1 fragment with binding activity. If binding activity is present, the added nuclease domain may be used to produce a DSB that will result in the production of insertion or deletion (indel) mutagenesis in maize cells. This outcome may then be used to detect and monitor the binding activity of a Cpf1 similar to that described in Karvelis et al. (2015). Briefly, appropriate CRISPR-Cpf1 maize genomic DNA target sites can be selected, guide RNA transcription cassettes and functional fragment Cpf1 binding and nuclease attached expression cassettes can be constructed and co-delivered by biolistic transformation into Hi-Type II 10-day-old immature maize embryos (IMEs) in the presence of BBM and WUS2 genes as described in Svitashev et al. (2015). A visual marker DNA expression cassette encoding a yellow fluorescent protein can also be co-delivered to aid in the selection of evenly transformed IMEs [immature maize embryos]. After 2 days, the 20-30 most evenly transformed IMEs can be harvested based on their fluorescence, total genomic DNA extracted, the region surrounding the intended target site PCR amplified with Phusion^{®} HighFidelity PCR Master Mix (New England Biolabs, M0531L) adding on the sequences necessary for amplicon-specific barcodes and Illumnia sequencing and deep sequenced. The resulting reads can then be examined for the presence of mutations at the expected site of cleavage by comparison to control experiments where the small RNA transcriptional cassette was omitted from the transformation.

Alternatively, the binding activity of maize chromosomal DNA target sites can be monitored by the transcriptional induction or repression of a gene. This can be accomplished by attaching a transcriptional activation or repression domain to the functional Cpf1 binding fragment and targeting it to the promoter region of a gene and binding monitored through an increase in accumulation of the gene transcript or protein. The gene targeted for either activation or repression can be any naturally occurring maize gene or engineered gene (e.g. a gene encoded a red fluorescent protein) introduced into the maize genome by methods known in the art (e.g. particle gun or agrobacterium transformation).

A Cpf1 protein can be part of a fusion protein comprising one or more heterologous protein domains (e.g., 1, 2, 3, or more domains in addition to the Cpf1 protein). Such a fusion protein may comprise any additional protein sequence, and optionally a linker sequence between any two domains, such as between Cpf1 and a first heterologous domain. Examples of protein domains that may be fused to a Cpf1 protein herein include, without limitation, epitope tags (e.g., histidine [His], V5, FLAG, influenza hemagglutinin [HA], myc, VSV-G, thioredoxin [Trx]), reporters (e.g., glutathione-5-transferase [GST], horseradish peroxidase [HRP], chloramphenicol acetyltransferase [CAT], beta-galactosidase, beta-glucuronidase [GUS], luciferase, green fluorescent protein [GFP], HcRed, DsRed, cyan fluorescent protein [CFP], yellow fluorescent protein [YFP], blue fluorescent protein [BFP]), and domains having one or more of the following activities: methylase activity, demethylase activity, transcription activation activity (e.g., VP16 or VP64), transcription repression activity, transcription release factor activity, histone modification activity, RNA cleavage activity and nucleic acid binding activity. A Cpf1 protein can also be in fusion with a protein that binds DNA molecules or other molecules, such as maltose binding protein (MBP), S-tag, Lex A DNA binding domain (DBD), GAL4A DNA binding domain, and herpes simplex virus (HSV) VP16.

A catalytically inactive Cpf1 can be fused to a heterologous sequence. Suitable fusion partners include, but are not limited to, a polypeptide that provides an activity that indirectly increases transcription by acting directly on the target DNA or on a polypeptide (e.g., a histone or other DNA-binding protein) associated with the target DNA. Additional suitable fusion partners include, but are not limited to, a polypeptide that provides for methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity, or demyristoylation activity. Further suitable fusion partners include, but are not limited to, a polypeptide that directly provides for increased transcription of the target nucleic acid (e.g., a transcription activator or a fragment thereof, a protein or fragment thereof that recruits a transcription activator, a small molecule/drug-responsive transcription regulator, etc.). A catalytically inactive Cpf1 can also be fused to a FokI nuclease to generate double-strand breaks.

As used herein, the term "guide polynucleotide", relates to a polynucleotide sequence that can form a complex with a Cas endonuclease, such as a Cpf1 endonuclease or Cas9 endonuclease, and enables the Cas endonuclease to recognize, bind to, and optionally cleave a DNA target site. For Cpf1 endonuclease systems, the term guide polynucleotide and crPolynucleotide can be used interchangeably. The guide polynucleotide sequence can be a RNA sequence, a DNA sequence, or a combination thereof (a RNA-DNA combination sequence, also referred to a RNA-DNA polynucleotide). Optionally, the guide polynucleotide can comprise at least one nucleotide, phosphodiester bond or linkage modification such as, but not limited, to Locked Nucleic Acid (LNA), 5-methyl dC, 2,6-Diaminopurine, 2'-Fluoro A, 2'-Fluoro U, 2'-O-Methyl RNA, phosphorothioate bond, linkage to a cholesterol molecule, linkage to a polyethylene glycol molecule, linkage to a spacer 18 (hexaethylene glycol chain) molecule, or 5' to 3' covalent linkage resulting in circularization. A guide polynucleotide that solely comprises ribonucleic acids is also referred to as a "guide RNA" or "gRNA" (See also U.S. Patent Application US20150082478, published on March 19, 2015 and US20150059010, published on February 26, 2015).

The guide polynucleotide comprises a first nucleotide sequence domain that is recognized by a Cas endonuclease (such as a Cpf1 endonuclease), referred to as Cas endonuclease recognition domain (CER domain; Cpf1 recognition domain for Cpf1 endonucleases) and a Variable Targeting domain or VT domain that can hybridize to a nucleotide sequence in a target DNA. For Cpf1 endonuclease systems, the term guide RNA and crRNA can be used interchangeably. The guide polynucleotide includes a single molecule (also referred to as single guide polynucleotide, sgRNA). By "domain" it is meant a contiguous stretch of nucleotides that can be RNA, DNA, and/or RNA-DNA-combination sequence (also referred to as hybrid DNA-RNA or hDRNA). The VT domain and /or the CER domain of a guide polynucleotide can comprise a RNA sequence, a DNA sequence, or a RNA-DNA-combination sequence. The guide polynucleotide may be referred to as "guide RNA" (when composed of a contiguous stretch of RNA nucleotides) or "guide DNA" (when composed of a contiguous stretch of DNA nucleotides) or "guide RNA-DNA" (when composed of a combination of RNA and DNA nucleotides). In one aspect, the guide polynucleotide can form a complex with a Cpf1 endonuclease, where the guide polynucleotide/Cpf1 endonuclease complex (also referred to as a guide polynucleotide/Cpf1 endonuclease system) can direct the Cpf1 endonuclease to a genomic target site, enabling the Cpf1 endonuclease to recognize, bind to, and optionally nick or cleave (introduce a single or double-strand break) the target site.

In some aspects of the disclosure, the VT domain and /or the Cpf1 recognition domain of a guide polynucleotide can comprise a RNA sequence, a DNA sequence, or a RNA-DNA-combination sequence. For example, the VT domain can comprise an RNA sequence, or an RNA sequence containing one or more DNA nucleotides within the VT sequence. Similarly, the Cpf1 recognition domain can comprise an RNA sequence, or an RNA sequence containing one or more DNA nucleotides within the CER sequence. In another example, the guide polynucleotide can comprise an RNA sequence containing one or more DNA nucleotides within the VT domain and one or more DNA nucleotides within the CER domain.

In one aspect the guide polynucleotide is a DNA-RNA guide polynucleotide (also referred to as hybrid DNA-RNA guide polynucleotide, hDRNA, RNA-DNA polynucleotide) comprising at least one DNA nucleotide in the Cpf1 recognition domain and/or at least one DNA nucleotide in the variable targeting domain.

In one aspect the Cpf1 recognition domain of a guide polynucleotide can form a 5' stem loop 3' stem secondary structure, where the Cpf1 recognition domain comprises RNA and at least one DNA nucleotide. The

In one aspect the Cpf1 recognition domain comprises DNA nucleotides either at the 5' end or the loop structure.

In one aspect the variable targeting domain comprises DNA nucleotides at the 3' end.

In one aspect, the guide polynucleotide is selected from the group consisting of SEQ ID NOs: 40, 41, 42, 43, 44, 45 and 46.

In some aspects, herein disclosed is a single guide polynucleotide for use with a Cpf1 system comprising a variable targeting domain comprising deoxyribonucleic acid (DNA), ribonucleic acid (RNA) or a mixture of DNA and RNA, and configured to hybridize with a target sequence in a nucleic acid; and a Cpf1 recognition domain adjacent to the variable targeting domain comprising a DNA, RNA or a mixture of DNA and RNA. In some aspects of the disclosure, the Cpf1 recognition domain is upstream of the variable targeting domain. In some aspects of the disclosure, the Cpf1 recognition domain comprises at least one secondary structure selected from the group consisting of a lower stem, loop, an upper stem, hairpin, one or more of these, and combinations thereof. In some aspects of the disclosure, the Cpf1 recognition domain comprises a stem loop structure forming an internal duplex. Cpf1 can bind to the Cpf1 recognition domain of the guide polynucleotide in a sequence and structure specific manner, that recognizes the stem loop and sequences adjacent to the stem loop, most notably, the nucleotide 5' of the variable targeting domain that hybridizes to the target nucleic acid. This stem loop structure can be at least 8, 9, 10, 11, 12, 13, 14, 15, 16, 17, 18, 19 up to 100 nucleotides in length. The stem loop structure can further comprise an insertion of additional polynucleotides in the loop structure of the stem loop.

A stem loop comprises a 5' element and a 3' element.

In some aspects of the disclosure, the 5' element of the stem loop of the Cpf1 recognition domain comprises a DNA, a RNA or a mixture of DNA-RNA.

In some aspects of the disclosure, the 3' element of the stem loop of the Cpf1 recognition domain comprises a DNA, a RNA or a mixture of DNA-RNA.

In some aspects of the disclosure, the loop of the stem loop of the Cpf1 recognition domain comprises a DNA, a RNA or a mixture of DNA-RNA.

In some aspects of the disclosure, the Cpf1 recognition domain comprises a stem loop structure comprising a mixture of DNA and RNA. The DNA-RNA mixture of the Cpf1 recognition domain may comprise various proportions of DNA and RNA. In certain aspects of the disclosure, this apportionment may be 5%, 10%, 15%, 20%, 25%, 30%, 35%, 40%, 45%, 50%, 55%, 60%, 65%, 70%, 75%, 80%, 85%, 90%, 95%, or 99% RNA and ranges there between.

In some aspects of the disclosure, the variable targeting domain comprises a mixture of DNA and RNA. The DNA-RNA mixture of the variable targeting domain may comprise various proportions of DNA and RNA. In certain aspects of the disclosure, this apportionment may be 5%, 10%, 15%, 20%, 25%, 30%, 35%, 40%, 45%, 50%, 55%, 60%, 65%, 70%, 75%, 80%, 85%, 90%, 95%, or 99% RNA and ranges there between.

In some aspects of the disclosure, the variable targeting domain comprises a DNA and the Cpf1 recognition domain comprises a DNA.

In some aspects of the disclosure, the variable targeting domain comprises an DNA and the Cpf1 recognition domain comprises a RNA.

In some aspects of the disclosure, the variable targeting domain comprises a DNA and the Cpf1 recognition domain comprises a mixture of DNA and RNA.

In some aspects of the disclosure, the variable targeting domain comprises a RNA and the Cpf1 recognition domain comprises a DNA.

In some aspects of the disclosure, the variable targeting domain comprises an RNA and the Cpf1 recognition domain comprises a RNA.

In some aspects of the disclosure, the variable targeting domain comprises a RNA and the Cpf1 recognition domain comprises a mixture of DNA and RNA.

In some aspects of the disclosure, the variable targeting domain comprises a mixture of DNA and RNA and the Cpf1 recognition domain comprises a DNA.

In some aspects of the disclosure, the variable targeting domain comprises a mixture of DNA and RNA and the Cpf1 recognition domain comprises a RNA.

In some aspects of the disclosure, the variable targeting domain comprises a mixture of DNA and RNA and the Cpf1 recognition domain comprises a mixture of DNA and RNA.

In some aspects, herein disclosed is a single guide polynucleotide comprising a variable targeting domain comprising deoxyribonucleic acid (DNA) and configured to hybridize with a target sequence in a nucleic acid; an Cpf1 recognition domain adjacent to the variable targeting domain comprising a ribonucleic acid (RNA), where the single guide polynucleotide forms a complex with a Cpf1 polypeptide and optionally, where the target nucleic acid molecule is cleaved or edited at the target sequence more preferentially than at other sequences in the target nucleic acid, thereby reducing off-target modification. In some aspects of the disclosure, the target nucleic acid is DNA, in some aspects of the disclosure, the target nucleic acid is RNA, in some aspects of the disclosure, the target nucleic acid is a mixture of RNA and DNA. In some aspects of the disclosure, the Cpf1 recognition domain is downstream of the targeting region. In some aspects of the disclosure, the Cpf1 recognition domain is upstream of the targeting region. In some aspects of the disclosure, the Cpf1 recognition domain comprises a structure selected from the group consisting of a lower stem, a bulge, an upper stem, a nexus, and a hairpin. In some aspects of the disclosure, the Cpf1 recognition domain comprises a stem loop structure. In some aspects of the disclosure, the Cpf1 recognition domain interacts with the Cpf1 polypeptide. In some aspects of the disclosure, the Cpf1 recognition domain comprises a mixture of DNA and RNA. In some aspects of the disclosure, the variable targeting domain comprises a mixture of DNA and RNA. In some aspects of the disclosure, the variable targeting domain is free of uracil.

In some aspects of the disclosure, the Cpf1 recognition domain comprises a stem loop structure comprising a mixture of DNA and RNA. The DNA-RNA, mixture of the Cpf1 recognition domain may comprise various proportions of DNA and RNA. In certain aspects of the disclosure, this apportionment may be 5%, 10%, 15%, 20%, 25%, 30%, 35%, 40%, 45%, 50%, 55%, 60%, 65%, 70%, 75%, 80%, 85%, 90%, 95%, or 99% RNA and ranges there between.

The guide polynucleotide includes a chimeric non-naturally occurring guide RNA that comprises regions that are not found together in nature (i.e., they are heterologous with each other. For example, a chimeric non-naturally occurring guide RNA (crRNA) comprising a first nucleotide sequence domain (VT domain) that can hybridize to a nucleotide sequence in a target DNA, linked to a second nucleotide sequence domain (Cpf1 recognition domain) such that the first and second nucleotide sequence domains are not found linked together in nature.

The term "variable targeting domain" or "VT domain" is used interchangeably herein and includes a nucleotide sequence that can hybridize (is complementary) to one strand (nucleotide sequence) of a double strand DNA target site. The % complementation between the first nucleotide sequence domain (VT domain) and the target sequence can be at least 50%, 51%, 52%, 53%, 54%, 55%, 56%, 57%, 58%, 59%, 60%, 61%, 62%, 63%, 63%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. The variable targeting domain can be at least 12, 13, 14, 15, 16, 17, 18, 19, 20, 21, 22, 23, 24, 25, 26, 27, 28, 29 or 30 nucleotides up to 50 nucleotides in length.

In some aspects, the percent complementation between the variable targeting domain of the guide polynucleotide (crPolynucleotide) capable of forming a functional guide polynucleotide/Cpf1 endonuclease complex, and the target sequence is less than 100% due to the presence of mismatches between the variable targeting domain of the guide polynucleotide and the target sequence. As described herein Cpf1endonuclease cleaves its DNA target site in a region with high tolerance for mismatches between the guide RNA (crRNA) and complementary DNA target. Nucleotide positions in a target site located +21, +22, +23 and +24 and 3 prime of the PAM can be mismatched with the guide polynucleotide and still result in precise DNA cleavage at the intended target site (Kim, D. et al. (2016) Nature Biotechnology. 34863-868; Kleinstiver, B. et al. (2016) Nature Biotechnology. 34, 869-874). The presence of 4 mismatches in a VT domain of 21 nucleotides, represents a percent complementation of about 80%.

In one aspect of the disclosure, the guide polynucleotides comprise a mismatch with regard to the target site at a position selected from +21, +22, +23, +24, or combinations of positions +21, +22, +23, and +24, 3' of the PAM sequence.

In some aspects, the VT domain can have a mismatch at a nucleotide positions in a target site selected from the group of nucleotides in a target site located at +21, +22, +23 and +24. In some aspects, the VT domain can have at least 1, 2, 3, 4, 5, 6, 7, 8, 9, 10, 11, 12, 13, 14, up to 15 mismatches with its DNA target site and still result in guiding the Cpf1 protein to its intended DNA target site.

In some aspects of the disclosure, the variable targeting domain comprises a contiguous stretch of 12 to 30, 12 to 29, 12 to 28, 12 to 27, 12 to 26, 12 to 25, 12 to 26, 12 to 25, 12 to 24, 12 to 23, 12 to 22, 12 to 21, 12 to 20, 12 to 19, 12 to 18, 12 to 17, 12 to 16, 12 to 15, 12 to 14, 12 to 13, 13 to 30, 13 to 29, 13 to 28, 13 to 27, 13 to 26, 13 to 25, 13 to 26, 13 to 25, 13 to 24, 13 to 23, 13 to 22, 13 to 21, 13 to 20, 13 to 19, 13 to 18, 13 to 17, 13 to 16, 13 to 15, 13 to 14, 14 to 30, 14 to 29, 14 to 28, 14 to 27, 14 to 26, 14 to 25, 14 to 26, 14 to 25, 14 to 24, 14 to 23, 14 to 22, 14 to 21, 14 to 20, 14 to 19, 14 to 18, 14 to 17, 14 to 16, 14 to 15, 15 to 30, 15 to 29, 15 to 28, 15 to 27, 15 to 26, 15 to 25, 15 to 26, 15 to 25, 15 to 24, 15 to 23, 15 to 22, 15 to 21, 15 to 20, 15 to 19, 15 to 18, 15 to 17, 15 to 16, 16 to 30, 16 to 29, 16 to 28, 16 to 27, 16 to 26, 16 to 25, 16 to 24, 16 to 23, 16 to 22, 16 to 21, 16 to 20, 16 to 19, 16 to 18, 16 to 17, 17 to 30, 17 to 29, 17 to 28, 17 to 27, 17 to 26, 17 to 25, 17 to 24, 17 to 23, 17 to 22, 17 to 21, 17 to 20, 17 to 19, 17 to 18, 18 to 30, 18 to 29, 18 to 28, 18 to 27, 18 to 26, 18 to 25, 18 to 24, 18 to 23, 18 to 22, 18 to 21, 18 to 20, 18 to 19, 19 to 30, 19 to 29, 19 to 28, 19 to 27, 19 to 26, 19 to 25, 19 to 24, 19 to 23, 19 to 22, 19 to 21, 19 to 20, 20 to 30, 20 to 29, 20 to 28, 20 to 27, 20 to 26, 20 to 25, 20 to 24, 20 to 23, 20 to 22, 20 to 21, 21 to 30, 21 to 29, 21 to 28, 21 to 27, 21 to 26, 21 to 25, 21 to 24, 21 to 23, 21 to 22, 22 to 30, 22 to 29, 22 to 28, 22 to 27, 22 to 26, 22 to 25, 22 to 24, 22 to 23, 23 to 30, 23 to 29, 23 to 28, 23 to 27, 23 to 26, 23 to 25, 23 to 24, 24 to 30, 24 to 29, 24 to 28, 24 to 27, 24 to 26, 24 to 25, 25 to 30, 25 to 29, 25 to 28, 25 to 27, 25 to 26, 26 to 30, 26 to 29, 26 to 28, 26 to 27, 27 to 30, 27 to 29, 27 to 28, 28 to 30, 28 to 29, or 29 to 30 nucleotides.

The variable targeting domain can be composed of a DNA sequence, a RNA sequence, a modified DNA sequence, a modified RNA sequence, or any combination thereof.

The term "Cpf1 endonuclease recognition domain" or "Cpf1 recognition domain" (of a guide polynucleotide, crRNA) is used interchangeably herein and refers to a nucleotide sequence that interacts with a Cpf1 endonuclease polypeptide. The Cpf1 endonuclease recognition domain can be composed of a DNA sequence, a RNA sequence, a modified DNA sequence, a modified RNA sequence, or any combination thereof.

The terms "functional fragment ", "fragment that is functionally equivalent" and "functionally equivalent fragment" of a guide polynucleotide (or of a guide RNA, crRNA) are used interchangeably herein, and refer to a portion or subsequence of the guide polynucleotide (or of the guide RNA, crRNA) , of the present disclosure in which the ability to function as a guide polynucleotide (guide RNA, crRNA) is retained.

The terms "functional variant ", "Variant that is functionally equivalent" and "functionally equivalent variant" of a guide polynucleotide (or of a guide RNA, crRNA) are used interchangeably herein, and refer to a variant of the guide polynucleotide (or of the guide RNA, crRNA), of the present disclosure in which the ability to function as a guide polynucleotide (guide RNA, crRNA) is retained.

A functional fragments of a guide RNA or guide polynucleotide of the present disclosure include a fragment of 20-40, 20-45, 20-50, 20-55, 20-60, 20-65, 20-70, 20-75, 20-80, 25-40, 25-45, 25-50, 25-55, 25-60, 25-65, 25-70, 25-75, 25-80, 30-40, 30-45, 30-50, 30-55, 30-60, 30-65, 30-70, 30-75, 30-80, 35-40, 35-45, 35-50, 35-55, 35-60, 35-65, 35-70, 35-75, 35-80, 40-45, 40-50, 40-55, 40-60, 40-65, 40-70, 40-75, 40-80, 45-50, 45-55, 45-60, 45-65, 45-70, 45-75, 45-80, 50-55, 50-60, 50-65, 50-70, 50-75, 50-80, 55-55, 55-60, 55-65, 55-70, 55-75, 55-80, 60-65, 60-70, 60-75, 60-80, 65-70, 65-75, 65-80, 70-75, 70-80 or 75-80 nucleotides of a reference guide RNA, such as the reference guide RNAs of SEQ ID NOs: 15-18.

A functional variant of a single guide RNA or guide polynucleotide of the present disclosure may comprise a nucleotide sequence that is at least about 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% identical to reference single guide RNA, such as the reference single guide RNA of SEQ ID NOs: 15-18, described herein. In some aspects of the disclosure, a functional variant of a single guide RNA comprises a nucleotide sequence having at least about 60%, at least about 65%, at least about 70%, at least about 75%, at least about 80%, at least about 85%, at least about 90%, at least about 95%, at least about 98%, at least about 99%, or 100% nucleotide sequence identity over a stretch of at least 8, 9, 10, 11, 12, 13, 14, 15, 16, 17, 18, 19, 20, 21, 22, 23, 24, 25, 26, 27, 28, 29, 30 contiguous nucleotides to any one of the nucleotide sequences set forth in SEQ ID NOs: 15-18.

Functional variants of a guide polynucleotide of the present disclosure can comprise a modified guide polynucleotide where the modification comprises an engineered secondary structure, and/or an artificial loop, and/or a reduction in the length and/or degree of complementation in a region of hybridization compared to a region of hybridization of a reference guide polynucleotide, including the guide polynucleotides of SEQ ID NOs: 15-18, and/or a reduction in the length and/or degree of complementation in the portion of the protein-binding segment that forms a double stranded RNA duplex.

Functional variants of a guide polynucleotide of the present disclosure can comprise a modified guide polynucleotide where the modification comprises adding, removing, or otherwise altering loops and/or hairpins in the single guide RNA. Functional variants of a guide polynucleotide of the present disclosure includes guide polynucleotides of the present disclosure that have been modified at the 5' end, at the 3' end, or at any location occurring within the nucleotide sequence, or any combination thereof.

Functional variants of a guide polynucleotide of the present disclosure can comprise a modified guide polynucleotide where the modification comprises one or more modified nucleotides in the nucleotide sequence, or at the 5' or 3' end, where the one or more modified nucleotides comprises at least one non-naturally-occurring nucleotide, nucleotide mimetic (as described in US application US2014/0068797, published March 6, 2014), or analog thereof, or where the one or more modified nucleotides are selected from the group consisting of 2'-0-methylanalogs, 2'-fluoro analogs 2-aminopurine, 5-bromo-uridine, pseudouridine, and 7 -methylguanosine.

In one aspect, the functional variant of the guide RNA (crRNA) can form a guide RNA/Cpf1 endonuclease complex that can recognize, bind to, and optionally nick or cleave a target sequence.

The guide polynucleotide can be produced by any method known in the art, including chemically synthesizing guide polynucleotides (such as but not limiting to Hendel et al. 2015, Nature Biotechnology 33, 985-989), in vitro generated guide polynucleotides, and/or self-splicing guide RNAs (such as but not limiting to Xie et al. 2015, PNAS 112:3570-3575).

A method of expressing RNA components such as gRNA in eukaryotic cells for performing Cas-mediated DNA targeting has been to use RNA polymerase III (Pol III) promoters, which allow for transcription of RNA with precisely defined, unmodified, 5'- and 3'-ends (DiCarlo et al., Nucleic Acids Res. 41: 4336-4343; Ma et al., Mol. Ther. Nucleic Acids 3:e161). This strategy has been successfully applied in cells of several different species including maize and soybean (US 20150082478, published on March 19, 2015). Methods for expressing RNA components that do not have a 5' cap have been described (WO 2016/025131, published on February 18, 2016). Any promoter capable of expressing a guide RNA in prokaryotic or eukaryotic cells can be used. For example, a promoter capable of transcribing the guide RNA in a cell includes a heat shock /heat inducible promoter operably linked to a nucleotide sequence encoding the guide RNA.

A multitude of guide polynucleotides (at least two single guide polynucleotides) can be produced simultaneously from a single precursor polynucleotide that is processed into at least two single guide polynucleotides, where each single guide polynucleotide comprises a variable targeting domain (3' of a Protospacer Adjacent Motif) that is substantially complementary to a single target sequence in the plant genome, where the guide polynucleotide is capable of forming a guide polynucleotide /Cpf1 endonuclease complex that can recognize, bind to, and optionally nick or cleave a target sequence. Processing of the precursor polynucleotide into at least two guide polynucleotides can be accomplished by the ribonuclease activity of the Cpf1 endonuclease. In short, a single precursor polynucleotide can be synthesized to multiple guide polynucleotide, each of such guide polynucleotides containing a variable targeting domain comprising deoxyribonucleic acid (DNA), ribonucleic acid (RNA) or a mixture of DNA and RNA, and configured to hybridize with a target sequence in a nucleic acid, and a Cpf1 recognition domain adjacent to the variable targeting domain comprising DNA, RNA or a mixture of DNA and RNA. The precursor polynucleotide can then be processed into a multitude of single guide polynucleotides by the ribonucleotide activity of the Cpf1 endonuclease.

In one aspect, the precursor polynucleotide is a precursor polynucleotide capable of being processed by a Cpf1 endonuclease into multiple single guide polynucleotides, where each of the single guide polynucleotide and Cpf1 endonuclease are capable of forming a complex that can recognize, bind to, and optionally nick or cleave a DNA target sequence.

In one aspect, the precursor polynucleotide is a precursor polynucleotide capable of being processed by a Cpf1 endonuclease into multiple single guide polynucleotides, where the precursor polynucleotide is processed into at least 2, 3, 4, 5, 6, 7, 8, 9, 10, 11, 12, 13, 14, 15, 16, 17, 18, 19, 20, 25, 20, 35, 40, 45, 50, 55, 60, 65, 70, 75, 80, 85, 90, 95 up to 100 single guide polynucleotides, where each of the single guide polynucleotide and Cpf1 endonuclease are capable of forming a complex that can recognize, bind to, and optionally nick or cleave a DNA target sequence.

In one aspect, the precursor polynucleotide can be be synthesized to comprise at least 2, 3, 4, 5, 6, 7, 8, 9, 10, 11, 12, 13, 14, 15, 16, 17, 18, 19, 20, 25, 20, 35, 40, 45, 50, 55, 60, 65, 70, 75, 80, 85, 90, 95 up to 100 single guide polynucleotides, where each single guide polynucleotide comprises a variable targeting domain located 3' of a Protospacer Adjacent Motif, and where the variable targeting domain is substantially complementary to a single target sequence in the plant genome.

A multitude of guide RNAs (at least two single guide RNAs) can be produced simultaneously from a single precursor RNA that is processed into at least two single guide RNAs, where each single guide RNA comprises a variable targeting domain (3' of a Protospacer Adjacent Motif) that is substantially complementary to a single target sequence in the plant genome, where the guide RNA is capable of forming a guide RNA/Cpf1 endonuclease complex that can recognize, bind to, and optionally nick or cleave a target sequence. Processing of the precursor RNA into at least two guide RNAs can be accomplished by the ribonuclease activity of the Cpf1 endonuclease (Fonfara, I. et al. (2016) Nature. 532: 517-521). In short, a single precursor RNA molecule can be produced from a precursor guide RNA transcriptional initiation cassette capable of expressing the single precursor RNA. The precursor RNA can then be processed into a multitude of single guide RNAs by the ribonucleotide activity of the Cpf1 endonuclease.

As used herein the term "precursor guide RNA transcriptional initiation cassette" refers to a recombinant DNA molecule that encodes a precursor guide RNA comprising at least 2, 3, 4, 5, 6, 7, 8, 9, 10, 11, 12, 13, 14, 15, 16, 17, 18, 19, 20, 25, 20, 35, 40, 45, 50, 55, 60, 65, 70, 75, 80, 85, 90, 95 up to 100 single guide RNAs, where each single guide RNA comprises a variable targeting domain located 3' of a Protospacer Adjacent Motif, and where the variable targeting domain is substantially complementary to a single target sequence in the plant genome.

As used herein the term "precursor guide RNA" refers to a single RNA molecule that comprises at least 2, 3, 4, 5, 6, 7, 8, 9, 10, 11, 12, 13, 14, 15, 16, 17, 18, 19, 20, 25, 20, 35, 40, 45, 50, 55, 60, 65, 70, 75, 80, 85, 90, 95 up to 100 single guide RNAs, that can be processed into its individual single guide RNAs (at least 2, 3, 4, 5, 6, 7, 8, 9, 10, 11, 12, 13, 14, 15, 16, 17, 18, 19, 20, 25, 20, 35, 40, 45, 50, 55, 60, 65, 70, 75, 80, 85, 90, 95 up to 100 single guide RNAs) via the ribonuclease activity of Cpf1 protein, and where each single guide RNA comprises a variable targeting domain located 3' of a Protospacer Adjacent Motif, and is substantially complementary to a single target sequence in the plant genome.

In one aspect, the precursor RNA is a precursor guide RNA capable of being processed by a Cpf1 endonuclease into multiple single guide RNAs, where each of the single guide RNA and Cpf1 endonuclease are capable of forming a complex that can recognize, bind to, and optionally nick or cleave a DNA target sequence.

In one aspect, the precursor RNA is a precursor guide RNA capable of being processed by a Cpf1 endonuclease into multiple single guide RNAs, where the precursor RNA is processed into at least 2, 3, 4, 5, 6, 7, 8, 9, 10, 11, 12, 13, 14, 15, 16, 17, 18, 19, 20, 25, 20, 35, 40, 45, 50, 55, 60, 65, 70, 75, 80, 85, 90, 95 up to 100 single guide RNAs, where each of the single guide RNA and Cpf1 endonuclease are capable of forming a complex that can recognize, bind to, and optionally nick or cleave a DNA target sequence.

In one aspect, the precursor RNA can be encoded by a recombinant DNA comprising a Pol-II or a Pol-III promoter operably linked to a nucleotide sequence encoding a precursor guide RNA comprising at least 2, 3, 4, 5, 6, 7, 8, 9, 10, 11, 12, 13, 14, 15, 16, 17, 18, 19, 20, 25, 20, 35, 40, 45, 50, 55, 60, 65, 70, 75, 80, 85, 90, 95 up to 100 single guide RNAs, where each single guide RNA comprises a variable targeting domain located 3' of a Protospacer Adjacent Motif, and where the variable targeting domain is substantially complementary to a single target sequence in the plant genome. An example of a Pol-III promoter driving the expression of the precursor RNA can be, but is not limiting to, a maize U6 Polymerase III promoter. An example of a Pol-II promoter driving the expression of the precursor RNA can be, but is not limiting to, a ubiquitin promoter.

In one aspect the Cpf1 protein that processes the precursor RNA into at least two guide RNAs is the same Cpf1 protein that can form a guide RNA/Cpf1 endonuclease complex that can recognize, bind to, and optionally nick or cleave a target sequence. The precursor guide RNA transcriptional initiation cassette includes a recombinant DNA molecule (also referred to as expression cassette) comprising a Pol-II or a Pol-III promoter operably linked to a DNA sequence encoding the precursor RNA.

Nucleotide sequence modification of the guide polynucleotide, VT domain and/or Cpf1 recognition domain can be selected from, but not limited to , the group consisting of a 5' cap, a 3' polyadenylated tail, a riboswitch sequence, a stability control sequence, a sequence that forms a dsRNA duplex, a modification or sequence that targets the guide poly nucleotide to a subcellular location, a modification or sequence that provides for tracking , a modification or sequence that provides a binding site for proteins , a Locked Nucleic Acid (LNA), a 5-methyl dC nucleotide, a 2,6-Diaminopurine nucleotide, a 2'-Fluoro A nucleotide, a 2'-Fluoro U nucleotide; a 2'-O-Methyl RNA nucleotide, a phosphorothioate bond, linkage to a cholesterol molecule, linkage to a polyethylene glycol molecule, linkage to a spacer 18 molecule, a 5' to 3' covalent linkage, or any combination thereof. These modifications can result in at least one additional beneficial feature, where the additional beneficial feature is selected from the group of a modified or regulated stability, a subcellular targeting, tracking, a fluorescent label, a binding site for a protein or protein complex, modified binding affinity to complementary target sequence, modified resistance to cellular degradation, and increased cellular permeability.

The terms "5'-cap" and "7-methylguanylate (m⁷G) cap" are used interchangeably herein. A 7-methylguanylate residue is located on the 5' terminus of messenger RNA (mRNA) in eukaryotes. RNA polymerase II (Pol II) transcribes mRNA in eukaryotes. Messenger RNA capping occurs generally as follows: The most terminal 5' phosphate group of the mRNA transcript is removed by RNA terminal phosphatase, leaving two terminal phosphates. A guanosine monophosphate (GMP) is added to the terminal phosphate of the transcript by a guanylyl transferase, leaving a 5'-5' triphosphate-linked guanine at the transcript terminus. Finally, the 7-nitrogen of this terminal guanine is methylated by a methyl transferase.

The terminology "not having a 5'-cap" herein is used to refer to RNA having, for example, a 5'-hydroxyl group instead of a 5'-cap. Such RNA can be referred to as "uncapped RNA", for example. Uncapped RNA can better accumulate in the nucleus following transcription, since 5'-capped RNA is subject to nuclear export. One or more RNA components herein are uncapped.

As used herein, the terms "guide polynucleotide/Cas endonuclease complex", "guide polynucleotide/Cas endonuclease system", " guide polynucleotide/Cas complex", "guide polynucleotide/Cas system" and "guided Cas system" "Polynucleotide-guided endonuclease" , "PGEN" are used interchangeably herein and refer to at least one guide polynucleotide and at least one Cas endonuclease that are capable of forming a complex, where the guide polynucleotide/Cas endonuclease complex can direct the Cas endonuclease to a DNA target site, enabling the Cas endonuclease to recognize, bind to, and optionally nick or cleave (introduce a single or double-strand break) the DNA target site.

In one aspect, the guide polynucleotide/Cas endonuclease complex described herein is a guide polynucleotide/Cpf1 endonuclease complex. A guide polynucleotide/Cpf1 endonuclease complex herein can comprise Cpf1 protein(s) and suitable polynucleotide component(s) of any of the known Type V CRISPR systems (Makarova et al. 2015, Nature Reviews Microbiology Vol. 13:1-15; Zetsche et al., 2015, Cell 163, 1-13; Shmakov et al., 2015, Molecular_Cell 60, 1-13).

A guide polynucleotide/Cpf1 endonuclease complex can cleave one or both strands of a DNA target sequence in a prokaryotic and/or eukaryotic cell. A guide polynucleotide/Cpf1 endonuclease complex that can cleave both strands of a DNA target sequence typically comprises a Cpf1 protein that has all of its endonuclease domains in a functional state (e.g., wild type endonuclease domains or variants thereof retaining some or all activity in each endonuclease domain

A guide polynucleotide/Cas endonuclease complex that can cleave one strand of a DNA target sequence can be characterized herein as having nickase activity (e.g., partial cleaving capability). A Cpf1 nickase typically comprises one functional endonuclease domain that allows the Cpf1 to cleave only one strand (i.e., make a nick) of a DNA target sequence (Yamano et al., 2016, Cell 165:949-962). A pair of Cpf1 nickases can be used to increase the specificity of DNA targeting. In general, this can be done by providing two Cpf1 nickases that, by virtue of being associated with RNA components with different-guide sequences, target and nick nearby DNA sequences on opposite strands in the region for desired targeting.

A guide polynucleotide/Cpf1 endonuclease complex in certain aspects of the disclosure can bind to a DNA target site sequence, but does not cleave any strand at the target site sequence. Such a complex may comprise a Cpf1 protein in which all of its nuclease domains are mutant, dysfunctional. For example, a Cpf1 protein herein that can bind to a DNA target site sequence, but does not cleave any strand at the target site sequence, may comprise a mutant, dysfunctional RuvC domain.

A guide polynucleotide/Cpf1 endonuclease complex of the current disclosure can comprise any Cpf1 protein, and include, but is not limited to, the Cpf1 protein of SEQ ID NOs: 10, 11, 32 or 33, or a functional variant of SEQ ID NOs: 10, 11, 32 or 33, or a functional fragment of SEQ ID NOs: 10, 11, 32 or 33.

The terms "guide RNA/Cpf1 endonuclease complex", "guide RNA/Cpf1 endonuclease system", " guide RNA/Cpf1 complex", "guide RNA/Cpf1 system", "gRNA/Cpf1 complex", "gRNA/Cpf1 system" are used interchangeably herein and refer to at least one RNA component and at least one Cpf1 endonuclease that are capable of forming a complex , where the guide RNA/Cpf1 endonuclease complex can direct the Cpf1 endonuclease to a DNA target site, enabling the Cpf1 endonuclease to recognize, bind to, and optionally nick or cleave (introduce a single or double-strand break) the DNA target site.

In one aspect, the guide RNA/Cpf1 endonuclease complex, when incubated at a temperature greater than 28°C (or when present in a plant cell or plant that is incubated in (exposed to) a temperature greater than 28°C for at least 4 hrs., can result in a double strand break at or near the intended target site and subsequent increased mutation frequency of the target site when compared to the frequency of modification at the target sequence of a control plant cell comprising the guide RNA/Cpf1 endonuclease complex that was incubated at a temperature about 28 °C or between about 20°C to 28°C. In one aspect of the disclosure, the method comprises a method for modifying a target sequence in the genome of a plant cell, the method comprising: a) introducing into a plant cell a Cpf1 endonuclease or a plant-optimized polynucleotide encoding the Cpf1 endonuclease, and a guide polynucleotide comprising a variable targeting domain that is substantially complementary to a target sequence in the plant genome; and, b) incubating the plant cell at a temperature greater than 28°C for a period of at least about 4 hrs., where the guide polynucleotide and Cpf1 endonuclease are capable of forming a complex that can cleave the target sequence, where the frequency of mutation at the target site is increased by at least about 2, 3, 4, 5, 6, 7, 8, 9, 10, 11, 12, 13, 14, 15 fold when compared to the frequency of modification at the target site of a control plant cell (comprising the guide RNA/Cpf1 endonuclease complex ) that was incubated at a temperature about 28 °C. In one aspect, the increased frequency of mutation at the intended target site when the guide RNA/Cpf1 endonuclease complex is incubated at a temperature greater than 28C for at least 4 hrs. is increased by at least 2-fold, at least 3-fold, at least 4-fold, at least 5-fold, at least 6-fold, at least 7-fold, at least 8-fold, at least 9-fold, at least 10-fold, at least 10-fold, at least 11-fold , at least 12-fold , at least 13-fold, at least 14-fold, at least 15-fold, at least 30-fold, at least 40-fold, or at least 50-fold, when compared to the frequency of modification at the target site of a control plant cell (comprising the guide RNA/Cpf1 endonuclease complex ) that was incubated at a temperature about 28 °C.

Exposure of the plant cell comprising the guide RNA/Cpf1 endonuclease complex to the temperature greater than 28°C can be at any developmental stage of the plant cell, plant embryo and/or plant. Progeny of plants comprising a Cpf1 endonuclease (or a recombinant construct expressing the Cpf1 endonuclease) and a guide RNA (and/or a recombinant construct expressing the guide RNA) can also be exposed a temperature greater than 28°C, thereby activating the guide RNA/ Cpf1 complex within the progeny plant (at a desired stage during development) which allows for the progeny plant to be modified at a specific target site that is recognized by the guide RNA/Cpf1 endonuclease complex.

The terms "target site", "target sequence", "target site sequence, "target DNA", "target locus", "genomic target site", "genomic target sequence", "genomic target locus" and "protospacer", are used interchangeably herein and refer to a polynucleotide sequence such as, but not limited to, a nucleotide sequence on a chromosome, episome, a transgenic locus, or any other DNA molecule in the genome (including chromosomal, choloroplastic, mitochondrial DNA, plasmid DNA) of a cell, at which a guide polynucleotide/Cas endonuclease complex, such as a guide polynucleotide/Cpf1 endonuclease complex described herein, can recognize, bind to, and optionally nick or cleave . The target site can be an endogenous site in the genome of a cell, or alternatively, the target site can be heterologous to the cell and thereby not be naturally occurring in the genome of the cell, or the target site can be found in a heterologous genomic location compared to where it occurs in nature. As used herein, terms "endogenous target sequence" and "native target sequence" are used interchangeable herein to refer to a target sequence that is endogenous or native to the genome of a cell and is at the endogenous or native position of that target sequence in the genome of the cell. An "artificial target site" or "artificial target sequence" are used interchangeably herein and refer to a target sequence that has been introduced into the genome of a cell. Such an artificial target sequence can be identical in sequence to an endogenous or native target sequence in the genome of a cell but be located in a different position (i.e., a non-endogenous or non-native position) in the genome of a cell.

As described herein, a Cpf1 DNA target site refers to a DNA target site that is recognized by a Cpf1 endonuclease protein, where the Cpf1 endonuclease is capable of forming a complex that can recognize, bind to, and optionally nick or cleave the Cpf1 DNA target site

As described herein, an *Acidaminococcus* Cpf1 DNA target site includes a DNA target site that is recognized by a Cpf1 endonuclease protein from *Acidaminococcus* or *Acidaminococcus* Cpf1 endonuclease protein produced in a plant, where the Cpf1 endonuclease is capable of forming a complex that can recognize, bind to, and optionally nick or cleave the *Acidaminococcus* Cpf1 DNA target site.

An "altered target site", "altered target sequence", "modified target site", "modified target sequence" are used interchangeably herein and refer to a target sequence as disclosed herein that comprises at least one alteration when compared to non-altered target sequence. Such "alterations" include, for example: (i) replacement of at least one nucleotide, (ii) a deletion of at least one nucleotide, (iii) an insertion of at least one nucleotide, or (iv) any combination of (i) - (iii).

Methods for "modifying a target site" and "altering a target site" are used interchangeably herein and refer to methods for producing an altered target site.

The length of the target DNA sequence (target site) can vary, and includes, for example, target sites that are at least 12, 13, 14, 15, 16, 17, 18, 19, 20, 21, 22, 23, 24, 25, 26, 27, 28, 29, 30 or more nucleotides in length. It is further possible that the target site can be palindromic, that is, the sequence on one strand reads the same in the opposite direction on the complementary strand. The nick/cleavage site can be within the target sequence or the nick/cleavage site could be outside of the target sequence. In another variation, the cleavage could occur at nucleotide positions immediately opposite each other to produce a blunt end cut or, in other Cases, the incisions could be staggered to produce single-stranded overhangs, also called "sticky ends", which can be either 5' overhangs, or 3' overhangs. Active variants of genomic target sites can also be used. Such active variants can comprise at least 65%, 70%, 75%, 80%, 85%, 90%, 91%, 92%, 93%, 94%, 95%, 96%, 97%, 98%, 99% or more sequence identity to the given target site, where the active variants retain biological activity and hence are capable of being recognized and cleaved by an Cpf1 endonuclease.

Assays to measure the single or double-strand break of a target site by an endonuclease are known in the art and generally measure the overall activity and specificity of the agent on DNA substrates containing recognition sites.

The frequency of mutation at a target site of a targeted plant cell caused by the activity of the guide RNA/Cpf1 endonuclease system described herein, can be increased when the targeted plant cell is exposed to a temperature treatment that activates the CPf1 endonuclease as described herein, when compared to the frequency of modification at the target sequence of a control plant cell that was exposed to a temperature of about 28 °C. The increased mutation frequency of the temperature induced guide RNA/Cpf1 endonuclease system when compared to a control guide RNA/Cpf1 endonuclease system 28 °C can be at least 2-fold, at least 3-fold, at least 4-fold, at least 5-fold, at least 6-fold, at least 7-fold, at least 8-fold, at least 9-fold, at least 10-fold, at least 10-fold, at least 11-fold , at least 12-fold, at least 13-fold, at least 14-fold, at least 15-fold, at least 30-fold, at least 40-fold, or at least 50-fold.

A "protospacer adjacent motif" (PAM) herein refers to a short nucleotide sequence adjacent to a target sequence (protospacer) that is recognized (targeted) by a guide polynucleotide/Cas endonuclease system, such as a guide polynucleotide/Cpf1 endonuclease system described herein. The Cpf1 endonuclease may not successfully recognize a target DNA sequence if the target DNA sequence is not preceded by a PAM sequence. The sequence and length of a PAM herein can differ depending on the Cpf1 protein or Cpf1 protein complex used. The PAM sequence can be of any length but is typically 1, 2, 3, 4, 5, 6, 7, 8, 9, 10, 11, 12, 13, 14, 15, 16, 17, 18, 19 or 20 nucleotides long. The PAM sequence that is recognized by a Cpf1 endonuclease is located 5 of the target site.

The guide polynucleotide/Cpf1 systems described herein can be used for gene targeting.

The terms "gene targeting", "targeting", and "DNA targeting" are used interchangeably herein. DNA targeting herein may be the specific introduction of a knock-out, edit, or knock-in at a particular DNA sequence, such as in a chromosome or plasmid of a cell. In general, DNA targeting can be performed herein by cleaving one or both strands at a specific DNA sequence in a cell with a Cpf1 protein associated with a suitable polynucleotide component. Once a single or double-strand break is induced in the DNA, the cell's DNA repair mechanism is activated to repair the break via nonhomologous end-joining (NHEJ) or Homology-Directed Repair (HDR) processes which can lead to modifications at the target site.

The terms "knock-out", "gene knock-out" and "genetic knock-out" are used interchangeably herein. A knock-out represents a DNA sequence of a cell that has been rendered partially or completely inoperative by targeting with a Cpf1 protein; such a DNA sequence prior to knock-out could have encoded an amino acid sequence, or could have had a regulatory function (e.g., promoter), for example.

As described herein, a guided Cpf1 endonuclease can recognize, bind to a DNA target sequence and introduce a single strand (nick) or double-strand break. Once a single or double-strand break is induced in the DNA, the cell's DNA repair mechanism is activated to repair the break. Error-prone DNA repair mechanisms can produce mutations at double-strand break sites. The most common repair mechanism to bring the broken ends together is the nonhomologous end-joining (NHEJ) pathway (Bleuyard et al., (2006) DNA Repair 5:1-12). The structural integrity of chromosomes is typically preserved by the repair, but deletions, insertions, or other rearrangements (such as chromosomal translocations) are possible (Siebert and Puchta, 2002, Plant Cell 14:1121-31; Pacher et al., 2007, Genetics 175:21-9).

In one aspect of the disclosure, the method comprises a method for modifying a target sequence in the genome of a plant cell, the method comprising: a) introducing into a plant cell a Cpf1 endonuclease or a plant-optimized polynucleotide encoding the Cpf1 endonuclease, and a guide polynucleotide comprising a variable targeting domain that is substantially complementary to a target sequence in the plant genome; and, b) incubating the plant cell at a temperature greater than 28°C for a period of at least about 4 hrs., where the guide polynucleotide and Cpf1 endonuclease are capable of forming a complex that can recognize, bind to, and optionally nick or cleave the target sequence.

In one aspect of the disclosure, the method comprises a method for modifying a target sequence in the genome of a plant cell, the method comprising: a) introducing a guide polynucleotide comprising a variable targeting domain that is substantially complementary to a target sequence in the plant genome into a plant cell comprising in its genome a recombinant DNA construct comprising a promoter operably linked to a plant-optimized polynucleotide encoding a Cpf1 endonuclease; and, b) incubating the plant cell at a temperature greater than 28°C for a period of at least about 4 hrs., where the guide polynucleotide and Cpf1 endonuclease are capable of forming a complex that can recognize, bind to, and optionally nick or cleave the target sequence.

In one aspect of the disclosure, the method comprises a method for modifying a target sequence in the genome of a plant or plant cell, the method comprising: a) obtaining a plant or plant cell comprising in its genome a first recombinant DNA construct, the first recombinant DNA construct comprising a promoter operably linked to plant-optimized polynucleotide encoding a Cpf1 endonuclease, and a second recombinant DNA construct, the second recombinant DNA construct comprising a promoter operably linked to a nucleotide sequence encoding a guide RNA, where the guide RNA and Cpf1 endonuclease are capable of forming a complex that can recognize, bind to, and optionally nick or cleave the target sequence; and, b) incubating the plant or plant cell at a temperature greater than 28°C for a period of at least about 4 hrs., where the guide RNA and Cpf1 endonuclease are capable of forming a complex that can recognize, bind to, and optionally nick or cleave the target sequence.

The method can further comprise identifying at least one plant cell, plant or progeny plant that has a modification at the target sequence, where the modification at the target sequence is selected from the group consisting of (i) a replacement of at least one nucleotide, (ii) a deletion of at least one nucleotide, (iii) an insertion of at least one nucleotide, and (iv) any combination of (i) - (iii). The method can further provide a donor DNA to the plant cell, where the donor DNA comprises a polynucleotide of interest. This can produce a plant cell or plant having a detectable targeted genome modification.

A knock-out may be produced by an indel (insertion or deletion of nucleotide bases in a target DNA sequence through NHEJ), or by specific removal of sequence that reduces or completely destroys the function of sequence at or near the targeting site.

The guide polynucleotide/Cpf1 endonuclease system can be used in combination with at least one polynucleotide modification template to allow for editing (modification) of a genomic nucleotide sequence of interest.

A "modified nucleotide" or "edited nucleotide" refers to a nucleotide sequence of interest that comprises at least one alteration when compared to its non-modified nucleotide sequence. Such "alterations" include, for example: (i) replacement of at least one nucleotide, (ii) a deletion of at least one nucleotide, (iii) an insertion of at least one nucleotide, or (iv) any combination of (i) - (iii).

The term "polynucleotide modification template" includes a polynucleotide that comprises at least one nucleotide modification when compared to the nucleotide sequence to be edited. A nucleotide modification can be at least one nucleotide substitution, addition or deletion. Optionally, the polynucleotide modification template can further comprise homologous nucleotide sequences flanking the at least one nucleotide modification, where the flanking homologous nucleotide sequences provide sufficient homology to the desired nucleotide sequence to be edited. The polynucleotide modification template includes a single stranded or double stranded DNA molecule.

In one aspect the polynucleotide modification template can comprise at least one mismatch in a region of the DNA target sequence that has low tolerance for mismatches. A mismatch includes a base single nucleotide polymorphism (base snip), a base alteration (base insertion, base deletion or base modification), an insertion of a nucleotide sequence, or any combination thereof.

As described herein, Cpf1 endonuclease cleaves its DNA target site in a region with high tolerance for mismatches between the guide RNA (crRNA) and complementary DNA target. Nucleotide positions in a target site located +21, +22, +23 and +24 and 3 prime of the PAM can be mismatched with the guide polynucleotide and still result in precise DNA cleavage at the intended target site. The regions of low tolerance of mismatches at position +1 to +19 (3 prime) from the PAM sequence adjacent to the target site can be exploited to design polynucleotide modification templates that can introduce at least one base alteration (mismatch) at a low tolerance position in a DNA target site (such as at position +1 to +19 (3 prime) from the PAM sequence adjacent to the target site) as well as introduce the desired gene edit a t a position outside the low tolerance region of the DNA target. Such a design can result in the destruction of the original DNA target recognition by the Cpf1 endonuclease thereby ensuring that the Cpf1 endonuclease does not continue to be active once the desired gene edit has occurred. As such, by carefully designing the polynucleotide modification templates as described herein, one can reduce the CPf1 activity once the desired edit has occurred (as at least one mismatch is introduced into a low tolerance region of the DNA target) and thereby can reduce off-target cleavage by the Cpf1 endonuclease.

In one aspect of the disclosure, the polynucleotide modification template is a polynucleotide modification template comprising at least one region that is complementary to a DNA target sequence adjacent to a PAM sequence recognized by a Cpf1 complex, where the at least one region that corresponds to a DNA target sequence comprises at least one nucleotide mismatch compared to the DNA target sequence in a position from +1 to +19, 3' to the PAM sequence. The polynucleotide modification template can further comprise a modification in a region that corresponds to the target DNA sequence in a position from +20 to +21, +20 to +22, +20 to +23, +20 to +24, +21 to +22, +21 to +23, +21 to +24, +22 to +23, +22 to +24, +23 to +24, 3' to the PAM sequence.

In one aspect of the disclosure, the polynucleotide modification template is a polynucleotide modification template comprising at least one region that is complementary to a DNA target sequence adjacent to a PAM sequence recognized by a Cpf1 complex, where the at least one region that corresponds to a DNA target sequence comprises at least one nucleotide mismatch compared to the DNA target sequence in a position from +2 to +19, +3 to +19, +4 to +19, +5 to +19, +6 to +19, +7 to +19, +8 to +19, +9 to +19, +10 to +19, +11 to +19, +12 to +19, +13 to +19, +14 to +19,+15 to +19,+16 to +19,+17 to +19, +18 to +19, +19 to +19, 3' to the PAM sequence. The polynucleotide modification template can further comprise a modification in a region that corresponds to the target DNA sequence in a position from +20 to +24, 3' to the PAM sequence.

In one aspect of the disclosure, the polynucleotide modification template is a polynucleotide modification template comprising at least one region that is complementary to a DNA target sequence adjacent to a PAM sequence recognized by a Cpf1 complex, where the at least one region that corresponds to a DNA target sequence comprises at least one nucleotide mismatch compared to the DNA target sequence in a position from +1 to +19, +2 to +19, +3 to +19, +4 to +19, +5 to +19, +6 to +19, +7 to +19, +8 to +19, +9 to +19, +10 to +19, +11 to +19, +12 to +19, +13 to +19, +14 to +19,+15 to +19,+16 to +19,+17 to +19, +18 to +19, +19 to +193' to the PAM sequence. The polynucleotide modification template can further comprise a modification in a region that corresponds to the target DNA sequence in a position from +20 to +21, +20 to +22, +20 to +23, +20 to +24, +21 to +22, +21 to +23, +21 to +24, +22 to +23, +22 to +24, +23 to +24, 3' to the PAM sequence.

In one aspect, the polynucleotide modification template comprises 2, 3, 4, 5, 6, 7, 8, 9, 10, 11, 12, 13, 14,15 16, 17, 18 or more nucleotide mismatches compared to the DNA target sequence in a position from +1 to +19, 3' to the PAM sequence.

In one aspect of the disclosure, the polynucleotide modification template is a polynucleotide modification template comprising at least one region that is complementary to a DNA target sequence adjacent to a PAM sequence recognized by a Cpf1 complex, where the at least one region that corresponds to a DNA target sequence comprises at least one nucleotide mismatch compared to the DNA target sequence in a position from +1 to +19, +2 to +19, +3 to +19, +4 to +19, +5 to +19, +6 to +19, +7 to +19, +8 to +19, +9 to +19, +10 to +19, +11 to +19, +12 to +19, +13 to +19, +14 to +19,+15 to +19,+16 to +19,+17 to +19, +18 to +19, +19 to +193' to the PAM sequence. The polynucleotide modification template can further comprise at least one nucleotide insertion in a region that corresponds to the target DNA sequence in a position from +20 to +21, +20 to +22, +20 to +23, +20 to +24, +21 to +22, +21 to +23, +21 to +24, +22 to +23, +22 to +24, +23 to +24, 3' to the PAM sequence.

In one aspect of the disclosure, the polynucleotide modification template is a polynucleotide modification template comprising at least one region that is complementary to a DNA target sequence adjacent to a PAM sequence recognized by a Cpf1 complex, where the at least one region that corresponds to a DNA target sequence comprises at least one nucleotide mismatch compared to the DNA target sequence in a position from +1 to +19, +2 to +19, +3 to +19, +4 to +19, +5 to +19, +6 to +19, +7 to +19, +8 to +19, +9 to +19, +10 to +19, +11 to +19, +12 to +19, +13 to +19, +14 to +19,+15 to +19,+16 to +19,+17 to +19, +18 to +19, +19 to +193' to the PAM sequence. The polynucleotide modification template can further comprise a second DNA region that is complementary to a nucleotide sequence in the genome of a plant, where the polynucleotide modification template comprises at least one nucleotide modification of the nucleotide sequence. In one aspect, the polynucleotide modification template further comprises a first flanking region and a second flanking region, where the first and second flanking regions flank the target DNA sequence and are capable of directing homology directed repair.

In one aspect, the polynucleotide modification template is a polynucleotide modification template comprising at least one region that is complementary to a DNA target sequence adjacent to a PAM sequence recognized by a Cpf1 complex, where the at least one region that corresponds to a DNA target sequence comprises at least one nucleotide mismatch compared to the DNA target sequence in a position from +1 to +19, 3' to the PAM sequence, where the mismatch is caused by at least one nucleotide insertion or at least one polynucleotide insertion in a position from +1 to +19, 3' to the PAM sequence . In some aspects the polynucleotide insertion can be an insertion of a polynucleotide of interest described herein.

In one aspect, the polynucleotide modification template is selected from the group consisting of SEQ ID NOs: 23, 24 and 25.

In one aspect of the disclosure, the method comprises a method for editing a nucleotide sequence in the genome of a plant cell, the method comprising; a) introducing into a plant cell a Cpf1 endonuclease or a plant-optimized polynucleotide encoding the Cpf1 endonuclease, a guide polynucleotide comprising a variable targeting domain that is substantially complementary to a target sequence in the plant genome, and a polynucleotide modification template, where the polynucleotide modification template comprises at least one nucleotide modification of the nucleotide sequence; and, b) incubating the plant cell at a temperature greater than 28°C for a period of at least about 4 hrs., where the guide polynucleotide and Cpf1 endonuclease are capable of forming a complex that can recognize, bind to, and optionally nick or cleave all or part of the target sequence.

The nucleotide to be edited can be located within or outside a target site recognized and cleaved by a Cpf1 endonuclease. In one aspect of the disclosure, the at least one nucleotide modification is not a modification at a target site recognized and cleaved by a Cpf1 endonuclease. In another aspect of the disclosure, there are at least 1, 2, 3, 4, 5, 6, 7, 8, 9, 10, 11, 12, 13, 14, 15, 16, 17, 18, 19, 20, 21, 22, 23, 24, 25, 26, 27, 30, 40, 50, 100, 200, 300, 400, 500, 600, 700, 900 or 1000 nucleotides between the at least one nucleotide to be edited and the genomic target site.

In one aspect of the disclosure, the method comprises a method for editing a nucleotide sequence in the genome of a plant cell, the method comprising; a) introducing a polynucleotide modification template and a guide polynucleotide, comprising a variable targeting domain that is substantially complementary to a target sequence in the plant genome, into a plant cell comprising in its genome a recombinant DNA construct comprising a promoter operably linked to a plant-optimized polynucleotide encoding a Cpf1 endonuclease, where the polynucleotide modification template comprises at least one nucleotide modification of the nucleotide sequence; and, b) incubating the plant cell at a temperature greater than 28°C for a period of at least about 4 hrs., where the guide polynucleotide and Cpf1 endonuclease are capable of forming a complex that can recognize, bind to, and optionally nick or cleave the target sequence.

In one aspect of the disclosure, the method comprises for editing a nucleotide sequence in the genome of a plant or plant cell, the method comprising; a) introducing a polynucleotide modification template into a plant or plant cell comprising in its genome a first recombinant DNA construct, the first recombinant DNA construct comprising a promoter operably linked to plant-optimized polynucleotide encoding a Cpf1 endonuclease, and a second recombinant DNA construct, the second recombinant DNA construct comprising a promoter operably linked to a nucleotide sequence encoding guide RNA, where the guide RNA and Cpf1 endonuclease are capable of forming a complex that can recognize, bind to, and optionally nick or cleave the target sequence; and, b) incubating the plant cell at a temperature greater than 28°C for a period of at least about 4 hrs., where the guide RNA and Cpf1 endonuclease are capable of forming a complex that can recognize, bind to, and optionally nick or cleave the target sequence.

The terms "knock-in", "gene knock-in, "gene insertion" and "genetic knock-in" are used interchangeably herein. A knock-in represents the replacement or insertion of a DNA sequence at a specific DNA sequence in cell by targeting with a Cpf1 protein (for example by homologous recombination (HR), where a suitable donor DNA polynucleotide is also used). Examples of knock-ins are a specific insertion of a heterologous amino acid coding sequence in a coding region of a gene, or a specific insertion of a transcriptional regulatory element in a genetic locus.

Various methods and compositions can be employed to obtain a cell or organism having a polynucleotide of interest inserted in a target site for a Cpf1 endonuclease. Such methods can employ homologous recombination (HR) to provide integration of the polynucleotide of Interest at the target site. In one aspect of the disclosure, a polynucleotide of interest is introduced into the organism cell via a donor DNA construct. As used herein, "donor DNA" is a DNA construct that comprises a polynucleotide of Interest to be inserted into the target site of a Cpf1 endonuclease. The donor DNA construct further comprises a first and a second region of homology that flank the polynucleotide of Interest. The first and second regions of homology of the donor DNA share homology to a first and a second genomic region, respectively, present in or flanking the target site of the cell or organism genome.

In one aspect of the disclosure, the method comprises a method for introducing a polynucleotide of interest into the genome of a plant cell, the method comprising; a) introducing into a plant cell a Cpf1 endonuclease or a plant-optimized polynucleotide encoding the Cpf1 endonuclease, a guide polynucleotide comprising a variable targeting domain that is substantially complementary to a target sequence in the plant genome, and a donor DNA, where the donor DNA comprises a polynucleotide of interest; and, b) incubating the plant cell at a temperature greater than 28°C for a period of at least about 4 hrs., where the guide polynucleotide and Cpf1 endonuclease are capable of forming a complex that can recognize, bind to, and optionally nick or cleave all or part of the target sequence. The polynucleotide of interest can be integrated within or outside a target site recognized and cleaved by a Cpf1 endonuclease.

In one aspect of the disclosure, the method comprises a method for introducing a polynucleotide of interest into the genome of a plant cell, the method comprising; a) introducing a donor DNA and a guide polynucleotide, comprising a variable targeting domain that is substantially complementary to a target sequence in the plant genome, into a plant cell comprising in its genome a recombinant DNA construct comprising a promoter operably linked to a plant-optimized polynucleotide encoding a Cpf1 endonuclease, where the polynucleotide modification template comprises at least one nucleotide modification of the nucleotide sequence; and, b) incubating the plant cell at a temperature greater than 28°C for a period of at least about 4 hrs., where the donor DNA comprises a polynucleotide of interest, where the guide polynucleotide and Cpf1 endonuclease are capable of forming a complex that can recognize, bind to, and optionally nick or cleave the target sequence.

In one aspect of the disclosure, the method comprises for introducing a polynucleotide of interest into the genome of a plant cell, the method comprising; a) introducing a donor DNA into a plant or plant cell comprising in its genome a first recombinant DNA construct, the first recombinant DNA construct comprising a promoter operably linked to plant-optimized polynucleotide encoding a Cpf1 endonuclease, and a second recombinant DNA construct, the second recombinant DNA construct comprising a promoter operably linked to a nucleotide sequence encoding guide RNA, where the donor DNA comprises a polynucleotide of interest, where the guide RNA and Cpf1 endonuclease are capable of forming a complex that can recognize, bind to, and optionally nick or cleave the target sequence; and, b) incubating the plant cell at a temperature greater than 28°C for a period of at least about 4 hrs., where the guide RNA and Cpf1 endonuclease are capable of forming a complex that can recognize, bind to, and optionally nick or cleave the target sequence. The method can further comprise identifying at least one plant cell comprising in its genome the polynucleotide of Interest integrated into or near the target sequence.

The donor DNA can be tethered to the guide polynucleotide. Tethered donor DNAs can allow for co-localizing target and donor DNA, useful in genome editing, gene insertion, and targeted genome regulation, and can also be useful in targeting post-mitotic cells where function of endogenous HR machinery is expected to be highly diminished (Mali et al., 2013, Nature Methods Vol. 10 : 957-963).

Episomal DNA molecules can also be ligated into the double-strand break, for example, integration of T-DNAs into chromosomal double-strand breaks (Chilton and Que, (2003) Plant Physiol 133:956-65; Salomon and Puchta, (1998) EMBO J 17:6086-95). Once the sequence around the double-strand breaks is altered, for example, by exonuclease activities involved in the maturation of double-strand breaks, gene conversion pathways can restore the original structure if a homologous sequence is available, such as a homologous chromosome in non-dividing somatic cells, or a sister chromatid after DNA replication (Molinier et al., (2004) Plant Cell 16:342-52). Ectopic and/or epigenic DNA sequences may also serve as a DNA repair template (polynucleotide modification template) for homologous recombination (Puchta, (1999) Genetics 152:1173-81).

Homology-directed repair (HDR) is a mechanism in cells to repair double-stranded and single stranded DNA breaks. Homology-directed repair includes homologous recombination (HR) and single-strand annealing (SSA) (Lieber. 2010 Annu. Rev. Biochem. 79:181-211). The most common form of HDR is called homologous recombination (HR), which has the longest sequence homology requirements, between the donor and acceptor DNA. Other forms of HDR include single-stranded annealing (SSA) and breakage-induced replication, and these require shorter sequence homology relative to HR. Homology-directed repair at nicks (single-stranded breaks) can occur via a mechanism distinct from HDR at double-strand breaks (Davis and Maizels. PNAS (0027-8424), 111 (10), p. E924-E932).

By "homology" is meant DNA sequences that are similar. For example, a "region of homology to a genomic region" that is found on the donor DNA is a region of DNA that has a similar sequence to a given "genomic region" in the cell or organism genome. A region of homology can be of any length that is sufficient to promote homologous recombination at the cleaved target site. For example, the region of homology can comprise at least 5-10, 5-15, 5-20, 5-25, 5-30, 5-35, 5-40, 5-45, 5- 50, 5-55, 5-60, 5-65, 5- 70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800, 5-2900, 5-3000, 5-3100 or more bases in length such that the region of homology has sufficient homology to undergo homologous recombination with the corresponding genomic region. "Sufficient homology" indicates that two polynucleotide sequences have sufficient structural similarity to act as substrates for a homologous recombination reaction. The structural similarity includes overall length of each polynucleotide fragment, as well as the sequence similarity of the polynucleotides. Sequence similarity can be described by the percent sequence identity over the whole length of the sequences, and/or by conserved regions comprising localized similarities such as contiguous nucleotides having 100% sequence identity, and percent sequence identity over a portion of the length of the sequences.

The amount of homology or sequence identity shared by a target and a donor polynucleotide can vary and includes total lengths and/or regions having unit integral values in the ranges of about 1-20 bp, 20-50 bp, 50-100 bp, 75-150 bp, 100-250 bp, 150-300 bp, 200-400 bp, 250-500 bp, 300-600 bp, 350-750 bp, 400-800 bp, 450-900 bp, 500-1000 bp, 600-1250 bp, 700-1500 bp, 800-1750 bp, 900-2000 bp, 1-2.5 kb, 1.5-3 kb, 2-4 kb, 2.5-5 kb, 3-6 kb, 3.5-7 kb, 4-8 kb, 5-10 kb, or up to and including the total length of the target site. These ranges include every integer within the range, for example, the range of 1-20 bp includes 1, 2, 3, 4, 5, 6, 7, 8, 9, 10, 11, 12, 13, 14, 15, 16, 17, 18, 19 and 20 bps. The amount of homology can also be described by percent sequence identity over the full aligned length of the two polynucleotides which includes percent sequence identity of about at least 50%, 55%, 60%, 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. Sufficient homology includes any combination of polynucleotide length, global percent sequence identity, and optionally conserved regions of contiguous nucleotides or local percent sequence identity, for example sufficient homology can be described as a region of 75-150 bp having at least 80% sequence identity to a region of the target locus. Sufficient homology can also be described by the predicted ability of two polynucleotides to specifically hybridize under high stringency conditions, see, for example, Sambrook et al., (1989) Molecular Cloning: A Laboratory Manual, (Cold Spring Harbor Laboratory Press, NY); Current Protocols in Molecular Biology, Ausubel et al., Eds (1994) Current Protocols, (Greene Publishing Associates, Inc. and John Wiley & Sons, Inc.); and, Tijssen (1993) Laboratory Techniques in Biochemistry and Molecular BiologyHybridization with Nucleic Acid Probes, (Elsevier, New York).

As used herein, a "genomic region" is a segment of a chromosome in the genome of a cell that is present on either side of the target site or, alternatively, also comprises a portion of the target site. The genomic region can comprise at least 5-10, 5-15, 5-20, 5-25, 5-30, 5-35, 5-40, 5-45, 5- 50, 5-55, 5-60, 5-65, 5- 70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800. 5-2900, 5-3000, 5-3100 or more bases such that the genomic region has sufficient homology to undergo homologous recombination with the corresponding region of homology.

The structural similarity between a given genomic region and the corresponding region of homology found on the donor DNA can be any degree of sequence identity that allows for homologous recombination to occur. For example, the amount of homology or sequence identity shared by the "region of homology" of the donor DNA and the "genomic region" of the organism genome can be at least 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity, such that the sequences undergo homologous recombination

The region of homology on the donor DNA can have homology to any sequence flanking the target site. While in some instances the regions of homology share significant sequence homology to the genomic sequence immediately flanking the target site, it is recognized that the regions of homology can be designed to have sufficient homology to regions that may be further 5' or 3' to the target site. The regions of homology can also have homology with a fragment of the target site along with downstream genomic regions

In one aspect of the disclosure, the first region of homology further comprises a first fragment of the target site and the second region of homology comprises a second fragment of the target site, where the first and second fragments are dissimilar.

As used herein, "homologous recombination" includes the exchange of DNA fragments between two DNA molecules at the sites of homology. The frequency of homologous recombination is influenced by a number of factors. Different organisms vary with respect to the amount of homologous recombination and the relative proportion of homologous to non-homologous recombination. Generally, the length of the region of homology affects the frequency of homologous recombination events: the longer the region of homology, the greater the frequency. The length of the homology region needed to observe homologous recombination is also species-variable. In many cases, at least 5 kb of homology has been utilized, but homologous recombination has been observed with as little as 25-50 bp of homology. See, for example, Singer et al., (1982) Cell 31:25-33; Shen and Huang, (1986) Genetics 112:441-57; Watt et al., (1985) Proc. Natl. Acad. Sci. USA 82:4768-72, Sugawara and Haber, (1992) Mol Cell Biol 12:563-75, Rubnitz and Subramani, (1984) Mol Cell Biol 4:2253-8; Ayares et al., (1986) Proc. Natl. Acad. Sci. USA 83:5199-203; Liskay et al., (1987) Genetics 115:161-7.

Alteration of the genome of a plant cell, for example, through homologous recombination (HR), is a powerful tool for genetic engineering. Homologous recombination has been demonstrated in plants (Halfter et al., (1992) Mol Gen Genet 231:186-93) and insects (Dray and Gloor, 1997, Genetics 147:689-99). Homologous recombination has also been accomplished in other organisms. For example, at least 150-200 bp of homology was required for homologous recombination in the parasitic protozoan Leishmania (Papadopoulou and Dumas, (1997) Nucleic Acids Res 25:4278-86). In the filamentous fungus Aspergillus *nidulans*, gene replacement has been accomplished with as little as 50 bp flanking homology (Chaveroche et al., (2000) Nucleic Acids Res 28:e97). Targeted gene replacement has also been demonstrated in the ciliate *Tetrahymena thermophila* (Gaertig et al., (1994) Nucleic Acids Res 22:5391-8). In mammals, homologous recombination has been most successful in the mouse using pluripotent embryonic stem cell lines (ES) that can be grown in culture, transformed, selected and introduced into a mouse embryo (Watson et al., 1992, Recombinant DNA, 2nd Ed., (Scientific American Books distributed by WH Freeman & Co.).

DNA double-strand breaks appear to be an effective factor to stimulate homologous recombination pathways (Puchta et al., (1995) Plant Mol Biol 28:281-92; Tzfira and White, (2005) Trends Biotechnol 23:567-9; Puchta, (2005) J Exp Bot 56:1-14). Using DNA-breaking agents, a two- to nine-fold increase of homologous recombination was observed between artificially constructed homologous DNA repeats in plants (Puchta et al., (1995) Plant Mol Biol 28:281-92). In maize protoplasts, experiments with linear DNA molecules demonstrated enhanced homologous recombination between plasmids (Lyznik et al., (1991) Mol Gen Genet 230:209-18).

One skilled in the art can use the guided Cpf1 endonuclease system for any use known to the art in a similar fashion as the uses for guided Cas9 endonuclease systems, such as but not limiting to modifying or replacing nucleotide sequences of interest (such as a regulatory elements), multiplexing (two or more DNA target sites are targeted at the same time), insertion and or stacking of polynucleotides of interest, gene knock-out, gene-knock in, modification of splicing sites and/or introducing alternate splicing sites, modifications of nucleotide sequences encoding a protein of interest, amino acid and/or protein fusions, and gene silencing by expressing an inverted repeat into a gene of interest. (Patent Application US 2015-0082478 A1, published on March 19, 2015, WO2015/026886 A1, published on February 26, 2015, US 2015-0059010 A1, published on February 26, 2015, US application 62/023246, filed on July 07, 2014, WO2012/129373, published March 14, 2013,and in PCT/US13/22891, published January 24, 2013, and US application 62/036,652, filed on August 13, 2014)

A guide polynucleotide/Cpf1 system as described herein can also be used in methods for directing transgene insertion and / or for producing complex transgenic trait loci comprising multiple transgenes in a fashion similar as disclosed in WO2012/129373, published March 14, 2013where instead of using a double-strand break inducing agent to introduce a gene of interest, a guide polynucleotide/Cpf1 system as disclosed herein is used. A complex trait locus includes a genomic locus that has multiple transgenes genetically linked to each other. By inserting independent transgenes within 0.1, 0.2, 0.3, 0.4, 0.5, 1.0, 2, or even 5 centimorgans (cM) from each other, the transgenes can be bred as a single genetic locus (see, for example, U.S. patent application 13/427,138) or PCT application PCT/US2012/030061. After selecting a plant comprising a transgene, plants containing (at least) one transgenes can be crossed to form an F1 that contains both transgenes. In progeny from these F1 (F2 or BC1) 1/500 progeny would have the two different transgenes recombined onto the same chromosome. The complex locus can then be bred as single genetic locus with both transgene traits. This process can be repeated to stack as many traits as desired.

Any one component of the guide polynucleotide/Cpf1 endonuclease complex, the guide polynucleotide/Cpf1 endonuclease complex itself, as well as the polynucleotide modification template(s) and/or donor DNA(s), can be introduced into a cell by any method known in the art.

"Introducing" is intended to mean presenting to the organism, such as a cell or organism, the polynucleotide or polypeptide or polynucleotide-protein complex, in such a manner that the component(s) gains access to the interior of a cell of the organism or to the cell itself. The methods and compositions do not depend on a particular method for introducing a sequence into an organism or cell, only that the polynucleotide or polypeptide gains access to the interior of at least one cell of the organism. Introducing includes reference to the incorporation of a nucleic acid into a eukaryotic or prokaryotic cell where the nucleic acid may be incorporated into the genome of the cell, and includes reference to the transient (direct) provision of a nucleic acid, protein or polynucleotide-protein complex (PGEN) to the cell.

Methods for introducing polynucleotides or polypeptides or a polynucleotide-protein complex into cells or organisms are known in the art including, but not limited to, microinjection, electroporation, stable transformation methods, transient transformation methods, ballistic particle acceleration (particle bombardment), whiskers mediated transformation, *Agrobacterium*-mediated transformation, direct gene transfer, viral-mediated introduction, transfection, transduction, cell-penetrating peptides, mesoporous silica nanoparticle (MSN)-mediated direct protein delivery, topical applications, sexual crossing , sexual breeding, and any combination thereof.

For example, the guide polynucleotide (guide RNA, guide DNA guide RNA-DNA polynucleotde) can be introduced into a cell directly (transiently) as a single stranded or double stranded polynucleotide molecule. The guide RNA can also be introduced into a cell indirectly by introducing a recombinant DNA molecule comprising a heterologous nucleic acid fragment encoding the guide RNA, operably linked to a promoter that is capable of transcribing the guide RNA in the cell. The promoter can be, but is not limited to, a RNA polymerase III promoter, which allow for transcription of RNA with precisely defined, unmodified, 5'- and 3'-ends (Ma et al., 2014, Mol. Ther. Nucleic Acids 3:e161; DiCarlo et al., 2013, Nucleic Acids Res. 41: 4336-4343; WO2015026887, published on February 26, 2015). Any promoter capable of transcribing the guide RNA in a cell can be used and includes a heat shock /heat inducible promoter operably linked to a nucleotide sequence encoding the guide RNA.

The Cpf1 endonuclease can be introduced into a cell by directly introducing the Cpf1 protein itself (referred to as direct delivery of Cpf1 endonuclease), the mRNA encoding the Cpf1 protein, and/ or the guide polynucleotide/Cpf1 endonuclease complex itself (also referred to as the polynucleoprotein complex, polynucleotide protein complex, or PNP complex), using any method known in the art. The Cpf1 endonuclease can also be introduced into a cell indirectly by introducing a recombinant DNA construct comprising a promoter operably linked to a nucleotide sequence encoding a plant-optimized Cpf1 endonuclease. The recombinant DNA construct comprising a promoter operably linked to a nucleotide sequence encoding a plant-optimized Cpf1 endonuclease can be stably integrated into the genome of a plant. The endonuclease can be introduced into a cell transiently or can be incorporated into the genome of plant cell using any method known in the art. Uptake of the endonuclease and/or the guided polynucleotide into the cell can be facilitated with a Cell Penetrating Peptide (CPP) as described in WO 2016/073433, published on May 12, 2016. Any promoter capable of expressing the Cpf1 endonuclease in a cell can be used and includes a heat shock /heat inducible promoter operably linked to a nucleotide sequence encoding the Cpf1 endonuclease.

Direct delivery of a polynucleotide modification template into plant cells can be achieved through particle mediated delivery. Based on the experiments described herein, a skilled artesian can now envision that any other direct method of delivery, such as but not limiting to, polyethylene glycol (PEG)-mediated transfection to protoplasts, whiskers mediated transformation, electroporation, particle bombardment, cell-penetrating peptides, or mesoporous silica nanoparticle (MSN)-mediated direct protein delivery can be successfully used for delivering a polynucleotide modification template in plant cells.

The donor DNA may be introduced by any means known in the art. The donor DNA may be provided by any transformation method known in the art including, for example, Agrobacterium-mediated transformation or biolistic particle bombardment. The donor DNA may be present transiently in the cell or it could be introduced via a viral replicon. In the presence of the Cpf1 endonuclease and the target site, the donor DNA is inserted into the transformed plant's genome.

Direct delivery of any one of the guided Cpf1 system components can be accompanied by direct delivery (co-delivery) of other mRNAs that can promote the enrichment and/or visualization of cells receiving the guide polynucleotide/Cpf1 endonuclease complex components. For example, direct co-delivery of the guide polynucleotide/Cpf1 endonuclease components (and/or guide polynucleotide/Cpf1 endonuclease complex itself) together with mRNA encoding phenotypic markers (such as but not limiting to transcriptional activators such as CRC (Bruce et al. 2000 The Plant Cell 12:65-79) can enable the selection and enrichment of cells expressing the guide polynucleotide/Cpf1 endonuclease components without the need for stably introducing selectable, phenotypic or screenable markers into the cells.

Protocols for introducing polynucleotides, polypeptides or polynucleotide-protein complexes (PGEN) into plants or plant cells are known and include microinjection (Crossway et al., (1986) Biotechniques 4:320-34 and U.S. Patent No. 6,300,543), meristem transformation (U.S. Patent No. 5,736,369), electroporation (Riggs et al., (1986) Proc. Natl. Acad. Sci. USA 83:5602-6, *Agrobacterium-*mediated transformation (U.S. Patent Nos. 5,563,055 and 5,981,840), whiskers mediated transformation (Ainley et al. 2013, Plant Biotechnology Journal 11:1126-1134; Shaheen A. and M. Arshad 2011 Properties and Applications of Silicon Carbide (2011), 345-358 Editor(s): Gerhardt, Rosario. Publisher: InTech, Rijeka, Croatia. CODEN: 69PQBP; ISBN: 978-953-307-201-2), direct gene transfer (Paszkowski et al., (1984) EMBO J 3:2717-22), and ballistic particle acceleration (U.S. Patent Nos. 4,945,050; 5,879,918; 5,886,244; 5,932,782; Tomes et al., (1995) "Direct DNA Transfer into Intact Plant Cells via Microprojectile Bombardment" in Plant Cell, Tissue, and Organ Culture: Fundamental Methods, ed. Gamborg & Phillips (Springer-Verlag, Berlin); McCabe et al., (1988) Biotechnology 6:923-6; Weissinger et al., (1988) Ann Rev Genet 22:421-77; Sanford et al., (1987) Particulate Science and Technology 5:27-37 (onion); Christou et al., (1988) Plant Physiol 87:671-4 (soybean); Finer and McMullen, (1991) In Vitro Cell Dev Biol 27P:175-82 (soybean); Singh et al., (1998) Theor Appl Genet 96:319-24 (soybean); Datta et al., (1990) Biotechnology 8:736-40 (rice); Klein et al., (1988) Proc. Natl. Acad. Sci. USA 85:4305-9 (maize); Klein et al., (1988) Biotechnology 6:559-63 (maize); U.S. Patent Nos. 5,240,855; 5,322,783 and 5,324,646; Klein et al., (1988) Plant Physiol 91:440-4 (maize); Fromm et al., (1990) Biotechnology 8:833-9 (maize); Hooykaas-Van Slogteren et al., (1984) Nature 311:763-4; U.S. Patent No. 5,736,369 (cereals); Bytebier et al., (1987) Proc. Natl. Acad. Sci. USA 84:5345-9 (*Liliaceae*); De Wet et al., (1985) in The Experimental Manipulation of Ovule Tissues, ed. Chapman et al., (Longman, New York), pp. 197-209 (pollen); Kaeppler et al., (1990) Plant Cell Rep 9:415-8) and Kaeppler et al., (1992) Theor Appl Genet 84:560-6 (whisker-mediated transformation); D'Halluin et al., (1992) Plant Cell 4:1495-505 (electroporation); Li et al., (1993) Plant Cell Rep 12:250-5; Christou and Ford (1995) Annals Botany 75:407-13 (rice) and Osjoda et al., (1996) Nat Biotechnol 14:745-50 (maize via *Agrobacterium tumefaciens).*

Alternatively, polynucleotides may be introduced into plant or plant cells by contacting cells or organisms with a virus or viral nucleic acids. Generally, such methods involve incorporating a polynucleotide within a viral DNA or RNA molecule. In some examples a polypeptide of interest may be initially synthesized as part of a viral polyprotein, which is later processed by proteolysis *in vivo* or *in vitro* to produce the desired recombinant protein. Methods for introducing polynucleotides into plants and expressing a protein encoded therein, involving viral DNA or RNA molecules, are known, see, for example, U.S. Patent Nos. 5,889,191, 5,889,190, 5,866,785, 5,589,367 and 5,316,931.

The polynucleotide or recombinant DNA construct can be provided to or introduced into a plant using a variety of transient transformation methods. Such transient transformation methods include, but are not limited to, the introduction of the polynucleotide construct directly into the plant.

Nucleic acids and proteins can be provided to a cell by any method including methods using molecules to facilitate the uptake of anyone or all components of a guided Cpf1 system (protein and/or nucleic acids), such as cell-penetrating peptides and nanocariers. See also US20110035836 Nanocarier based plant transfection and transduction, and EP 2821486 A1 Method of introducing nucleic acid into plant cells.

Other methods of introducing polynucleotides into a plant or plant part can be used, including plastid transformation methods, and the methods for introducing polynucleotides into tissues from seedlings or mature seeds.

Stable transformation is intended to mean that the nucleotide construct introduced into an organism integrates into a genome of the organism and is capable of being inherited by the progeny thereof. Transient transformation is intended to mean that a polynucleotide is introduced into the organism and does not integrate into a genome of the organism or a polypeptide is introduced into an organism. Transient transformation indicates that the introduced composition is only temporarily expressed or present in the organism.

Factors influencing *Agrobacterium* -mediated transformation of plants are well known in the art (for review see Cheng et al., 2004, In vitro Cell. Dev. Biol. Plant 40:31-45). Plant transformation and growth are typically performed at temperatures below 30 °C (room temperature). A temperature of 22°C was found to be optimal for T-DNA delivery in dicots. The optimal temperature for T-DNA delivery and for stable transformation (co-culture of plant cells and *Agrobacterium*) may vary among specific explant and Agrobacterium strain but generally ranges from 23-25°C (room temperature) (Cheng et al., 2004, In vitro Cell. Dev. Biol. Plant 40:31-45; Methods in Molecular Biology. Kang Wang Editor, Agrobacterium Protocols Volume 1-2, 2015). Transgenic maize plants have been obtained from inbred lines by co-culture of *Agrobacterium* and immature embryos at 20°C, followed by 28°C subculture (Gordon-Kamm et al., 2002, PNAS vol.99(No.18) 11975-11980).

Particle mediated bombardment of maize cells is generally followed by letting the bombarded tissue remain on cultured medium for approximately 4 hours at room temperature, followed by subculturing (transferring of tissues to new media) at temperatures between 25°C to about 28°C (US2002/0042929, published April 11, 2002)

As described, herein the Cpf1 endonuclease can be used to modify a target sequence in the genome of a plant cell or plant. In one aspect, this document features a method for modifying a target sequence in the genome of a plant cell or plant. The method can include a) introducing into a plant cell that contains a target sequence, a Cpf1 endonuclease or a plant-optimized polynucleotide encoding the Cpf1 endonuclease, and a guide polynucleotide comprising a variable targeting domain that is substantially complementary to a target sequence in the plant genome; and, b) incubating the plant cell at a temperature greater than 28°C for a period of at least about 4 hrs., where the guide polynucleotide and Cpf1 endonuclease are capable of forming a complex that can recognize, bind to, and optionally nick or cleave the target sequence. The method can also include: a) introducing a guide polynucleotide comprising a variable targeting domain that is substantially complementary to a target sequence in the plant genome into a plant cell comprising in its genome a recombinant DNA construct comprising a promoter operably linked to plant-optimized polynucleotide encoding a Cpf1 endonuclease; and, b) incubating the plant cell at a temperature greater than 28°C for a period of at least about 4 hrs., where the guide polynucleotide and Cpf1 endonuclease are capable of forming a complex that can recognize, bind to, and optionally nick or cleave the target sequence. The method can also include: a) obtaining a plant comprising a first recombinant DNA construct, the first recombinant DNA construct comprising a promoter operably linked to a plant-optimized polynucleotide encoding a Cpf1 endonuclease, and a second recombinant DNA construct, the second recombinant DNA construct comprising a promoter operably linked to a nucleotide sequence encoding guide RNA, where the guide RNA and Cpf1 endonuclease are capable of forming a complex that can recognize, bind to, and optionally nick or cleave the target sequence; and,
b) incubating the plant cell at a temperature greater than 28°C for a period of at least about 4 hrs., where the guide RNA and Cpf1 endonuclease are capable of forming a complex that can recognize, bind to, and optionally nick or cleave the target sequence.

The method can further comprise identifying at least one plant cell, plant or progeny plant that has a modification at the target sequence, where the modification at the target sequence is selected from the group consisting of (i) a replacement of at least one nucleotide, (ii) a deletion of at least one nucleotide, (iii) an insertion of at least one nucleotide, and (iv) any combination of (i) - (iii). The method of can further provide a donor DNA to the plant cell, where the donor DNA comprises a polynucleotide of interest. This can produce a plant cell or plant having a detectable targeted genome modification.

The plant cell, plant or progeny plant can be incubated at a temperature of at least 29°C, 30°C, 31°C, 32°C, 33°C, 34°C, 35°C, 36°C, 37°C, 38°C, 39°C, 40°C, 41°C, 42°C, 43°C, 44°C or 45°C for a period of at least about 10 min., 20 min., 30 min. , 40 min., 50 min., 1 hr., 2 hrs., 3 hr., 4 hrs., 5 hrs., 6 hrs., 7 hrs., 8 hrs., 9 hrs., 10 hrs., 11 hrs., 12 hrs., 13 hrs., 14 hrs., 15 hrs,16 hrs., 17 hrs,18 hrs,19 hrs., 20 hrs., 21 hrs., 22 hrs., 23 hrs., 24 hrs,25 hrs., 26 hrs., 27 hrs., 28 hrs., 29 hrs., 30 hrs., 31 hrs., 32 hrs., 33 hrs., 34 hrs., 35 hrs., 36 hrs., 37 hrs., 38 hrs,39 hrs., 40 hrs., 41 hrs,42 hrs., 43 hrs., 44 hrs., 45 hrs., 46 hrs., 47 hrs., 48 hrs. to at least 3, 4, 5, 6, 7 days, to at least 1, 2, 3, 4, 5 weeks, or to at least multiple months.

Exposure of the plant cell to the temperature greater than 28°C can be at any developmental stage of the plant cell, plant embryo and/or plant. For example, the method for modifying a target sequence in the genome of a plant can include obtaining a plant comprising at least one Cpf1 endonuclease ( or a recombinant construct expressing the Cpf1 endonuclease) and a guide RNA (and/or a recombinant construct expressing the guide RNA) comprising a variable targeting domain that is substantially complementary to a target sequence in the plant genome; and, b) incubating the plant at a temperature greater than 28°C for a period of about at least 4 hrs. at a specific stage of the plant development, such as but not limiting to incubating the plant during the developmental stage of pollen production and development. Progeny of plants comprising a Cpf1 endonuclease (or a recombinant construct expressing the Cpf1 endonuclease) and a guide RNA (and/or a recombinant construct expressing the guide RNA) can also be exposed to the increased temperature (of at least 29°C), thereby activating the guide RNA/ Cpf1 complex within the progeny plant (at a desired stage during development) which allows for the progeny plant to be modified at a specific target site that is recognized by the guide RNA/Cpf1 endonuclease complex.

Polynucleotides of interest are further described herein and include polynucleotides reflective of the commercial markets and interests of those involved in the development of the crop. Crops and markets of interest change, and as developing nations open up world markets, new crops and technologies will emerge also. In addition, as our understanding of agronomic traits and characteristics such as yield and heterosis increase, the choice of genes for genetic engineering will change accordingly.

Polynucleotides of interest include, but are not limited to, polynucleotides encoding important traits for agronomics, herbicide-resistance, insecticidal resistance, disease resistance, nematode resistance, herbicide resistance. microbial resistance, fungal resistance, viral resistance, fertility or sterility, grain characteristics, and commercial products.

General categories of polynucleotides of interest include, for example, genes of interest involved in information, such as zinc fingers, those involved in communication, such as kinases, and those involved in housekeeping, such as heat shock proteins. More specific polynucleotides of interest include, but are not limited to, genes involved in crop yield, grain quality, crop nutrient content, starch and carbohydrate quality and quantity as well as those affecting kernel size, sucrose loading, protein quality and quantity, nitrogen fixation and/or utilization, fatty acid and oil composition, genes encoding proteins conferring resistance to abiotic stress (such as drought, nitrogen, temperature, salinity, toxic metals or trace elements, or those conferring resistance to toxins such as pesticides and herbicides), genes encoding proteins conferring resistance to biotic stress (such as attacks by fungi, viruses, bacteria, insects, and nematodes, and development of diseases associated with these organisms).

Agronomically important traits such as oil, starch, and protein content can be genetically altered in addition to using traditional breeding methods. Modifications include increasing content of oleic acid, saturated and unsaturated oils, increasing levels of lysine and sulfur, providing essential amino acids, and also modification of starch. Hordothionin protein modifications are described in U.S. Patent Nos. 5,703,049, 5,885,801, 5,885,802, and 5,990,389.

Polynucleotides of interest include, any nucleotide sequence encoding a protein or polypeptide that improves desirability of crops. Polynucleotide sequences of interest may encode proteins involved in providing disease or pest resistance. By "disease resistance" or "pest resistance" is intended that the plants avoid the harmful symptoms that are the outcome of the plant-pathogen interactions. Pest resistance genes may encode resistance to pests that have great yield drag such as rootworm, cutworm, and European Corn Borer. Disease resistance and insect resistance genes such as lysozymes or cecropins for antibacterial protection, or proteins such as defensins, glucanases or chitinases for antifungal protection, or *Bacillus thuringiensis* endotoxins, protease inhibitors, collagenases, lectins, or glycosidases for controlling nematodes or insects are all examples of useful gene products. Genes encoding disease resistance traits include detoxification genes, such as against fumonisin (U.S. Patent No. 5,792,931); avirulence (avr) and disease resistance (R) genes (Jones et al. (1994) Science 266:789; Martin et al. (1993) Science 262:1432; and Mindrinos et al. (1994) Cell 78:1089). Insect resistance genes may encode resistance to pests that have great yield drag such as rootworm, cutworm, and European Corn Borer. Such genes include, for example, *Bacillus thuringiensis* toxic protein genes (U.S. Patent Nos. 5,366,892; 5,747,450; 5,736,514; 5,723,756; 5,593,881; and Geiser et al. (1986) Gene 48:109).

An "herbicide resistance protein" or a protein resulting from expression of an "herbicide resistance-encoding nucleic acid molecule" includes proteins that confer upon a cell the ability to tolerate a higher concentration of an herbicide than cells that do not express the protein, or to tolerate a certain concentration of an herbicide for a longer period of time than cells that do not express the protein. Herbicide resistance traits may be introduced into plants by genes coding for resistance to herbicides that act to inhibit the action of acetolactate synthase (ALS, also referred to as acetohydroxyacid synthase, AHAS), in particular the sulfonylurea-type (UK: sulphonylurea) herbicides, genes coding for resistance to herbicides that act to inhibit the action of glutamine synthase, such as phosphinothricin or basta (e.g., the bar gene), glyphosate (e.g., the EPSP synthase gene and the GAT gene), HPPD inhibitors (e.g, the HPPD gene) or other such genes known in the art. See, for example, US Patent Nos. 7,626,077, 5,310,667, 5,866,775, 6,225,114, 6,248,876, 7,169,970, 6,867,293, and US Provisional Application No. 61/401,456. The *bar gene* encodes resistance to the herbicide basta, the *nptII* gene encodes resistance to the antibiotics kanamycin and geneticin, and the ALS-gene mutants encode resistance to the herbicide chlorsulfuron.

Furthermore, it is recognized that the polynucleotide of interest may also comprise antisense sequences complementary to at least a portion of the messenger RNA (mRNA) for a targeted gene sequence of interest. Antisense nucleotides are constructed to hybridize with the corresponding mRNA. Modifications of the antisense sequences may be made as long as the sequences hybridize to and interfere with expression of the corresponding mRNA. In this manner, antisense constructions having 70%, 80%, or 85% sequence identity to the corresponding antisense sequences may be used. Furthermore, portions of the antisense nucleotides may be used to disrupt the expression of the target gene. Generally, sequences of at least 50 nucleotides, 100 nucleotides, 200 nucleotides, or greater may be used.

In addition, the polynucleotide of interest may also be used in the sense orientation to suppress the expression of endogenous genes in plants. Methods for suppressing gene expression in plants using polynucleotides in the sense orientation are known in the art. The methods generally involve transforming plants with a DNA construct comprising a promoter that drives expression in a plant operably linked to at least a portion of a nucleotide sequence that corresponds to the transcript of the endogenous gene. Typically, such a nucleotide sequence has substantial sequence identity to the sequence of the transcript of the endogenous gene, generally greater than about 65% sequence identity, about 85% sequence identity, or greater than about 95% sequence identity. See, U.S. Patent Nos. 5,283,184 and 5,034,323.

The polynucleotide of interest can also be a phenotypic marker. A phenotypic marker is screenable or a selectable marker that includes visual markers and selectable markers whether it is a positive or negative selectable marker. Any phenotypic marker can be used. Specifically, a selectable or screenable marker comprises a DNA segment that allows one to identify, or select for or against a molecule or a cell that contains it, often under particular conditions. These markers can encode an activity, such as, but not limited to, production of RNA, peptide, or protein, or can provide a binding site for RNA, peptides, proteins, and inorganic and organic compounds or compositions.

Examples of selectable markers include, but are not limited to, DNA segments that comprise restriction enzyme sites; DNA segments that encode products which provide resistance against otherwise toxic compounds including antibiotics, such as, spectinomycin, ampicillin, kanamycin, tetracycline, Basta, neomycin phosphotransferase II (NEO) and hygromycin phosphotransferase (HPT)); DNA segments that encode products which are otherwise lacking in the recipient cell (e.g., tRNA genes, auxotrophic markers); DNA segments that encode products which can be readily identified (e.g., phenotypic markers such as β-galactosidase, GUS; fluorescent proteins such as green fluorescent protein (GFP), cyan (CFP), yellow (YFP), red (RFP), and cell surface proteins); the generation of new primer sites for PCR (e.g., the juxtaposition of two DNA sequence not previously juxtaposed), the inclusion of DNA sequences not acted upon or acted upon by a restriction endonuclease or other DNA modifying enzyme, chemical, etc.; and, the inclusion of a DNA sequences required for a specific modification (e.g., methylation) that allows its identification.

Additional selectable markers include genes that confer resistance to herbicidal compounds, such as sulphonylureas, glufosinate ammonium, bromoxynil, imidazolinones, and 2,4-dichlorophenoxyacetate (2,4-D). See for example, Acetolactase synthase (ALS) for resistance to sulfonylureas, imidazolinones, triazolopyrimidine sulfonamides, pyrimidinylsalicylates and sulphonylaminocarbonyl-triazolinones Shaner and Singh, 1997, Herbicide Activity: Toxicol Biochem Mol Biol 69-110); glyphosate resistant 5-enolpyruvylshikimate-3-phosphate (EPSPS)(Saroha et al. 1998, J. Plant Biochemistry & Biotechnology Vol 7:65-72);

Polynucleotides of interest includes genes that can be stacked or used in combination with other traits, such as but not limited to herbicide resistance or any other trait described herein. Polynucleotides of interest and/or traits can be stacked together in a complex trait locus as described in US-2013-0263324-A1, published 03 Oct 2013 and in PCT/US13/22891, published January 24, 2013.

A polypeptide of interest includes any protein or polypeptide that is encoded by a polynucleotide of interest described herein.

Further disclosed are methods for identifying at least one plant cell, comprising in its genome, a polynucleotide of interest integrated at the target site. A variety of methods are available for identifying those plant cells with insertion into the genome at or near to the target site without using a screenable marker phenotype. Such methods can be viewed as directly analyzing a target sequence to detect any change in the target sequence, including but not limited to PCR methods, sequencing methods, nuclease digestion, Southern blots, and any combination thereof. See, for example, US Patent Application 12/147,834. The method also comprises recovering a plant from the plant cell comprising a polynucleotide of Interest integrated into its genome. The plant may be sterile or fertile. It is recognized that any polynucleotide of interest can be provided, integrated into the plant genome at the target site, and expressed in a plant.

As used herein, "nucleic acid" means a polynucleotide and includes a single or a double-stranded polymer of deoxyribonucleotide or ribonucleotide bases. Nucleic acids may also include fragments and modified nucleotides. Thus, the terms "polynucleotide", "nucleic acid sequence", "nucleotide sequence" and "nucleic acid fragment" are used interchangeably to denote a polymer of RNA and/or DNA and/or RNA-DNA polynucleotide (a polynucleotide comprising both RNA and DNA) that is single- or double-stranded, optionally containing synthetic, non-natural, or altered nucleotide bases. Nucleotides (usually found in their 5'-monophosphate form) are referred to by their single letter designation as follows: "A" for adenosine or deoxyadenosine (for RNA or DNA, respectively), "C" for cytosine or deoxycytosine, "G" for guanosine or deoxyguanosine, "U" for uridine, "T" for deoxythymidine, "R" for purines (A or G), "Y" for pyrimidines (C or T), "K" for G or T, "H" for A or C or T, "I" for inosine, and "N" for any nucleotide.

"Open reading frame" is abbreviated ORF.

The terms "fragment that is functionally equivalent" and "functionally equivalent fragment" are used interchangeably herein. These terms refer to a portion or subsequence of an isolated nucleic acid fragment or polypeptide in which the ability to alter gene expression or produce a certain phenotype is retained whether or not the fragment encodes an active enzyme. For example, the fragment can be used in the design of genes to produce the desired phenotype in a modified plant. Genes can be designed for use in suppression by linking a nucleic acid fragment, whether or not it encodes an active enzyme, in the sense or antisense orientation relative to a plant promoter sequence.

The term "conserved domain" or "motif" means a set of amino acids conserved at specific positions along an aligned sequence of evolutionarily related proteins. While amino acids at other positions can vary between homologous proteins, amino acids that are highly conserved at specific positions indicate amino acids that are essential to the structure, the stability, or the activity of a protein. Because they are identified by their high degree of conservation in aligned sequences of a family of protein homologues, they can be used as identifiers, or "signatures", to determine if a protein with a newly determined sequence belongs to a previously identified protein family.

Polynucleotide and polypeptide sequences, variants thereof, and the structural relationships of these sequences can be described by the terms "homology", "homologous", "substantially identical", "substantially similar" and "corresponding substantially" which are used interchangeably herein. These refer to polypeptide or nucleic acid sequences where changes in one or more amino acids or nucleotide bases do not affect the function of the molecule, such as the ability to mediate gene expression or to produce a certain phenotype. These terms also refer to modification(s) of nucleic acid sequences that do not substantially alter the functional properties of the resulting nucleic acid relative to the initial, unmodified nucleic acid. These modifications include deletion, substitution, and/or insertion of one or more nucleotides in the nucleic acid fragment.

Substantially similar nucleic acid sequences encompassed may be defined by their ability to hybridize (under moderately stringent conditions, e.g., 0.5X SSC, 0.1% SDS, 60°C) with the sequences exemplified herein, or to any portion of the nucleotide sequences disclosed herein and which are functionally equivalent to any of the nucleic acid sequences disclosed herein. Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions.

The term "selectively hybridizes" includes reference to hybridization, under stringent hybridization conditions, of a nucleic acid sequence to a specified nucleic acid target sequence to a detectably greater degree (e.g., at least 2-fold over background) than its hybridization to non-target nucleic acid sequences and to the substantial exclusion of non-target nucleic acids. Selectively hybridizing sequences typically have about at least 80% sequence identity, or 90% sequence identity, up to and including 100% sequence identity (i.e., fully complementary) with each other.

The term "stringent conditions" or "stringent hybridization conditions" includes reference to conditions under which a probe will selectively hybridize to its target sequence in an *in vitro* hybridization assay. Stringent conditions are sequence-dependent and will be different in different circumstances. By controlling the stringency of the hybridization and/or washing conditions, target sequences can be identified which are 100% complementary to the probe (homologous probing). Alternatively, stringency conditions can be adjusted to allow some mismatching in sequences so that lower degrees of similarity are detected (heterologous probing). Generally, a probe is less than about 1000 nucleotides in length, optionally less than 500 nucleotides in length.

Typically, stringent conditions will be those in which the salt concentration is less than about 1.5 M Na ion, typically about 0.01 to 1.0 M Na ion concentration (or other salt(s)) at pH 7.0 to 8.3, and at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C.

"Sequence identity" or "identity" in the context of nucleic acid or polypeptide sequences refers to the nucleic acid bases or amino acid residues in two sequences that are the same when aligned for maximum correspondence over a specified comparison window.

The term "percentage of sequence identity" refers to the value determined by comparing two optimally aligned sequences over a comparison window, where the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the results by 100 to yield the percentage of sequence identity. Useful examples of percent sequence identities include, but are not limited to, 50%, 55%, 60%, 65%, 70%, 75%, 80%, 85%, 90% or 95%, or any integer percentage from 50% to 100%. These identities can be determined using any of the programs described herein.

Sequence alignments and percent identity or similarity calculations may be determined using a variety of comparison methods designed to detect homologous sequences including, but not limited to, the MegAlign^{™} program of the LASERGENE bioinformatics computing suite (DNASTAR Inc., Madison, WI). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized.

The "Clustal V method of alignment" corresponds to the alignment method labeled Clustal V (described by Higgins and Sharp, (1989) CABIOS 5:151-153; Higgins et al., (1992) Comput Appl Biosci 8:189-191) and found in the MegAlign^{™} program of the LASERGENE bioinformatics computing suite (DNASTAR Inc., Madison, WI). For multiple alignments, the default values correspond to GAP PENALTY=10 and GAP LENGTH PENALTY=10. Default parameters for pairwise alignments and calculation of percent identity of protein sequences using the Clustal method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4. After alignment of the sequences using the Clustal V program, it is possible to obtain a "percent identity" by viewing the "sequence distances" table in the same program.

The "Clustal W method of alignment" corresponds to the alignment method labeled Clustal W (described by Higgins and Sharp, (1989) CABIOS 5:151-153; Higgins et al., (1992) Comput Appl Biosci 8:189-191) and found in the MegAlign^{™} v6.1 program of the LASERGENE bioinformatics computing suite (DNASTAR Inc., Madison, WI). Default parameters for multiple alignment (GAP PENALTY=10, GAP LENGTH PENALTY=0.2, Delay Divergen Seqs (%)=30, DNA Transition Weight=0.5, Protein Weight Matrix=Gonnet Series, DNA Weight Matrix=IUB ). After alignment of the sequences using the Clustal W program, it is possible to obtain a "percent identity" by viewing the "sequence distances" table in the same program.

Unless otherwise stated, sequence identity/similarity values provided herein refer to the value obtained using GAP Version 10 (GCG, Accelrys, San Diego, CA) using the following parameters: % identity and % similarity for a nucleotide sequence using a gap creation penalty weight of 50 and a gap length extension penalty weight of 3, and the nwsgapdna.cmp scoring matrix; % identity and % similarity for an amino acid sequence using a GAP creation penalty weight of 8 and a gap length extension penalty of 2, and the BLOSUM62 scoring matrix (Henikoff and Henikoff, (1989) Proc. Natl. Acad. Sci. USA 89:10915). GAP uses the algorithm of Needleman and Wunsch, (1970) J Mol Biol 48:443-53, to find an alignment of two complete sequences that maximizes the number of matches and minimizes the number of gaps. GAP considers all possible alignments and gap positions and creates the alignment with the largest number of matched bases and the fewest gaps, using a gap creation penalty and a gap extension penalty in units of matched bases.

"BLAST" is a searching algorithm provided by the National Center for Biotechnology Information (NCBI) used to find regions of similarity between biological sequences. The program compares nucleotide or protein sequences to sequence databases and calculates the statistical significance of matches to identify sequences having sufficient similarity to a query sequence such that the similarity would not be predicted to have occurred randomly. BLAST reports the identified sequences and their local alignment to the query sequence.

It is well understood by one skilled in the art that many levels of sequence identity are useful in identifying polypeptides from other species or modified naturally or synthetically where such polypeptides have the same or similar function or activity. Useful examples of percent identities include, but are not limited to, 50%, 55%, 60%, 65%, 70%, 75%, 80%, 85%, 90% or 95%, or any integer percentage from 50% to 100%. Indeed, any integer amino acid identity from 50% to 100% may be useful in describing the present disclosure, such as 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%.

"Gene" includes a nucleic acid fragment that expresses a functional molecule such as, but not limited to, a specific protein, including regulatory sequences preceding (5' non-coding sequences) and following (3' non-coding sequences) the coding sequence. "Native gene" refers to a gene as found in nature with its own regulatory sequences.

A "mutated gene" is a gene that has been altered through human intervention. Such a "mutated gene" has a sequence that differs from the sequence of the corresponding non-mutated gene by at least one nucleotide addition, deletion, or substitution. In certain aspects of the disclosure, the mutated gene comprises an alteration that results from a guide polynucleotide/Cpf1 endonuclease system as disclosed herein. A mutated plant is a plant comprising a mutated gene.

As used herein, a "targeted mutation" is a mutation in a gene (referred to as the target gene), including a native gene, that was made by altering a target sequence within the target gene using any method known to one skilled in the art, including a method involving a guided Cpf1 endonuclease system as disclosed herein.

A guide polynucleotide/Cpf1 endonuclease induced targeted mutation can occur in a nucleotide sequence that is located within or outside a genomic target site that is recognized and cleaved by the Cpf1 endonuclease.

The term "genome" as it applies to a plant cells encompasses not only chromosomal DNA found within the nucleus, but organelle DNA found within subcellular components (e.g., mitochondria, or plastid) of the cell.

An "allele" is one of several alternative forms of a gene occupying a given locus on a chromosome. When all the alleles present at a given locus on a chromosome are the same, that plant is homozygous at that locus. If the alleles present at a given locus on a chromosome differ, that plant is heterozygous at that locus.

"Coding sequence" refers to a polynucleotide sequence which codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences include, but are not limited to, promoters, translation leader sequences, 5' untranslated sequences, 3' untranslated sequences, introns, polyadenylation target sequences, RNA processing sites, effector binding sites, and stem-loop structures.

A "codon-modified gene" or "codon-preferred gene" or "codon-optimized gene" is a gene having its frequency of codon usage designed to mimic the frequency of preferred codon usage of the host cell.

"A plant-optimized nucleotide sequence" is a nucleotide sequence that has been optimized for expression in plants, particularly for increased expression in plants. A plant-optimized nucleotide sequence includes a codon-optimized gene. A plant-optimized nucleotide sequence can be synthesized by modifying a nucleotide sequence encoding a protein such as, for example, a Cpf1 endonuclease as disclosed herein, using one or more plant-preferred codons for improved expression. *See*, for example, Campbell and Gowri (1990) Plant Physiol. 92:1-11 for a discussion of host-preferred codon usage.

Methods are available in the art for synthesizing plant-preferred genes. See, for example, U.S. Patent Nos. 5,380,831, and 5,436,391, and Murray et al. (1989) Nucleic Acids Res. 17:477-498. Additional sequence modifications are known to enhance gene expression in a plant host. These include, for example, elimination of: one or more sequences encoding spurious polyadenylation signals, one or more exon-intron splice site signals, one or more transposon-like repeats, and other such well-characterized sequences that may be deleterious to gene expression. The G-C content of the sequence may be adjusted to levels average for a given plant host, as calculated by reference to known genes expressed in the host plant cell. When possible, the sequence is modified to avoid one or more predicted hairpin secondary mRNA structures. Thus, "a plant-optimized nucleotide sequence" of the present disclosure comprises one or more of such sequence modifications.

A "promoter" is a region of DNA involved in recognition and binding of RNA polymerase and other proteins to initiate transcription. The promoter sequence consists of proximal and more distal upstream elements, the latter elements often referred to as enhancers. An "enhancer" is a DNA sequence that can stimulate promoter activity, and may be an innate element of the promoter or a heterologous element inserted to enhance the level or tissue-specificity of a promoter. Promoters may be derived in their entirety from a native gene, or be composed of different elements derived from different promoters found in nature, and/or comprise synthetic DNA segments. It is understood by those skilled in the art that different promoters may direct the expression of a gene in different tissues or cell types, or at different stages of development, or in response to different environmental conditions. It is further recognized that since in most cases the exact boundaries of regulatory sequences have not been completely defined, DNA fragments of some variation may have identical promoter activity. Promoters that cause a gene to be expressed in most cell types at most times are commonly referred to as "constitutive promoters".

It has been shown that certain promoters are able to direct RNA synthesis at a higher rate than others. These are called "strong promoters". Certain other promoters have been shown to direct RNA synthesis at higher levels only in particular types of cells or tissues and are often referred to as "tissue specific promoters", or "tissue-preferred promoters" if the promoters direct RNA synthesis preferably in certain tissues but also in other tissues at reduced levels.

A plant promoter includes a promoter capable of initiating transcription in a plant cell. For a review of plant promoters, see, Potenza et al., 2004, In Vitro Cell Dev Biol 40:1-22; Porto et al., 2014, Molecular Biotechnology (2014), 56(1), 38-49.

Constitutive promoters include, for example, the core CaMV 35S promoter (Odell et al., (1985) Nature 313:810-2); rice actin (McElroy et al., (1990) Plant Cell 2:163-71); ubiquitin (Christensen et al., (1989) Plant Mol Biol 12:619-32; and ALS promoter (U.S. Patent No. 5,659,026).

Tissue-preferred promoters can be utilized to target enhanced expression within a particular plant tissue. Tissue-preferred promoters include, for example, WO2013/103367 published on 11 July 2013, Kawamata et al., (1997) Plant Cell Physiol 38:792-803; Hansen et al., (1997) Mol Gen Genet 254:337-43; Russell et al., (1997) Transgenic Res 6:157-68; Rinehart et al., (1996) Plant Physiol 112:1331-41; Van Camp et al., (1996) Plant Physiol 112:525-35; Canevascini et al., (1996) Plant Physiol 112:513-524; Lam, (1994) Results Probl Cell Differ 20:181-96; and Guevara-Garcia et al., (1993) Plant J 4:495-505. Leaf-preferred promoters include, for example, Yamamoto et al., (1997) Plant J 12:255-65; Kwon et al., (1994) Plant Physiol 105:357-67; Yamamoto et al., (1994) Plant Cell Physiol 35:773-8; Gotor et al., (1993) Plant J 3:509-18; Orozco et al., (1993) Plant Mol Biol 23:1129-38; Matsuoka et al., (1993) Proc. Natl. Acad. Sci. USA 90:9586-90; Simpson et al., (1958) EMBO J 4:2723-9; Timko et al., (1988) Nature 318:57-8. Root-preferred promoters include, for example, Hire et al., (1992) Plant Mol Biol 20:207-18 (soybean root-specific glutamine synthase gene); Miao et al., (1991) Plant Cell 3:11-22 (cytosolic glutamine synthase (GS)); Keller and Baumgartner, (1991) Plant Cell 3:1051-61 (root-specific control element in the GRP 1.8 gene of French bean); Sanger et al., (1990) Plant Mol Biol 14:433-43 (root-specific promoter of *A. tumefaciens* mannopine synthase (MAS)); Bogusz et al., (1990) Plant Cell 2:633-41 (root-specific promoters isolated from *Parasponia andersonii* and *Trema tomentosa*); Leach and Aoyagi, (1991) Plant Sci 79:69-76 (*A. rhizogenes* rolC and rolD root-inducing genes); Teeri et al., (1989) EMBO J 8:343-50 (*Agrobacterium* wound-induced TR1' and TR2' genes); VfENOD-GRP3 gene promoter (Kuster et al., (1995) Plant Mol Biol 29:759-72); and rolB promoter (Capana et al., (1994) Plant Mol Biol 25:681-91; phaseolin gene (Murai et al., (1983) Science 23:476-82; Sengopta-Gopalen et al., (1988) Proc. Natl. Acad. Sci. USA 82:3320-4). See also, U.S. Patent Nos. 5,837,876; 5,750,386; 5,633,363; 5,459,252; 5,401,836; 5,110,732 and 5,023,179.

Seed-preferred promoters include both seed-specific promoters active during seed development, as well as seed-germinating promoters active during seed germination. See, Thompson et al., (1989) BioEssays 10:108. Seed-preferred promoters include, but are not limited to, Cim1 (cytokinin-induced message); cZ19B1 (maize 19 kDa zein); and milps (myo-inositol-1-phosphate synthase); (WO00/11177; and U.S. Patent 6,225,529). For dicots, seed-preferred promoters include, but are not limited to, bean β-phaseolin, napin, β-conglycinin, soybean lectin, and cruciferin. For monocots, seed-preferred promoters include, but are not limited to, maize 15 kDa zein, 22 kDa zein, 27 kDa gamma zein, waxy, shrunken 1, shrunken 2, globulin 1, oleosin, and nuc1. See also, WO00/12733, where seed-preferred promoters from *END1* and *END2* genes are disclosed.

The term "inducible promoter" refers to a promoter that selectively express a coding sequence or functional RNA in response to the presence of an endogenous or exogenous stimulus, for example by chemical compounds (chemical inducers) or in response to environmental, hormonal, chemical, and/or developmental signals. Inducible or regulated promoters include, for example, promoters induced or regulated by light, heat, stress, flooding or drought, salt stress, osmotic stress, phytohormones, wounding, or chemicals such as ethanol, abscisic acid (ABA), jasmonate, salicylic acid, or safeners.

Chemical inducible (regulated) promoters can be used to modulate the expression of a gene in a plant through the application of an exogenous chemical regulator. The promoter may be a chemical-inducible promoter, where application of the chemical induces gene expression, or a chemical-repressible promoter, where application of the chemical represses gene expression. Chemical-inducible promoters include, but are not limited to, the maize In2-2 promoter, activated by benzene sulfonamide herbicide safeners (De Veylder et al., (1997) Plant Cell Physiol 38:568-77), the maize GST promoter (GST-II-27, WO93/01294), activated by hydrophobic electrophilic compounds used as pre-emergent herbicides, and the tobacco PR-1a promoter (Ono et al., (2004) Biosci Biotechnol Biochem 68:803-7) activated by salicylic acid. Other chemical-regulated promoters include steroid-responsive promoters (see, for example, the glucocorticoid-inducible promoter (Schena et al., (1991) Proc. Natl. Acad. Sci. USA 88:10421-5; McNellis et al., (1998) Plant J 14:247-257); tetracycline-inducible and tetracycline-repressible promoters (Gatz et al., (1991) Mol Gen Genet 227:229-37; U.S. Patent Nos. 5,814,618 and 5,789,156).

Pathogen inducible promoters induced following infection by a pathogen include, but are not limited to those regulating expression of PR proteins, SAR proteins, beta-1,3-glucanase, chitinase, *etc.*

A stress-inducible promoter includes the RD29A promoter (Kasuga et al. (1999) Nature Biotechnol. 17:287-91). One of ordinary skill in the art is familiar with protocols for simulating stress conditions such as drought, osmotic stress, salt stress and temperature stress and for evaluating stress tolerance of plants that have been subjected to simulated or naturally-occurring stress conditions.

Another example of an inducible promoter useful in plant cells is the heat inducible and/or chemical inducible promoter ZmCAS1 promoter, described in US patent application, US 2013-0312137A1, published on November 21, 2013. Other heat inducible promoters or promoters comprising or fused to heat shock elements are known in the art, such as but not limiting to, the plant heat shock promoter or elements described in US544858 and US561399.

New promoters of various types useful in plant cells are constantly being discovered; numerous examples may be found in the compilation by Okamuro and Goldberg, (1989) In The Biochemistry of Plants, Vol. 115, Stumpf and Conn, eds (New York, NY: Academic Press), pp. 1-82.

"Translation leader sequence" refers to a polynucleotide sequence located between the promoter sequence of a gene and the coding sequence. The translation leader sequence is present in the mRNA upstream of the translation start sequence. The translation leader sequence may affect processing of the primary transcript to mRNA, mRNA stability or translation efficiency. Examples of translation leader sequences have been described (e.g., Turner and Foster, (1995) Mol Biotechnol 3:225-236).

"3' non-coding sequences", "transcription terminator" or "termination sequences" refer to DNA sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al., (1989) Plant Cell 1:671-680.

"RNA transcript" refers to the product resulting from RNA polymerase-catalyzed transcription of a DNA sequence. When the RNA transcript is a perfect complimentary copy of the DNA sequence, it is referred to as the primary transcript or pre-mRNA. A RNA transcript is referred to as the mature RNA or mRNA when it is a RNA sequence derived from post-transcriptional processing of the primary transcript pre-mRNA. "Messenger RNA" or "mRNA" refers to the RNA that is without introns and that can be translated into protein by the cell. "cDNA" refers to a DNA that is complementary to, and synthesized from, an mRNA template using the enzyme reverse transcriptase. The cDNA can be single-stranded or converted into double-stranded form using the Klenow fragment of DNA polymerase I. "Sense" RNA refers to RNA transcript that includes the mRNA and can be translated into protein within a cell or *in vitro.* "Antisense RNA" refers to an RNA transcript that is complementary to all or part of a target primary transcript or mRNA, and that blocks the expression of a target gene (see, e.g., U.S. Patent No. 5,107,065). The complementarity of an antisense RNA may be with any part of the specific gene transcript, i.e., at the 5' non-coding sequence, 3' non-coding sequence, introns, or the coding sequence. "Functional RNA" refers to antisense RNA, ribozyme RNA, or other RNA that may not be translated but yet has an effect on cellular processes. The terms "complement" and "reverse complement" are used interchangeably herein with respect to mRNA transcripts, and are meant to define the antisense RNA of the message.

The term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., the coding sequence is under the transcriptional control of the promoter). Coding sequences can be operably linked to regulatory sequences in a sense or antisense orientation. In another example, the complementary RNA regions can be operably linked, either directly or indirectly, 5' to the target mRNA, or 3' to the target mRNA, or within the target mRNA, or a first complementary region is 5' and its complement is 3' to the target mRNA.

Standard recombinant DNA and molecular cloning techniques used herein are well known in the art and are described more fully in Sambrook et al., Molecular Cloning: A Laboratory Manual; Cold Spring Harbor Laboratory: Cold Spring Harbor, NY (1989). Transformation methods are well known to those skilled in the art and are described *infra.*

The term "recombinant" refers to an artificial combination of two otherwise separated segments of sequence, e.g., by chemical synthesis, or manipulation of isolated segments of nucleic acids by genetic engineering techniques.

The terms "plasmid", "vector" and "cassette" refer to a linear or circular extra chromosomal element often carrying genes that are not part of the central metabolism of the cell, and usually in the form of double-stranded DNA. Such elements may be autonomously replicating sequences, genome integrating sequences, phage, or nucleotide sequences, in linear or circular form, of a single- or double-stranded DNA or RNA, derived from any source, in which a number of nucleotide sequences have been joined or recombined into a unique construction which is capable of introducing a polynucleotide of interest into a cell. "Transformation cassette" refers to a specific vector containing a gene and having elements in addition to the gene that facilitates transformation of a particular host cell. "Expression cassette" refers to a specific vector containing a gene and having elements in addition to the gene that allow for expression of that gene in a host.

The terms "recombinant DNA molecule", "recombinant DNA construct", "expression construct", "construct", and "recombinant construct" are used interchangeably herein. A recombinant DNA construct comprises an artificial combination of nucleic acid sequences, e.g., regulatory and coding sequences that are not all found together in nature. For example, a recombinant DNA construct may comprise regulatory sequences and coding sequences that are derived from different sources, or regulatory sequences and coding sequences derived from the same source, but arranged in a manner different than that found in nature. Such a construct may be used by itself or may be used in conjunction with a vector. If a vector is used, then the choice of vector is dependent upon the method that will be used to introduce the vector into the host cells as is well known to those skilled in the art. For example, a plasmid vector can be used. The skilled artisan is well aware of the genetic elements that must be present on the vector in order to successfully transform, select and propagate host cells. The skilled artisan will also recognize that different independent transformation events may result in different levels and patterns of expression (Jones et al., (1985) EMBO J 4:2411-2418; De Almeida et al., (1989) Mol Gen Genetics 218:78-86), and thus that multiple events are typically screened in order to obtain lines displaying the desired expression level and pattern. Such screening may be accomplished standard molecular biological, biochemical, and other assays including Southern analysis of DNA, Northern analysis of mRNA expression, PCR, real time quantitative PCR (qPCR), reverse transcription PCR (RT-PCR), immunoblotting analysis of protein expression, enzyme or activity assays, and/or phenotypic analysis.

The term "expression", as used herein, refers to the production of a functional end-product (e.g., an mRNA, guide RNA, or a protein) in either precursor or mature form.

"Mature" protein refers to a post-translationally processed polypeptide (i.e., one from which any pre- or propeptides present in the primary translation product have been removed). "Precursor" protein refers to the primary product of translation of mRNA (i.e., with pre- and propeptides still present). Pre- and propeptides may be but are not limited to intracellular localization signals.

The presently disclosed guide polynucleotides, Cpf1 endonucleases, polynucleotide modification templates, donor DNAs, guide polynucleotide/Cpf1 endonuclease systems and any one combination thereof, can be introduced into a cell such as a prokaryotic or eukaryotic Cell.

Cells include, but are not limited to, human, non-human, animal, bacterial, fungal, insect, yeast, non-conventional yeast, and plant cells as well as plants and seeds produced by the methods described herein.

Any plant or plant part can be used, including monocot and dicot plants or plant part.

Examples of monocot plants that can be used include, but are not limited to, corn (*Zea mays*), rice (*Oryza sativa*), rye (*Secale cereale*), sorghum (*Sorghum bicolor, Sorghum vulgare*), millet (e.g., pearl millet (*Pennisetum glaucum*), proso millet (*Panicum miliaceum*), foxtail millet (*Setaria italica*), finger millet (*Eleusine coracana*)), wheat (*Triticum* species, *Triticum aestivum, Triticum monococcum*), sugarcane (*Saccharum spp.*), oats (*Avena*), barley (*Hordeum*), switchgrass (*Panicum virgatum*), pineapple (*Ananas comosus*), banana (*Musa spp.*), palm, ornamentals, turfgrasses, and other grasses.

The term "dicotyledonous" or "dicot" refers to the subclass of angiosperm plants also knows as "dicotyledoneae" and includes reference to whole plants, plant organs (e.g., leaves, stems, roots, etc.), seeds, plant cells, and progeny of the same. Examples of dicot plants that can be used include, but are not limited to, soybean (*Glycine max*), *Brassica* species (Canola) (*Brassica napus, B. campestris, Brassica rapa, Brassica. juncea), alfalfa (Medicago sativa),*), alfalfa (*Medicago sativa*), tobacco (*Nicotiana tabacum*), *Arabidopsis* (*Arabidopsis thaliana*), sunflower (*Helianthus annuus*), cotton (*Gossypium arboreum, Gossypium barbadense*), and peanut (*Arachis hypogaea*), tomato (*Solanum lycopersicum*), potato (*Solanum tuberosum.*

Plant that can be used include safflower (Carthamus tinctorius), sweet potato (Ipomoea batatus), cassava (Manihot esculenta), coffee (Coffea spp.), coconut (Cocos nucifera), citrus trees (Citrus spp.), cocoa (Theobroma cacao), tea (Camellia sinensis), banana (Musa spp.), avocado (Persea americana), fig (Ficus casica), guava (Psidium guajava), mango (Mangifera indica), olive (Olea europaea), papaya (Carica papaya), cashew (Anacardium occidentale), macadamia (Macadamia integrifolia), almond (Prunus amygdalus), sugar beets (Beta vulgaris), vegetables, ornamentals, and conifers.

Vegetables include tomatoes (Lycopersicon esculentum), lettuce (e.g., Lactuca sativa), green beans (Phaseolus vulgaris), lima beans (Phaseolus limensis), peas (Lathyrus spp.), and members of the genus Cucumis such as cucumber (C. sativus), cantaloupe (C. cantalupensis), and musk melon (C. melo). Ornamentals include azalea (Rhododendron spp.), hydrangea (Macrophylla hydrangea), hibiscus (Hibiscus rosasanensis), roses (Rosa spp.), tulips (Tulipa spp.), daffodils (Narcissus spp.), petunias (Petunia hybrida), carnation (Dianthus caryophyllus), poinsettia (Euphorbia pulcherrima), and chrysanthemum.

Conifers that may be employed herein include, for example, pines such as loblolly pine (Pinus taeda), slash pine (Pinus elliotii), ponderosa pine (Pinus ponderosa), lodgepole pine (Pinus contorta), and Monterey pine (Pinus radiata); Douglas fir (Pseudotsuga menziesii); Western hemlock (Tsuga canadensis); Sitka spruce (Picea glauca); redwood (Sequoia sempervirens); true firs such as silver fir (Abies amabilis) and balsam fir (Abies balsamea); and cedars such as Western red cedar (Thuja plicata) and Alaska yellow cedar (Chamaecyparis nootkatensis).

The term "plant" includes whole plants, plant organs, plant tissues, seeds, plant cells, seeds and progeny of the same. Plant cells include, without limitation, cells from seeds, suspension cultures, embryos, meristematic regions, callus tissue, leaves, roots, shoots, gametophytes, sporophytes, pollen and microspores. Plant parts include differentiated and undifferentiated tissues including, but not limited to roots, stems, shoots, leaves, pollens, seeds, tumor tissue and various forms of cells and culture (e.g., single cells, protoplasts, embryos, and callus tissue). The plant tissue may be in plant or in a plant organ, tissue or cell culture. The term "plant organ" refers to plant tissue or a group of tissues that constitute a morphologically and functionally distinct part of a plant. The term "genome" refers to the entire complement of genetic material (genes and non-coding sequences) that is present in each cell of an organism, or virus or organelle; and/or a complete set of chromosomes inherited as a (haploid) unit from one parent. "Progeny" comprises any subsequent generation of a plant.

As used herein, the term "plant part" refers to plant cells, plant protoplasts, plant cell tissue cultures from which plants can be regenerated, plant calli, plant clumps, and plant cells that are intact in plants or parts of plants such as embryos, pollen, ovules, seeds, leaves, flowers, branches, fruit, kernels, ears, cobs, husks, stalks, roots, root tips, and anthers, as well as the parts themselves. Grain is intended to mean the mature seed produced by commercial growers for purposes other than growing or reproducing the species. Progeny, variants, and mutants of the regenerated plants are also disclosed herein, provided that these parts comprise the introduced polynucleotides.

A transgenic plant includes, for example, a plant which comprises within its genome a heterologous polynucleotide introduced by a transformation step. The heterologous polynucleotide can be stably integrated within the genome such that the polynucleotide is passed on to successive generations. The heterologous polynucleotide may be integrated into the genome alone or as part of a recombinant DNA construct. A transgenic plant can also comprise more than one heterologous polynucleotide within its genome. Each heterologous polynucleotide may confer a different trait to the transgenic plant. A heterologous polynucleotide can include a sequence that originates from a foreign species, or, if from the same species, can be substantially modified from its native form. Transgenic can include any cell, cell line, callus, tissue, plant part or plant, the genotype of which has been altered by the presence of heterologous nucleic acid including those transgenics initially so altered as well as those created by sexual crosses or asexual propagation from the initial transgenic. The alterations of the genome (chromosomal or extra-chromosomal) by conventional plant breeding methods, by the genome editing procedure described herein that does not result in an insertion of a foreign polynucleotide, or by naturally occurring events such as random cross-fertilization, non-recombinant viral infection, non-recombinant bacterial transformation, non-recombinant transposition, or spontaneous mutation are not intended to be regarded as transgenic.

In certain aspects of the disclosure, a fertile plant is a plant that produces viable male and female gametes and is self-fertile. Such a self-fertile plant can produce a progeny plant without the contribution from any other plant of a gamete and the genetic material contained therein. Other aspects of the disclosure can involve the use of a plant that is not self-fertile because the plant does not produce male gametes, or female gametes, or both, that are viable or otherwise capable of fertilization. As used herein, a "male sterile plant" is a plant that does not produce male gametes that are viable or otherwise capable of fertilization. As used herein, a "female sterile plant" is a plant that does not produce female gametes that are viable or otherwise capable of fertilization. It is recognized that male-sterile and female-sterile plants can be female-fertile and male- fertile, respectively. It is further recognized that a male fertile (but female sterile) plant can produce viable progeny when crossed with a female fertile plant and that a female fertile (but male sterile) plant can produce viable progeny when crossed with a male fertile plant.

The term "crossed" or "cross" or "crossing" in the context of this disclosure means the fusion of gametes via pollination to produce progeny (i.e., cells, seeds, or plants). The term encompasses both sexual crosses (the pollination of one plant by another) and selfing (self-pollination, i.e., when the pollen and ovule (or microspores and megaspores) are from the same plant or genetically identical plants).

The term "introgression" refers to the transmission of a desired allele of a genetic locus from one genetic background to another. For example, introgression of a desired allele at a specified locus can be transmitted to at least one progeny plant via a sexual cross between two parent plants, where at least one of the parent plants has the desired allele within its genome. Alternatively, for example, transmission of an allele can occur by recombination between two donor genomes, e.g., in a fused protoplast, where at least one of the donor protoplasts has the desired allele in its genome. The desired allele can be, e.g., a transgene, a modified (mutated or edited) native allele, or a selected allele of a marker or QTL.

A "centimorgan" (cM) or "map unit" is the distance between two linked genes, markers, target sites, loci, or any pair thereof, where 1% of the products of meiosis are recombinant. Thus, a centimorgan is equivalent to a distance equal to a 1% average recombination frequency between the two linked genes, markers, target sites, loci, or any pair thereof.

The present disclosure finds use in the breeding of plants comprising one or more introduced traits.

Maize plants (Zea mays L.) can be bred by both self-pollination and cross-pollination techniques. Maize has male flowers, located on the tassel, and female flowers, located on the ear, on the same plant. It can self-pollinate ("selfing") or cross pollinate. Natural pollination occurs in maize when wind blows pollen from the tassels to the silks that protrude from the tops of the incipient ears. Pollination may be readily controlled by techniques known to those of skill in the art. The development of maize hybrids requires the development of homozygous inbred lines, the crossing of these lines, and the evaluation of the crosses. Pedigree breeding and recurrent selections are two of the breeding methods used to develop inbred lines from populations. Breeding programs combine desirable traits from two or more inbred lines or various broad-based sources into breeding pools from which new inbred lines are developed by selfing and selection of desired phenotypes. A hybrid maize variety is the cross of two such inbred lines, each of which may have one or more desirable characteristics lacked by the other or which complement the other. The new inbreds are crossed with other inbred lines and the hybrids from these crosses are evaluated to determine which have commercial potential. The hybrid progeny of the first generation is designated F1. The F1 hybrid is more vigorous than its inbred parents. This hybrid vigor, or heterosis, can be manifested in many ways, including increased vegetative growth and increased yield.

Hybrid maize seed can be produced by a male sterility system incorporating manual detasseling. To produce hybrid seed, the male tassel is removed from the growing female inbred parent, which can be planted in various alternating row patterns with the male inbred parent. Consequently, providing that there is sufficient isolation from sources of foreign maize pollen, the ears of the female inbred will be fertilized only with pollen from the male inbred. The resulting seed is therefore hybrid (F1) and will form hybrid plants.

Field variation impacting plant development can result in plants tasseling after manual detasseling of the female parent is completed. Or, a female inbred plant tassel may not be completely removed during the detasseling process. In any event, the result is that the female plant will successfully shed pollen and some female plants will be self-pollinated. This will result in seed of the female inbred being harvested along with the hybrid seed which is normally produced. Female inbred seed does not exhibit heterosis and therefore is not as productive as F1 seed. In addition, the presence of female inbred seed can represent a germplasm security risk for the company producing the hybrid.

Alternatively, the female inbred can be mechanically detasseled by machine. Mechanical detasseling is approximately as reliable as hand detasseling, but is faster and less costly. However, most detasseling machines produce more damage to the plants than hand detasseling. Thus, no form of detasseling is presently entirely satisfactory, and a need continues to exist for alternatives which further reduce production costs and to eliminate self-pollination of the female parent in the production of hybrid seed.

A regulated (temperature inducible) guide RNA/Cpf1 system mediating gene targeting can be used in methods for directing gene insertion and / or for producing complex trait loci comprising multiple genes (traits) in a fashion similar as disclosed in WO2013/0198888 (published August 1, 2013) where instead of using a double strand break inducing agent to introduce a gene of interest, a regulated guide RNA/Cpf1 system as disclosed herein is used. In one aspect of the disclosure, a complex transgenic trait locus is a genomic locus that has multiple genes of interest genetically linked to each other. By inserting independent genes within 0.1, 0.2, 0.3, 04, 0.5 ,1, 2, or even 5 centimorgans (cM) from each other, the genes can be bred as a single genetic locus (see, for example, U.S. patent application 13/427,138 or PCT application PCT/US2012/030061). After selecting a plant comprising a gene of interest such as a transgene, plants containing (at least) one gene(s) of interest can be crossed to form an F1 that contains both genes of interest. In progeny from these F1 (F2 or BC1) 1/500 progeny would have the two different genes recombined onto the same chromosome. The complex locus can then be bred as single genetic locus with both gene of interests (both traits). This process can be repeated to stack as many traits as desired.

Mutations that cause male sterility in plants have the potential to be useful in methods for hybrid seed production for crop plants such as maize and can lower production costs by eliminating the need for the labor-intensive removal of male flowers (also known as de-tasseling) from the maternal parent plants used as a hybrid parent. Examples of genes used in such ways include male fertility genes such as MS26 (see for example U.S. Patents 7,098,388, 7,517,975, 7,612,251), MS45 (see for example U.S. Patents 5,478,369, 6,265,640) or MSCA1 (see for example U.S. Patent 7,919,676).

Mutations that cause male sterility in maize have been produced by a variety of methods such as X-rays or UV-irradiations, chemical treatments, or transposable element insertions (ms23, ms25, ms26, ms32) (Chaubal et al. (2000) Am J Bot 87:1193-1201). Conditional regulation of fertility genes through fertility/sterility "molecular switches" could enhance the options for designing new male-sterility systems for crop improvement (Unger et al. (2002) Transgenic Res 11:455-465).

Chromosomal intervals that correlate with a phenotype or trait of interest can be identified. A variety of methods well known in the art are available for identifying chromosomal intervals. The boundaries of such chromosomal intervals are drawn to encompass markers that will be linked to the gene controlling the trait of interest. In other words, the chromosomal interval is drawn such that any marker that lies within that interval (including the terminal markers that define the boundaries of the interval) can be used as a marker for northern leaf blight resistance. In one aspect of the disclosure, the chromosomal interval comprises at least one QTL, and furthermore, may indeed comprise more than one QTL. Close proximity of multiple QTLs in the same interval may obfuscate the correlation of a particular marker with a particular QTL, as one marker may demonstrate linkage to more than one QTL. Conversely, e.g., if two markers in close proximity show co-segregation with the desired phenotypic trait, it is sometimes unclear if each of those markers identifies the same QTL or two different QTL. The term "quantitative trait locus" or "QTL" refers to a region of DNA that is associated with the differential expression of a quantitative phenotypic trait in at least one genetic background, e.g., in at least one breeding population. The region of the QTL encompasses or is closely linked to the gene or genes that affect the trait in question. An "allele of a QTL" can comprise multiple genes or other genetic factors within a contiguous genomic region or linkage group, such as a haplotype. An allele of a QTL can denote a haplotype within a specified window where the window is a contiguous genomic region that can be defined, and tracked, with a set of one or more polymorphic markers. A haplotype can be defined by the unique fingerprint of alleles at each marker within the specified window.

A variety of methods are available to identify those cells having an altered genome at or near a target site without using a screenable marker phenotype. Such methods can be viewed as directly analyzing a target sequence to detect any change in the target sequence, including but not limited to PCR methods, sequencing methods, nuclease digestion, Southern blots, and any combination thereof.

Standard DNA isolation, purification, molecular cloning, vector construction, and verification/characterization methods, protein modification/aleration are well established, see, for example Sambrook et al., (1989) Molecular Cloning: A Laboratory Manual, (Cold Spring Harbor Laboratory Press, NY). Vectors and constructs include circular plasmids, and linear polynucleotides, comprising a polynucleotide of interest and optionally other components including linkers, adapters, regulatory or analysis. In some examples a recognition site and/or target site can be contained within an intron, coding sequence, 5' UTRs, 3' UTRs, and/or regulatory regions.

The meaning of abbreviations is as follows: "sec" means second(s), "min" means minute(s), "h" means hour(s), "d" means day(s), "µL" means microliter(s), "mL" means milliliter(s), "L" means liter(s), "µM" means micromolar, "mM" means millimolar, "M" means molar, "mmol" means millimole(s), "µmole" mean micromole(s), "g" means gram(s), "µg" means microgram(s), "ng" means nanogram(s), "U" means unit(s), "bp" means base pair(s) and "kb" means kilobase(s).

### EXAMPLES

In the following Examples, unless otherwise stated, parts and percentages are by weight and degrees are Celsius. It should be understood that these Examples are given by way of illustration only.

### EXAMPLE 1

### Maize optimized Cpf1 endonuclease and guide polynucleotide expression cassettes for genome modification in maize plants

To confer efficient expression in maize cells, the Cpf1 endonuclease gene from *Acidaminococcus* (SEQ ID NO: 1) was maize codon optimized per standard techniques known in the art and the potato ST-LS1 intron 2 (SEQ ID NO: 2) was introduced in order to eliminate its expression in *E. coli* and *Agrobacterium.* To facilitate nuclear localization of the maize optimized Cpf1 endonuclease protein in maize cells, a nucleotide sequence encoding the *Simian virus* 40 (SV40) monopartite nuclear localization signal (SRADPKKKRKV, SEQ ID NO: 3) was added to the 3' end resulting in SEQ ID NO: 4. The nucleotide sequence of SEQ ID NO: 4, encoding the maize optimized *Acidaminococcus* Cpf1 endonuclease gene and nuclear localization signal, was then operably linked to a maize ubiquitin promoter (SEQ ID NO: 12), maize ubiquitin 5' untranslated region (UTR) (SEQ ID NO: 13), maize ubiquitin intron 1 (SEQ ID NO: 14) and suitable terminator by standard molecular biological techniques. The key components of the resulting maize optimized Cpf1 expression cassette are illustrated in Figure 1.

The Cpf1 endonuclease is directed by small RNAs (referred to herein as guide RNAs) to cleave double-stranded DNA. These guide RNAs are comprised of a ~20 nt 5' sequence (SEQ ID NO: 5) that aids recognition by Cpf1 (referred to as Cpf1 recognition domain) followed by a ~23 nt sequence that serves to direct Cpf1 cleavage by base pairing with one strand of the DNA target site (Cpf1 variable targeting domain). To transcribe small RNAs necessary for directing Cpf1 endonuclease cleavage activity in maize cells, a U6 polymerase III promoter (SEQ ID NO: 6) and terminator (TTTTTTTT) were isolated from maize and operably fused to the ends of DNA sequences that upon transcription would result in a suitable guide RNA for Cpf1. To promote optimal transcription of the guide RNA from the maize U6 polymerase III promoter a G nucleotide was added to the 5' end of the sequence to be transcribed. The key components of the resulting maize optimized Cpf1 small RNA transcriptional cassette generated here are illustrated in Figure 2. The maize optimized Cpf1 guide RNA transcribed from this cassette is as listed in SEQ ID NO: 15 (Ns represent the variable targeting domain where any nucleotide may be present).

### EXAMPLE 2

### The maize optimized Cpf1/quide polynucleotide system from Acidaminococcus may be used to direct cleavage of maize chromosomal DNA

To test whether the maize optimized *Acidaminococcus* Cpf1 endonuclease (SEQ ID NO:11) and guide polynucleotide system described in Example 1 could be used to recognize, cleave, and mutate maize chromosomal DNA, three different genomic target sequences (Table 2) were targeted for cleavage and examined by deep sequencing for the presence of mutations indicative of cleavage.

**Table 2. Acidaminococcus Cpf1 Maize Genomic Target Sequences.**

| Location | Target Site Designation | Target Site | | Target Site SEQ ID NO: |
|---|---|---|---|---|
| | | PAM | 23 nts 3' of the PAM | |
| Chr1: 51.81 cM | AsMS26-1 | TTTC | ATTCATATGGTGTC CCTGACCTA | 7 |
| Chr2: 28.45 cM | AsLIGULELESS-1 | TTTA | CTCTTAGCTCATCA CCTATGTGG | 8 |
| Chr9: 119.15cM | AsMS45-1 | TTTG | GGAACACCAGCCC CTTGAGCACG | 9 |

Target sites with an appropriate protospacer adjacent motif (PAM) (TTTN) for *Acidaminococcus* Cpf1 and approximately 23 nts 3' of the PAM were selected (Table 2). The DNA sequence 3' of the PAM for each target was then separately introduced into the region of the maize optimized small RNA transcriptional cassette encoding for the variable targeting domain described in Example 1 per standard molecular biology techniques so that upon transcription each cassette produced a guide RNA (SEQ ID NOs:16-18) that would form a guide RNA/Cpf1 endonuclease complex capable of directing the Cpf1 endonuclease to its target site. Each of the resulting guide RNA transcriptional cassettes were then separately co-delivered with the maize optimized Cpf1 endonuclease expression cassette described in Example 1 by biolistic transformation into Hi-Type II 10-day-old immature maize embryos (IMEs) in the presence of BBM and WUS2 genes as described in Svitashev et al. (2015) Plant Physiology. 169:931-945. To compare the maize optimized Cpf1/guide RNA system described in Example 1 with the maize optimized *Streptococcus pyogenes* (Spy) Cas9/guide RNA system described previously (Svitashev et al. (2015)), a target site was selected to cleave within each of the AsMS26-1, AsLIGULELESS-1 and AsMS45-1 target site regions (referred to as SpyMS26-1 (SEQ ID NO: 34), SpyLIGULELESS-1 (SEQ ID NO: 26), SpyMS45-1 (SEQ ID NO: 35), respectively). Spy Cas9 guide RNA expression vectors were then constructed and co-delivered along with the Spy Cas9 endonuclease expression vector and BBM and WUS2 genes by biolistic transformation as described above for Cpf1. Since particle gun transformation can be highly variable, a visual marker DNA expression cassette encoding a yellow fluorescent protein was also co-delivered to aid in the selection of evenly transformed IMEs and each treatment was performed in triplicate. To determine the plant transformation culture conditions optimal for guide RNA/Cpf1 mutational activity, transformed IMEs were incubated at 28°C for 48 hrs., standard for particle gun transformation, and at an elevated temperature, 37°C for 48 hrs., immediately following transformation. After 2 days, the 20-30 most evenly transformed IMEs were harvested based on their fluorescence. Total genomic DNA was extracted and the region surrounding the intended target site was PCR amplified with Phusion^{®} HighFidelity PCR Master Mix (New England Biolabs, M0531L) adding on the sequences necessary for amplicon-specific barcodes and Illumnia sequencing using "tailed" primers through two rounds of PCR and deep sequenced similar to that described in Karvelis *et al.* (2015) (Methods Section: *In planta* mutation detection). The resulting reads were examined for the presence of mutations at the expected site of cleavage by comparison to control experiments where the small RNA transcriptional cassette was omitted from the transformation as described in Karvelis *et al.* (2015) except the 24 most prevalent mutation types were used to calculate the overall mutation frequency.

All 3 sites targeted for cleavage with the guided polynucleotide/Cpf1 system described herein exhibited the presence of mutations in the form of small insertions or deletions (indels) indicative of chromosomal cleavage and repair. Unexpectedly, as shown in Table 3, the frequency of mutations at these target sites was the greatest in the experiment where plant cells were incubated at 37°C. This is in stark contrast to that observed for the *S. pyogenes* Cas9/guide polynuclotide system where the difference in chromosomal DNA target site mutation frequency between the 28°C and 37°C temperature treatments was negligible (Table 3). The fold increase in the mutation frequency at 37°C relative to the 28°C temperature treatment (calculated by dividing the Mutation Frequency at the 37°C Temperature Treatment with the Mutation Frequency at 28°C Temperature Treatment) ranged from approximately 2 fold for target sequence AsLIGULELESS-1, to about 15 fold for AsMS26-1 (Table 3).

**Table 3. Frequency of indels at 3 maize sites under different embryo temperature incubations following transformation for both the AsCpf1/quide polynucleotide system and the Spy Cas9/guide polynucleotide system.**

| Particle Gun Transformation Experiment | Maize Target(s) Assayed for Mutations | Average Mutation Frequency at 28°C | Average Mutation Frequency at 37°C | Fold Increase in Mutation Frequency at 37°C |
|---|---|---|---|---|
| *Acidaminococcus* Cpf1 Only (Negative Control) | AsMS26-1 (SEQ ID NO: 7), AsLIGULELESS-1(SEQ ID NO: 8), AsMS45-1 (SEQ ID NO: 9) | 0.00% | 0.00% | 0.0 |
| *Acidaminococcus* Cpf1 and guide RNA | AsMS26-1 (SEQ ID NO: 7) | 0.03% | 0.44% | 14.6 |
| *Acidaminococcus* Cpf1 and guide RNA | AsLIGULELESS-1(SEQ ID NO: 8) | 0.35% | 0.80% | 2.3 |
| *Acidaminococcus* Cpf1 and guide RNA | AsMS45-1 (SEQ ID NO: 9) | 0.18% | 0.80% | 4.4 |
| *S. pyogenes* Cas9 Only (Negative Control) | SpyMS26-1 (SEQ ID NO: 34), SpyLIGULELESS-1(SEQ ID NO: 26), SpyMS45-1 (SEQ ID NO: 35) | 0.00% | 0.00% | 0.0 |
| *S. pyogenes* Cas9 and guide RNA | SpyMS26-1 (SEQ ID NO: 34) | 0.84% | 1.3% | 1.5 |
| *S. pyogenes* Cas9 and guide RNA | SpyLIGULELESS-1(SEQ ID NO: 26) | 1.02% | 1.39% | 1.4 |
| *S. pyogenes* Cas9 and guide RNA | SpyMS45-1 (SEQ ID NO: 35) | 0.8% | 0.87% | 1.1 |

To further define the optimal plant tissue culture conditions for the AsCpf1/guide RNA system described herein, IMEs biolistically transformed with the maize optimized AsCpf1 and AsMS26-1 guide RNA expression cassettes described herein were incubated for 2 days at either 25°C, 28°C, 31°C, 34°C, 37°C, 40°C or 43°C. The frequency of indel mutations resulting from chromosomal DNA target site cleavage and imperfect cellular DNA repair was then assayed by deep sequencing as described above. As shown in Table 4, temperatures around 37°C (e.g. 34°C, 37°C and 40°C) produced the highest frequency of DNA target site indels while temperatures outside of this range accumulated significantly lower frequencies of indels. The fold decrease in mutation frequency relative to the 37°C temperature treatment (calculated by dividing the Mutation Frequency at the 37°C Temperature Treatment with the Mutation Frequency at a Different Temperature Treatment) ranged from 1.0 to 154.5 fold at the 40°C and 25°C temperature treatments, respectively (Table 4).

**Table 4. Effect of plant tissue culture temperature on the mutational activity of the AsCpf1/quide polynucleotide system described herein.**

| Particle Gun Transformation Experiment | Maize Target Assayed for Mutations | Plant Tissue Culture Temperature | Average Mutation Frequency | Fold Decrease in Mutation Frequency Compared to 37°C Treatment |
|---|---|---|---|---|
| *Acidaminococcus* Cpf1 and guide RNA | AsMS26-1 (SEQ ID NO: 7) | 25°C | 0.00% | 153.5 |
| *Acidaminococcus* Cpf1 and guide RNA | AsMS26-1 (SEQ ID NO: 7) | 28°C | 0.03% | 10.8 |
| *Acidaminococcus* Cpf1 and guide RNA | AsMS26-1 (SEQ ID NO: 7) | 31°C | 0.09% | 3.9 |
| *Acidaminococcus* Cpf1 and guide RNA | AsMS26-1 (SEQ ID NO: 7) | 34°C | 0.28% | 1.2 |
| *Acidaminococcus* Cpf1 and guide RNA | AsMS26-1 (SEQ ID NO: 7) | 37°C | 0.33% | 1.0 |
| *Acidaminococcus* Cpf1 and guide RNA | AsMS26-1 (SEQ ID NO: 7) | 40°C | 0.32% | 1.0 |
| *Acidaminococcus* Cpf1 and guide RNA | AsMS26-1 (SEQ ID NO: 7) | 43°C | 0.05% | 6.4 |

Next, the temperature sensitivity of the guide RNA/Cpf1 complex described herein was further confirmed by examining its activity when paired with two different plant promoters, the Banana Streak Virus (BSV) (SEQ ID NO: 19) and the Phospholipid Transfer Protein (PLTP) (SEQ ID NO: 20) promoters. For optimal expression, the BSV promoter was paired with HPLV9 intron 1 (SEQ ID NO: 21) while the PLTP promoter was combined with the 5' untranslated region (UTR) from the PLTP gene (SEQ ID NO: 22). The new promoter and intron or 5' UTR combinations were operably linked to the maize codon optimized intron and NLS containing AsCpf1 gene (SEQ ID NO: 4) and suitable terminator by methods known in the art. The resulting maize optimized Cpf1 expression cassettes are illustrated in Figures 3 and 4. Next, the effect of temperature on the ability of AsCpf1 and its cognate guide RNA to cleave maize chromosomal DNA and induce targeted mutations was assessed as described above at the AsMS26-1 target site (SEQ ID NO: 7). As shown in Table 5, the frequency of mutagenesis indicative of AsCpf1 cleavage activity was temperature dependent irrespective of the maize promoter used.

**Table 5. Frequency of indels with BSV and PLTP maize promoters under different embryo temperature regimens following transformation.**

| Promoter Driving *Acidaminococcus* Cpf1 expression | Maize Target Assayed for Mutations | Average Mutation Frequency at 28°C | Average Mutation Frequency at 37°C | Fold Increase in Mutation Frequency at 37°C |
|---|---|---|---|---|
| Banana Streak Virus (BSV) | AsMS26-1 (SEQ ID NO: 7) | 0.01% | 0.37% | 37 |
| Phospholipid Transfer Protein (PLTP) | AsMS26-1(SEQ ID NO: 7) | 0.02% | 0.32% | 16 |

Taken together, these data indicate that the Cpf1 endonuclease and guide polynucleotide system described herein for *Acidaminococcus* may be used to target maize chromosomal DNA for cleavage and be utilized as a tool to bind, nick, or cleave plant chromosomal DNA for the purposes of plant genomic and gene manipulation. Furthermore, plant transformation and growth conditions requiring incubations at elevated temperatures (>28°C) are needed for optimal activity of the maize optimized *Acidaminococcus* guide polynucleotide/Cpf1 system described herein.

### EXAMPLE 3

### Orthologous maize optimized Cpf1/quide polynucleotide systems may be used to direct cleavage of maize chromosomal DNA

To investigate the activity of orthologous Cpf1/guide polynucleotide systems in plant cells, the Cpf1 endonucleases and small guide RNAs from *Fransincella novicida* U112 (Fn) and *Lachnospiraceae bacterium* ND2006 (Lb) were optimized for use in maize as described in Example 1. Briefly, the bacterial sequences (SEQ ID NOs: 30 and 31) encoding the Fn and Lb Cpf1 endonucleases (SEQ ID NOs: 32 and 33) were maize codon optimized. Next, the potato ST-LS1 Intron 2 was inserted (SEQ ID NO: 2) and a sequence encoding a *Simian virus* 40 (SV40) nuclear localization signal (NLS) (SRADPKKKRKV, SEQ ID NO: 3) was appended to the 3' end of the genes resulting in SEQ ID NOs: 36 and 37. Next, the maize optimized genes were assembled into a plant expression cassette utilizing a maize ubiquitin promoter (SEQ ID NO: 12), maize ubiquitin 5' untranslated region (UTR) (SEQ ID NO: 13), maize ubiquitin intron 1 (SEQ ID NO: 14) and suitable terminator by standard molecular biological techniques as described in Example 1 and illustrated in Figure 1. To transcribe small RNAs capable of directing Cpf1 endonuclease cleavage activity in maize cells, guide RNA transcriptional cassettes were assembled as described in Example 1 and illustrated in Figure 2 except the region encoding the Cpf1 endonuclease recognition domain was modified to contain sequences specific to the Cpf1/guide polynucleotide systems of Fn and Lb (Table 6). The maize optimized Fn and LbCpf1 guide RNAs transcribed from this cassette are as listed in Table 6 (Ns represent the variable targeting domain where any nucleotide may be present).

**Table 6. Francisella novicida U112 and Lachnospiraceae bacterium ND2006 maize optimized Cpf1 guide polynucleotides.**

| Cpf1 Origin | Maize Optimized Guide RNA | | | |
|---|---|---|---|---|
| | G Nucleotide to Promote Robust U6 Expression | Cpf1 Recognition Sequence | Variable Targeting Domain (SEQ ID NO: 50) | Transcribed Guide RNA |
| *F. novicidia* | G | SEQ ID NO: 38 | NNNNNNNNNNNNNNNNN NNNNNN | SEQ ID NO: 40 |
| *L. bacterium* | G | SEQ ID NO: 39 | NNNNNNNNNNNNNNNNN NNNNNN | SEQ ID NO: 41 |

Since PAM recognition for FnCpf1 (TTN) and LbCpf1 (TTTN) overlap with the PAM recognition of AsCpf1 (TTTN), the AsMS26-1 target sequence (SEQ ID NO: 7) was choosen to evaluate the activity of the FnCpf1 and LbCpf1 guide polynucleotide systems. Next, the DNA sequence 3' of the PAM for AsMS26-1 (Table 2) was introduced into the variable targeting domain of the maize optimized small RNA transcriptional cassettes described above per standard molecular biology techniques so that upon transcription each cassette produced a guide RNA (SEQ ID NOs: 42 and 43) that is capable of forming a guide RNA/Cpf1 endonuclease complex that may direct maize chromosomal DNA target site cleavage.

Next, Cpf1 endonuclease and guide RNA expression cassettes (appriopriately paired together (e.g. FnCpf1 endonuclease and corresponding FnCpf1 guide RNA expression cassettes were used together)) were biolistically co-transformed into IMEs and assayed for insertion or deletion (indel) mutations indicative of chromosomal DNA target site cleavage and imperfect cellular DNA repair as described above. Particle gun transformations where the guide RNA transcriptional cassette was omitted served as a negative control. Transformations where AsCpf1 endonuclease and AsMS26-1 expression cassettes were co-delivered served as a positive control.

As shown in Table 7, both FnCpf1 and LbCpf1 guide polynucleotide systems generated indel mutations indicitative of DNA target site cleavage and imperfect cellular repair. Additionally, orthologous Cpf1 systems exhibited a response to elevated temperatures similar to that observed for the AsCpf1/guide polynucleotide system described herein. The fold increase in the mutation frequency observed for the 37°C temperature treatment (calculated by dividing the Mutation Frequency at the 37°C Temperature Treatment with the Mutation Frequency at the 28°C Temperature Treatment) ranged from nearly 2 fold for the LbCpf1 guide polynucleotide system to greater than 5 fold for the FnCpf1 guide polynucleotide system, respectively (Table 7).

**Table 7. Frequency of indels at a maize target site following transformation of maize optimized Cpf1 guide polynucleotide systems from Francisella novicida U112 and Lachnospiraceae bacterium ND2006.**

| Particle Gun Transformation Experiment | Maize Target Assayed for Mutations | Average Mutation Frequency at 28°C | Average Mutation Frequency at 37°C | Fold Increase in Mutation Frequency at 37°C |
|---|---|---|---|---|
| *Acidaminococcus* Cpf1 Only (Negative Control) | AsMS26-1 (SEQ ID NO: 7) | 0.00% | 0.00% | 0.0 |
| *F. novicidia* Cpf1 Only (Negative Control) | AsMS26-1 (SEQ ID NO: 7) | 0.00% | 0.00% | 0.0 |
| *L. bacterium* Cpf1 Only (Negative Control) | AsMS26-1 (SEQ ID NO: 7) | 0.00% | 0.00% | 0.0 |
| *Acidaminococcus* Cpf1 and guide RNA | AsMS26-1 (SEQ ID NO: 7) | 0.01% | 0.2% | 20.0 |
| *F. novicidia* Cpf1 and guide RNA | AsMS26-1 (SEQ ID NO: 7) | 0.05% | 0.27% | 5.4 |
| *L. bacterium* Cpf1 and guide RNA | AsMS26-1 (SEQ ID NO: 7) | 0.29% | 0.56% | 1.9 |

Taken together, these data indicate that orthologous Cpf1 endonuclease and guide polynucleotide systems described herein may be used to target maize chromosomal DNA for cleavage and be utilized as a tool to bind, nick, or cleave plant chromosomal DNA for the purposes of plant genomic and gene manipulation. Furthermore, plant transformation and growth conditions requiring incubations at elevated temperatures (>28°C) are needed for optimal activity of the guide polynucleotide/Cpf1 systems described herein.

### EXAMPLE 4

### The maize optimized Cpf1 endonuclease and guide polynucleotide systems, supplemented with polynucleotide modification templates, may be used to precisely modify maize chromosomal DNA

Cpf1 cleaves its DNA target site in a region with high tolerance for mismatches between the guide RNA and complementary DNA target (approximately positions +20 to +24 3' of the PAM) (Figure 5A, Kim, D. et al. (2016) Nature Biotechnology. 34863-868; Kleinstiver, B. et al. (2016) Nature Biotechnology. 34, 869-874). This is distinct from other RNA guided endonucleases (e.g. Cas9 from *Streptococcus pyogenes*) which cleave their DNA target site in regions with low tolerance for guide RNA and target DNA mismatches (approximately positions +3 to +4 5' of the PAM) (Figure 5B, Jinek, M. et al. (2012) Science. 337, 816-821). This makes the use of homology directed polynucleotide modification templates (DNA repair templates) difficult as desired alterations typically introduced directly at the site of cleavage may still be cleaved by Cpf1. To overcome this, polynucleotide modification templates were tailored to the specificity of the *Acidaminococcus* (As) Cpf1 polynucleotide guided system. This was accomplished by introducing base alterations in the polynucleotide modification template in the region of the AsCpf1 target site with low tolerance for mismatches between the guide RNA and complementary DNA target strand (positions +1 to +19 from the PAM) (Figure 5A) adjacent to the site of cleavage. In total, three types of polynucleotide modification templates were designed. The first incorporated a two base difference (positions +15 and +16 from the PAM) (DNA repair template-1, SEQ ID NO: 23) in the region 5' of that cleaved in the AsLIGULELESS-1 target site. The second repair template design included 5 base differences (positions +14 to +20 from the PAM) (DNA repair template-2, SEQ ID NO: 24) relative to the AsLIGULELESS-1 target site. And the third repair template built upon the first and included a two base difference (positions +15 and +16 from the PAM) in addition to a 9 base insertion between positions +21 and +22 from the PAM (DNA repair template-3, SEQ ID NO: 25). To promote homology directed repair (HDR), all polynucleotide modification templates were designed to have homology with the DNA region flanking the AsLIGULELESS-1 target site. This varied slightly depending on the polynucleotide modification template design and was 65-66 base pairs for the 5' homology flanking region and 79-83 base pairs for the 3 prime homology flanking region.

To test the efficiency of the designed DNA templates to direct repair of the AsLIGULELESS-1 Cpf1 cleaved chromosomal DNA target site, the maize optimized AsCpf1 and guide polynucleotide described in Example 1 were co-delivered with 20 ng of each double stranded polynucleotide modification template into immature maize embryos by particle gun biolistic transformation as described in Example 2 and the frequency of HDR compared with the Cas9 and guide polynucleotide system from *Streptococcus pyogenes* (Spy) Cas9 (as described in Svitashev *et al.* (2015)). To enable a more precise comparison between the two RNA guided endonucleases, a Spy Cas9 target site (SpyLIGULELESS-1) (SEQ ID NO: 26) was selected to cleave within the AsLIGULELESS-1 target site region cleaved by AsCpf1 and the same repair templates designed for AsCpf1 were co-delivered with the Spy Cas9 guide polynucleotide system into immature maize embryos by biolistic transformation. Particle gun transformations where the guide RNA transcriptional cassette was omitted served as a negative control. As described in Example 2, embryos were incubated at 37°C for 48 hours following transformation to induce optimal AsCpf1 activity. Following temperature treatment, equally transformed embryos were harvested based on fluorescence of the co-delivered color marker gene. Then, genomic DNA was isolated and PCR and deep sequencing carried-out as described in Example 2. Finally, HDR was detected by examining the resulting sequence reads for incorporation of the changes specified in the polynucleotide modification templates. To further normalize for differences in transformation efficiencies, the frequency of HDR was calculated by dividing the total number of mutant sequence reads resulting from non-homologous end joining (NHEJ) repair of the DSB by the number of HDR reads. As shown in Table 8, all three repair templates exhibited a similar ratio of NHEJ to HDR reads to that produced by the Spy Cas9.

Taken together, these data indicate that the Cpf1 endonuclease and guide polynucleotide system described herein, supplemented with the Cpf1 customized polynucleotide modification templates as described herein, may be used to precisely modify maize chromosomal DNA.

**Table 8. Frequency of NHEJ and HDR outcomes at the maize chromosomal AsLIGULELESS-1 target site.**

| Particle Gun Transformation Experiment | Maize Target Assayed for Mutations | Polynucleotide Modification Templates | Average Mutation Frequency | Average HDR Frequency | Mutant-HDR Reads Ratio |
|---|---|---|---|---|---|
| *Acidaminococcus* Cpf1 Only (Negative Control) | AsLIGULEL ESS-1(SEQ ID NO: 8) | DNA repair template-1, 2 and 3 | 0.00% | 0.00% | 0.0 |
| *Acidaminococcus* Cpf1 and Guide RNA | AsLIGULEL ESS-1(SEQ ID NO: 8) | DNA repair template-1 | 0.60% | 0.014% | 42.86 |
| *Acidaminococcus* Cpf1 and Guide RNA | AsLIGULEL ESS-1(SEQ ID NO: 8) | DNA repair template-2 | 0.57% | 0.012% | 47.5 |
| *Acidaminococcus* Cpf1 and Guide RNA | AsLIGULEL ESS-1(SEQ ID NO: 8) | DNA repair template-3 | 0.66% | 0.014% | 47.14 |
| *S. pyogenes* Cas9 Only (Negative Control) | AsLIGULEL ESS-1(SEQ ID NO: 8) | DNA repair template-1, 2 and 3 | 0.00% | 0.00% | 0.0 |
| *S. pyogenes* Cas9 and Guide RNA | SpyLIGULE LESS-1(SEQ ID NO: 26) | DNA repair template-1 | 1.08% | 0.031% | 34.84 |
| *S. pyogenes* Cas9 and Guide RNA | SpyLIGULE LESS-1(SEQ ID NO: 26) | DNA repair template-2 | 0.93% | 0.028% | 33.21 |
| *S. pyogenes* Cas9 and Guide RNA | SpyLIGULE LESS-1(SEQ ID NO: 26) | DNA repair template-3 | 1.16% | 0.029% | 40 |

### EXAMPLE 5

### Maize optimized Cpf1 endonuclease and guide polynucleotide systems may be used to simultaneously modify multiple maize chromosomal DNA target sites

To examine the ability of the maize optimized Cpf1 endonuclease and guide polynucleotide systems described herein to simultaneously introduce double-strand breaks (DSBs) into maize chromosomal DNA, Cpf1 guide RNAs targeting the AsLIGULELESS-1, AsMS45-1 and AsMS26-1 loci were transcribed from a single promoter and examined by deep sequencing for the presence of mutations indicative of cleavage.

Guide RNA transcriptional cassettes were designed in a way to produce a single RNA transcript that is later processed by the ribonuclease activity of Cpf1 to produce three mature small RNAs capable of directing Cpf1 endonuclease cleavage. Two transcriptional cassettes were designed containing the AsLIGULELESS-1, AsMS45-1 and AsMS26-1 maize targeting sequences described in Example 2 flanked on both sides by a 35 bp sequence (SEQ ID NO: 27) (Figures 6 and 7) that upon RNA transcription would be cleaved by Cpf1 into guide RNAs capable of directing Cpf1 chromosomal DNA target site cleavage. The first guide RNA transcriptional cassette incorporated a U6 polymerase III promoter (SEQ ID NO: 6) and terminator (TTTTTTTT) operably fused to the ends of the multiple targeting sequences (Figure 6) resulting in SEQ ID NO: 28. The second guide RNA transcriptional cassette incorporated a maize ubiquitin polymerase II promoter (SEQ ID NO: 12), ubiquitin 5' untranslated region (UTR) (SEQ ID NO: 13), ubiquitin intron 1 (SEQ ID NO: 14) and suitable terminator operably fused to the ends of the multiple targeting sequences (Figure 7) resulting in SEQ ID NO: 29.

Each guide RNA transcriptional cassette was separately co-delivered into maize immature embryos (IMEs) with the maize optimized *Acidaminococcus* (As) Cpf1 endonuclease expression cassette (described in Example 1) by biolistic transformation as described in Example 2. Transformed IMEs were incubated at 37°C for 48 hrs., immediately following transformation as described in Example 2. After temperature treatment, the 20-30 most evenly transformed IMEs were harvested based on their fluorescence and total genomic DNA was extracted. PCR and deep sequencing were performed as described in Example 2 and the resulting reads examined for the presence of mutations at the expected site of cleavage by comparison to control experiments where the small RNA transcriptional cassette was omitted from the transformation as described in Karvelis *et al.* (2015).

Surprisingly, all three sites targeted for cleavage with the AsCpf1 endonuclease and multiplexed guide polynucleotide system described herein exhibited insertion and deletion (indel) mutations indicative of chromosomal cleavage and imprecise DNA repair relative to the negative control (Table 9). Additionally, both polymerase II and III transcriptional constructs permitted targeting of all three maize chromosomal target sites.

Taken together, these data indicate that the Cpf1 endonuclease and polymerase II and III multiplexed guide polynucleotide systems described herein may be used to simultaneously target multiple maize chromosomal DNA target sites.

**Table 9. Frequency of mutations at AsMS26-1, AsLIGULELESS-1 and AsMS45-1 maize sites when guide RNAs are transcribed in tandem from either a single polymerase II or III promoter.**

| Particle Gun Transformation Experiment | Maize Target(s) Assayed for Mutations | Guide RNA transcriptional cassette | Average Mutation Frequency |
|---|---|---|---|
| *Acidaminococcus* Cpf1 Only (Negative Control) | AsMS26-1 (SEQ ID NO: 7), AsLIGULELESS-1(SEQ ID NO: 8), AsMS45-1 (SEQ ID NO: 9) | None | 0.00% |
| *Acidaminococcus* Cpf1 and Multiplex Guide RNA | AsMS26-1 (SEQ ID NO: 7) | U6 (pol III) | 0.11% |
| | | multiplex | |
| *Acidaminococcus* Cpf1 and Multiplex Guide RNA | AsLIGULELESS-1(SEQ ID NO: 8) | U6 (pol III) | 0.29% |
| | | multiplex | |
| *Acidaminococcus* Cpf1 and Multiplex Guide RNA | AsMS45-1 (SEQ ID NO: 9) | U6 (pol III) | 0.23% |
| | | multiplex | |
| *Acidaminococcus* Cpf1 and Multiplex Guide RNA | AsMS26-1 (SEQ ID NO: 7) | Ubiquitin (pol II) | 0.09% |
| | | multiplex | |
| *Acidaminococcus* Cpf1 and Multiplex Guide RNA | AsLIGULELESS-1(SEQ ID NO: 8) | Ubiquitin (pol II) | 0.35% |
| | | multiplex | |
| *Acidaminococcus* Cpf1 and Multiplex Guide RNA | AsMS45-1 (SEQ ID NO: 9) | Ubiquitin (pol II) | 0.59% |
| | | multiplex | |

### EXAMPLE 6

### Temperature regulatable guide polynucleotide Cpf1 system

The observed temperature induction of the guide polynucleotide Cpf1 systems described herein may be utilized to regulate the cleavage activity of the Cpf1 endonuclease. In one aspect, this can provide a transient pulse of Cpf1 endonuclease activity from plasmid DNA expression cassettes which would reduce the potential for off-target cleavage activity. This is in contrast with other methods which deliver genome-editing reagents transiently into cells as ribonucleoprotein (RNP) complexes to reduce off-target cleavage activity (Svitashev, S. et al. (2016) Nature Communications. 7: 13274). Additionally, the temperature induction of guide RNA/Cpf1 activity from plasmid DNA expression cassettes could be utilized to time cleavage activity with the desired cellular, tissue culture or plant developmental stages long after it has been introduced into the cell which is not possible by RNP delivery. The guide polynucleotide Cpf1 system described herein may also be combined with inducible (e.g. heat shock, chemical or cell cycle) or tissue specific promoters to provide additional control over Cpf1 endonuclease activity.

### EXAMPLE 7

### DNA RNA hybrid guide polynucleotides direct Cpf1 endonuclease cleavage at maize chromosomal DNA target sites

This example illustrates the method by which hybrid DNA RNA (hDRNA) guide polynucleotides (also referred to as RNA-DNA guide polynucleotides) are capable of complexing with Cpf1 and directing endonuclease cleavage at maize chromosomal DNA target sites.

First, hDRNA designs were devised. As shown in Figure 8, DNA nucleotides were incorporated into both the Cpf1 recognition and variable targeting domains. In the Cpf1 recognition domain, DNA nucleotides were incorporated into either the 5' end or the loop structure while in the variable targeting domain DNA nucleotides were introduced at the 3' end (Figure 8). In total, 4 hDRNAs, were designed targeting the AsLIGULELESS-1 target sequence and synthesized (Integrated DNA Technologies, USA). The four hDRNAs included the hybrid DNA-RNA guide polynucleotide 1,
[T]AAUUUCUACUCUUGUAGAUCUCUUAGCUCAUCACCUAUGUGG (SEQ ID NO:44), the hybrid DNA-RNA guide polynucleotide 2,
UAAUUUCUACU[C]UUGUAGAUCUCUUAGCUCAUCACCUAUGUGG (SEQ ID NO:45), the hybrid DNA-RNA Guide Polynucleotide 3,
UAAUUUCUACUC[T]UGUAGAUCUCUUAGCUCAUCACCUAUGUGG, and the DNA-RNA guide polynucleotide 4,
UAAUUUCUACUCUUGUAGAUCUCUUAGCUCAUCACCUAUGUG[G].

Next, the ability of the hDRNAs to form a complex with Cpf1 and direct maize chromosomal DNA cleavage was assessed by co-delivering 35 ng of the synthesized hDRNAs with the Cpf1 DNA expression cassette, visual selectable marker and cell cycle stimulating genes, BBM (ZmODP2 (US Publ. No. 20050257289)) and WUS2 (ZmWUS2 (U.S. Pat. No. 7,256,322)) by particle gun transformation into immature maize embyros (IMEs) as described in Example 2 and Svitashev *et al.* (2015). As a positive control, the maize optimized *S. pyogenes* (Spy) Cas9 DNA expression cassette (as described in Svitashev *et al.* 2015) was co-delivered with 75 ng of an in vitro T7 transcribed (MegaScript T7 transcription kit (ThermoFisher Scientific, USA)) Spy single guide RNA (sgRNA) (SEQ ID NO: 48) targeting the sequence, GGCCGAGGTCGACTACCGGCCGG (SpyMS45-2 (SEQ ID NO: 49)), into IMEs. Transformation experiments were performed in duplicate, tests assembled without guide polynucleotides served as negative controls and IMEs were incubated at 37°C following transformation. Two days after transformation, total genomic DNA was extracted from the transformed maize embryos and PCR amplification of the target sites was performed. The resulting amplicons were then subjected to Illumina deep sequencing to determine the frequency of small insertion or deletion (indel) mutations indicative of maize chromosomal DNA target site cleavage and imperfect repair as described in Example 2.

All four hDRNAs produced indel mutations at the expected site of cleavage for the AsLIGULELESS-1 target site. Additionally, the frequency of mutations was near that of the positive control utilizing a *S*. *pyogenes* Cas9 DNA expression cassette and T7 transcribed sgRNA (Table 10). Taken together, these data indicate that the Cpf1 endonuclease and hDRNA guide polynucleotide systems described herein are capable of forming a functional complex that may recognize and cleave maize chromosomal DNA target sites and be utilized as a tool to bind, nick, or cleave plant chromosomal DNA for the purposes of plant genomic and gene manipulation.

**Table 10. Frequency of indels induced by Cpf1 hDRNA guide polynucleotide Cas9 complexes at maize chromosomal DNA target sites compared with a Spy Cas9 guide RNA complex.**

| Particle Gun Transformation Experiment | Maize Target(s) Assayed for Mutations | Average Mutation Frequency |
|---|---|---|
| *Acidaminococcus* Cpf1 Only (Negative Control) | AsLIGULELESS-1(SEQ ID NO: 8) | 0.00% |
| *Acidaminococcus* Cpf1 and hDRNA1 (SEQ ID NO: 44) | AsLIGULELESS-1(SEQ ID NO: 8) | 0.03% |
| *Acidaminococcus* Cpf1 and hDRNA2 (SEQ ID NO: 45) | AsLIGULELESS-1(SEQ ID NO: 8) | 0.02% |
| *Acidaminococcus* Cpf1 and hDRNA3 (SEQ ID NO: 46) | AsLIGULELESS-1(SEQ ID NO: 8) | 0.01% |
| *Acidaminococcus* Cpf1 and hDRNA4 (SEQ ID NO: 47) | AsLIGULELESS-1(SEQ ID NO: 8) | 0.03% |
| *S. pyogenes* Cas9 Only (Negative Control) | SpyMS45-2 (SEQ ID NO: 49) | 0.00% |
| *S. pyogenes* Cas9 and guide RNA (SEQ ID NO: 48) | SpyMS45-2 (SEQ ID NO: 49) | 0.05% |

### EXAMPLE 8

### Transformation of Maize Immature Embryos

Transformation can be accomplished by various methods known to be effective in plants, including particle-mediated delivery, *Agrobacterium-mediated* transformation, PEG-mediated delivery, and electroporation.

### a. Particle-mediated delivery

Transformation of maize immature embryos using particle delivery is performed as follows. Media recipes follow below.

The ears are husked and surface sterilized in 30% Clorox bleach plus 0.5% Micro detergent for 20 minutes, and rinsed two times with sterile water. The immature embryos are isolated and placed embryo axis side down (scutellum side up), 25 embryos per plate, on 560Y medium for 4 hours and then aligned within the 2.5-cm target zone in preparation for bombardment. Alternatively, isolated embryos are placed on 560L (Initiation medium) and placed in the dark at temperatures ranging from 26°C to 37°C for 8 to 24 hours prior to placing on 560Y for 4 hours at 26°C prior to bombardment as described above.

Plasmids containing the double strand brake inducing agent and donor DNA are constructed using standard molecular biology techniques and co-bombarded with plasmids containing the developmental genes ODP2 (AP2 domain transcription factor ODP2 (Ovule development protein 2); US20090328252 A1) and Wushel (US2011/0167516).

The plasmids and DNA of interest are precipitated onto 0.6 µm (average diameter) gold pellets using a water-soluble cationic lipid transfection reagent as follows. DNA solution is prepared on ice using 1 µg of plasmid DNA and optionally other constructs for co-bombardment such as 50 ng (0.5 µl) of each plasmid containing the developmental genes ODP2 (AP2 domain transcription factor ODP2 (Ovule development protein 2); US20090328252 A1) and Wushel. To the pre-mixed DNA, 20 µl of prepared gold particles (15 mg/ml) and 5 µl of a water-soluble cationic lipid transfection reagent is added in water and mixed carefully. Gold particles are pelleted in a microfuge at 10,000 rpm for 1 min and supernatant is removed. The resulting pellet is carefully rinsed with 100 ml of 100% EtOH without resuspending the pellet and the EtOH rinse is carefully removed. 105 µl of 100% EtOH is added and the particles are resuspended by brief sonication. Then, 10 µl is spotted onto the center of each macrocarrier and allowed to dry about 2 minutes before bombardment.

Alternatively, the plasmids and DNA of interest are precipitated onto 1.1 µm (average diameter) tungsten pellets using a calcium chloride (CaCl₂) precipitation procedure by mixing 100 µl prepared tungsten particles in water, 10 µl (1 µg) DNA in Tris EDTA buffer (1 µg total DNA), 100 µl 2.5 M CaC12, and 10 µl 0.1 M spermidine. Each reagent is added sequentially to the tungsten particle suspension, with mixing. The final mixture is sonicated briefly and allowed to incubate under constant vortexing for 10 minutes. After the precipitation period, the tubes are centrifuged briefly, liquid is removed, and the particles are washed with 500 ml 100% ethanol, followed by a 30 second centrifugation. Again, the liquid is removed, and 105 µl of 100% ethanol is added to the final tungsten particle pellet. For particle gun bombardment, the tungsten/DNA particles are briefly sonicated. 10 µl of the tungsten/DNA particles is spotted onto the center of each macrocarrier, after which the spotted particles are allowed to dry about 2 minutes before bombardment.

The sample plates are bombarded at level #4 with a Biorad Helium Gun. All samples receive a single shot at 450 PSI, with a total of ten aliquots taken from each tube of prepared particles/DNA.

Following bombardment, the embryos are incubated on 560P (maintenance medium) for 12 to 48 hours at temperatures ranging from 26C to 37C, and then placed at 26C. After 5 to 7 days the embryos are transferred to 560R selection medium containing 3 mg/liter Bialaphos, and subcultured every 2 weeks at 26C. After approximately 10 weeks of selection, selection-resistant callus clones are transferred to 288J medium to initiate plant regeneration. Following somatic embryo maturation (2-4 weeks), well-developed somatic embryos are transferred to medium for germination and transferred to a lighted culture room. Approximately 7-10 days later, developing plantlets are transferred to 272V hormone-free medium in tubes for 7-10 days until plantlets are well established. Plants are then transferred to inserts in flats (equivalent to a 2.5" pot) containing potting soil and grown for 1 week in a growth chamber, subsequently grown an additional 1-2 weeks in the greenhouse, then transferred to Classic 600 pots (1.6 gallon) and grown to maturity. Plants are monitored and scored for transformation efficiency, and/or modification of regenerative capabilities.

Initiation medium (560L) comprises 4.0 g/l N6 basal salts (SIGMA C-1416), 1.0 ml/l Eriksson's Vitamin Mix (1000X SIGMA-1511), 0.5 mg/l thiamine HCI, 20.0 g/l sucrose, 1.0 mg/l 2,4-D, and 2.88 g/l L-proline (brought to volume with D-I H2O following adjustment to pH 5.8 with KOH); 2.0 g/l Gelrite (added after bringing to volume with D-I H2O); and 8.5 mg/l silver nitrate (added after sterilizing the medium and cooling to room temperature).

Maintenance medium (560P) comprises 4.0 g/l N6 basal salts (SIGMA C-1416), 1.0 ml/I Eriksson's Vitamin Mix (1000X SIGMA-1511), 0.5 mg/l thiamine HCI, 30.0 g/l sucrose, 2.0 mg/l 2,4-D, and 0.69 g/l L-proline (brought to volume with D-I H2O following adjustment to pH 5.8 with KOH); 3.0 g/l Gelrite (added after bringing to volume with D-I H2O); and 0.85 mg/l silver nitrate (added after sterilizing the medium and cooling to room temperature).

Bombardment medium (560Y) comprises 4.0 g/l N6 basal salts (SIGMA C-1416), 1.0 ml/I Eriksson's Vitamin Mix (1000X SIGMA-1511), 0.5 mg/l thiamine HCI, 120.0 g/l sucrose, 1.0 mg/l 2,4-D, and 2.88 g/l L-proline (brought to volume with D-I H2O following adjustment to pH 5.8 with KOH); 2.0 g/l Gelrite (added after bringing to volume with D-I H2O); and 8.5 mg/l silver nitrate (added after sterilizing the medium and cooling to room temperature).
Selection medium (560R) comprises 4.0 g/l N6 basal salts (SIGMA C-1416), 1.0 ml/l Eriksson's Vitamin Mix (1000X SIGMA-1511), 0.5 mg/l thiamine HCI, 30.0 g/l sucrose, and 2.0 mg/l 2,4-D (brought to volume with D-I H2O following adjustment to pH 5.8 with KOH); 3.0 g/l Gelrite (added after bringing to volume with D-I H2O); and 0.85 mg/l silver nitrate and 3.0 mg/l bialaphos (both added after sterilizing the medium and cooling to room temperature).

Plant regeneration medium (288J) comprises 4.3 g/l MS salts (GIBCO 11117-074), 5.0 ml/l MS vitamins stock solution (0.100 g nicotinic acid, 0.02 g/l thiamine HCL, 0.10 g/l pyridoxine HCL, and 0.40 g/l glycine brought to volume with polished D-I H2O) (Murashige and Skoog (1962) Physiol. Plant. 15:473), 100 mg/l myo-inositol, 0.5 mg/l zeatin, 60 g/l sucrose, and 1.0 ml/l of 0.1 mM abscisic acid (brought to volume with polished D-I H2O after adjusting to pH 5.6); 3.0 g/l Gelrite (added after bringing to volume with D-I H2O); and 1.0 mg/l indoleacetic acid and 3.0 mg/l bialaphos (added after sterilizing the medium and cooling to 60°C). Hormone-free medium (272V) comprises 4.3 g/l MS salts (GIBCO 11117-074), 5.0 ml/l MS vitamins stock solution (0.100 g/l nicotinic acid, 0.02 g/l thiamine HCL, 0.10 g/l pyridoxine HCL, and 0.40 g/l glycine brought to volume with polished D-I H2O), 0.1 g/l myo-inositol, and 40.0 g/l sucrose (brought to volume with polished D-I H2O after adjusting pH to 5.6); and 6 g/l bacto-agar (added after bringing to volume with polished D-I H2O), sterilized and cooled to 60°C.

### b. Agrobacterium-mediated transformation

*Agrobacterium-mediated* transformation was performed essentially as described in Djukanovic et al. (2006) Plant Biotech J 4:345-57. Briefly, 10-12 day old immature embryos (0.8 -2.5 mm in size) were dissected from sterilized kernels and placed into liquid medium (4.0 g/L N6 Basal Salts (Sigma C-1416), 1.0 ml/L Eriksson's Vitamin Mix (Sigma E-1511), 1.0 mg/L thiamine HCI, 1.5 mg/L 2, 4-D, 0.690 g/L L-proline, 68.5 g/L sucrose, 36.0 g/L glucose, pH 5.2). After embryo collection, the medium was replaced with 1 ml *Agrobacterium* at a concentration of 0.35-0.45 OD550. Maize embryos were incubated with *Agrobacterium* for 5 min at room temperature, then the mixture was poured onto a media plate containing 4.0 g/L N6 Basal Salts (Sigma C-1416), 1.0 ml/L Eriksson's Vitamin Mix (Sigma E-1511), 1.0 mg/L thiamine HCI, 1.5 mg/L 2, 4-D, 0.690 g/L L-proline, 30.0 g/L sucrose, 0.85 mg/L silver nitrate, 0.1 nM acetosyringone, and 3.0 g/L Gelrite, pH 5.8. Embryos were incubated axis down, in the dark for 3 days at 20°C, then incubated 4 days in the dark at 28°C, then transferred onto new media plates containing 4.0 g/L N6 Basal Salts (Sigma C-1416), 1.0 ml/L Eriksson's Vitamin Mix (Sigma E-1511), 1.0 mg/L thiamine HCI, 1.5 mg/L 2, 4-D, 0.69 g/L L-proline, 30.0 g/L sucrose, 0.5 g/L MES buffer, 0.85 mg/L silver nitrate, 3.0 mg/L Bialaphos, 100 mg/L carbenicillin, and 6.0 g/L agar, pH 5.8. Embryos were subcultured every three weeks until transgenic events were identified. Somatic embryogenesis was induced by transferring a small amount of tissue onto regeneration medium (4.3 g/L MS salts (Gibco 11117), 5.0 ml/L MS Vitamins Stock Solution, 100 mg/L myo-inositol, 0.1 µM ABA, 1 mg/L IAA, 0.5 mg/L zeatin, 60.0 g/L sucrose, 1.5 mg/L Bialaphos, 100 mg/L carbenicillin, 3.0 g/L Gelrite, pH 5.6) and incubation in the dark for two weeks at 28°C. All material with visible shoots and roots were transferred onto media containing 4.3 g/L MS salts (Gibco 11117), 5.0 ml/L MS Vitamins Stock Solution, 100 mg/L myo-inositol, 40.0 g/L sucrose, 1.5 g/L Gelrite, pH 5.6, and incubated under artificial light at 28°C. One week later, plantlets were moved into glass tubes containing the same medium and grown until they were sampled and/or transplanted into soil.

### EXAMPLE 9

### Transient Expression of BBM Enhances Transformation

Parameters of the transformation protocol can be modified to ensure that the BBM activity is transient. One such method involves precipitating the BBM-containing plasmid in a manner that allows for transcription and expression, but precludes subsequent release of the DNA, for example, by using the chemical PEI. In one example, the BBM plasmid is precipitated onto gold particles with PEI, while the transgenic expression cassette (UBI::moPAT~GFPm::PinII; moPAT is the maize optimized PAT gene) to be integrated is precipitated onto gold particles using the standard calcium chloride method.

Briefly, gold particles were coated with PEI as follows. First, the gold particles were washed. Thirty-five mg of gold particles, 1.0 in average diameter (A.S.I. #162-0010), were weighed out in a microcentrifuge tube, and 1.2 ml absolute EtOH was added and vortexed for one minute. The tube was incubated for 15 minutes at room temperature and then centrifuged at high speed using a microfuge for 15 minutes at 4oC. The supernatant was discarded and a fresh 1.2 ml aliquot of ethanol (EtOH) was added, vortexed for one minute, centrifuged for one minute, and the supernatant again discarded (this is repeated twice). A fresh 1.2 ml aliquot of EtOH was added, and this suspension (gold particles in EtOH) was stored at -20oC for weeks. To coat particles with polyethylimine (PEI; Sigma #P3143), 250 µl of the washed gold particle/EtOH mix was centrifuged and the EtOH discarded. The particles were washed once in 100 µl ddH2O to remove residual ethanol, 250 µl of 0.25 mM PEI was added, followed by a pulse-sonication to suspend the particles and then the tube was plunged into a dry ice/EtOH bath to flash-freeze the suspension, which was then lyophilized overnight. At this point, dry, coated particles could be stored at -80oC for at least 3 weeks. Before use, the particles were rinsed 3 times with 250 µl aliquots of 2.5 mM HEPES buffer, pH 7.1, with 1x pulse-sonication, and then a quick vortex before each centrifugation. The particles were then suspended in a final volume of 250 µl HEPES buffer. A 25 µl aliquot of the particles was added to fresh tubes before attaching DNA. To attach uncoated DNA, the particles were pulse-sonicated, then 1 µg of DNA (in 5 µl water) was added, followed by mixing by pipetting up and down a few times with a Pipetteman and incubated for 10 minutes. The particles were spun briefly (i.e. 10 seconds), the supernatant removed, and 60 µl EtOH added. The particles with PEI-precipitated DNA-1 were washed twice in 60 µl of EtOH. The particles were centrifuged, the supernatant discarded, and the particles were resuspended in 45 µl water. To attach the second DNA (DNA-2), precipitation using a water-soluble cationic lipid transfection reagent was used. The 45 µl of particles/DNA-1 suspension was briefly sonicated, and then 5 µl of 100 ng/µl of DNA-2 and 2.5 µl of the water-soluble cationic lipid transfection reagent were added. The solution was placed on a rotary shaker for 10 minutes, centrifuged at 10,000g for 1 minute. The supernatant was removed, and the particles resuspended in 60 µl of EtOH. The solution was spotted onto macrocarriers and the gold particles onto which DNA-1 and DNA-2 had been sequentially attached were delivered into scutellar cells of 10 DAP Hi-II immature embryos using a standard protocol for the PDS-1000. For this experiment, the DNA-1 plasmid contained a UBI::RFP::pinII expression cassette, and DNA-2 contained a UBI::CFP::pinII expression cassette. Two days after bombardment, transient expression of both the CFP and RFP fluorescent markers was observed as numerous red & blue cells on the surface of the immature embryo. The embryos were then placed on non-selective culture medium and allowed to grow for 3 weeks before scoring for stable colonies. After this 3-week period, 10 multicellular, stably-expressing blue colonies were observed, in comparison to only one red colony. This demonstrated that PEI-precipitation could be used to effectively introduce DNA for transient expression while dramatically reducing integration of the PEI-introduced DNA and thus reducing the recovery of RFP-expressing transgenic events. In this manner, PEI-precipitation can be used to deliver transient expression of BBM and/or WUS2.

For example, the particles are first coated with UBI::BBM::pinII using PEI, then coated with UBI::moPAT~YFP using a water-soluble cationic lipid transfection reagent, and then bombarded into scutellar cells on the surface of immature embryos. PEI-mediated precipitation results in a high frequency of transiently expressing cells on the surface of the immature embryo and extremely low frequencies of recovery of stable transformants Thus, it is expected that the PEI-precipitated BBM cassette expresses transiently and stimulates a burst of embryogenic growth on the bombarded surface of the tissue (i.e. the scutellar surface), but this plasmid will not integrate. The PAT~GFP plasmid released from the Ca++/gold particles is expected to integrate and express the selectable marker at a frequency that results in substantially improved recovery of transgenic events. As a control treatment, PEI-precipitated particles containing a UBI::GUS::pinII (instead of BBM) are mixed with the PAT~GFP/Ca++ particles. Immature embryos from both treatments are moved onto culture medium containing 3mg/l bialaphos. After 6-8 weeks, it is expected that GFP+, bialaphos-resistant calli will be observed in the PEI/BBM treatment at a much higher frequency relative to the control treatment (PEI/GUS).

As an alternative method, the BBM plasmid is precipitated onto gold particles with PEI, and then introduced into scutellar cells on the surface of immature embryos, and subsequent transient expression of the BBM gene elicits a rapid proliferation of embryogenic growth. During this period of induced growth, the explants are treated with Agrobacterium using standard methods for maize (see Example 1), with T-DNA delivery into the cell introducing a transgenic expression cassette such as UBI::moPAT~GFPm::pinII. After co-cultivation, explants are allowed to recover on normal culture medium, and then are moved onto culture medium containing 3 mg/l bialaphos. After 6-8 weeks, it is expected that GFP+, bialaphos-resistant calli will be observed in the PEI/BBM treatment at a much higher frequency relative to the control treatment (PEI/GUS).

It may be desirable to "kick start" callus growth by transiently expressing the BBM and/or WUS2 polynucleotide products. This can be done by delivering BBM and WUS2 5'-capped polyadenylated RNA, expression cassettes containing BBM and WUS2 DNA, or BBM and/or WUS2 proteins. All of these molecules can be delivered using a biolistics particle gun. For example 5'-capped polyadenylated BBM and/or WUS2 RNA can easily be made in vitro using Ambion's mMessage mMachine kit. RNA is co-delivered along with DNA containing a polynucleotide of interest and a marker used for selection/screening such as Ubi::moPAT~GFPm::PinII. It is expected that the cells receiving the RNA will immediately begin dividing more rapidly and a large portion of these will have integrated the agronomic gene. These events can further be validated as being transgenic clonal colonies because they will also express the PAT~GFP fusion protein (and thus will display green fluorescence under appropriate illumination). Plants regenerated from these embryos can then be screened for the presence of the polynucleotide of interest.

## Claims

1. A method for modifying a target sequence in the genome of a plant cell, the method comprising:
a) introducing into a plant cell a Cpf1 endonuclease protein or a plant-optimized polynucleotide encoding said Cpf1 endonuclease protein, and a guide polynucleotide comprising a variable targeting domain that is substantially complementary to a target sequence in the plant genome or a recombinant DNA expressing said guide polynucleotide; and
b) incubating said plant cell at a temperature of 28°C to 37°C for a period of at least about 4 hrs.;
wherein said guide polynucleotide and Cpf1 endonuclease are capable of forming a complex that can recognize, bind to, and optionally nick or cleave said target sequence;
wherein said method does not comprise an essentially biological process for the production of plants.

2. The method of claim 1, wherein the plant-optimized polynucleotide is a plant-optimized mRNA encoding said Cpf1 endonuclease protein or a recombinant DNA construct comprising a promoter operably linked to a plant-optimized polynucleotide encoding said Cpf1 endonuclease protein.

3. The method of claim 1, further comprising identifying at least one plant cell that has a modification at said target sequence, wherein the modification at said target sequence is selected from the group consisting of (i) a replacement of at least one nucleotide, (ii) a deletion of at least one nucleotide, (iii) an insertion of at least one nucleotide, and (iv) any combination of (i) - (iii).

4. The method of claim 3, wherein said modification at said target sequence occurs at an increased frequency when compared to a control method, wherein the plant cell of b) is incubated at a typical plant tissue culture temperature of 28°C.

5. The method of claim 4, wherein the increased frequency when compared to said control method is increased by at least 2-fold, at least 3-fold, at least 4-fold, at least 5-fold, at least 6-fold, at least 7-fold, at least 8-fold, at least 9-fold, at least 10-fold, at least 10-fold, at least 11-fold , at least 12-fold, at least 13-fold, at least 14-fold, at least 15-fold, at least 30-fold, at least 40-fold, or at least 50-fold.

6. The method of claim 1, further comprising introducing a donor DNA to the plant cell, wherein said donor DNA comprises a polynucleotide of interest.

7. The method of claim 6, further comprising identifying at least one plant cell comprising in its genome the polynucleotide of interest integrated into or near said target sequence.

8. A method for editing a nucleotide sequence in the genome of a plant cell, the method comprising;
a) introducing into a plant cell a Cpf1 endonuclease protein or a plant-optimized polynucleotide encoding said Cpf1 endonuclease protein, a guide polynucleotide comprising a variable targeting domain that is substantially complementary to a target sequence in the plant genome, and a polynucleotide modification template,
wherein said polynucleotide modification template comprises at least one nucleotide modification of said nucleotide sequence; and,
b) incubating said plant cell at a temperature of 28°C to 37°C for a period of at least about 4 hrs.;
wherein said guide polynucleotide and Cpf1 endonuclease protein are capable of forming a complex that can recognize, bind to, and optionally nick or cleave all or part of said target sequence;
wherein said method does not comprise an essentially biological process for the production of plants.

9. The method of claim 8, further comprising identifying at least one plant cell comprising in its genome said at least one nucleotide modification of said nucleotide sequence.

10. The method of claim 1 or 8, wherein said guide polynucleotide is selected from the group consisting of a RNA polynucleotide, a DNA polynucleotide, or a RNA-DNA polynucleotide.

11. The method of claim 1, wherein the plant cell is a monocot plant cell or a dicot plant cell.

12. The method of claim 11, wherein the plant cell is selected from the group consisting of maize, rice, sorghum, rye, barley, wheat, millet, oats, sugarcane, turfgrass, or switchgrass, soybean, canola, alfalfa, sunflower, cotton, tobacco, peanut, potato, tobacco, Arabidopsis, and safflower cell.

13. A method of simultaneously modifying multiple target sequences in the genome of a plant cell, the method comprising:
a) introducing into said plant cell a Cpf1 endonuclease protein, or a plant-optimized polynucleotide encoding said Cpf1 endonuclease protein, and a precursor guide RNA transcriptional initiation cassette capable of expressing a single precursor RNA that is processed into a multitude of single guide RNAs, wherein each single guide RNA comprises a variable targeting domain 3' of a Protospacer Adjacent Motif (PAM), wherein said variable targeting domain is complementary to a single target sequence in the plant genome; and
b) incubating the plant cell of (a) at a temperature of 28°C to 37°C for a period of at least about 4 hrs.;
wherein each of said single guide RNA and said Cpf1 endonuclease protein is capable of forming a ribonucleotide complex that can recognize, bind to, and optionally nick or cleave a target sequence;
wherein said method does not comprise an essentially biological process for the production of plants.

14. The method of claim 13, wherein:
a) the multiple target sequences consist of at least 2, 3, 4, 5, 6, 7, 8, 9, 10, 11, 12, 13, 14, 15, 16, 17, 18, 19, 20 target sequences;
b) the precursor guide RNA transcriptional initiation cassette comprises a Pol-II promoter operably linked to the precursor guide RNA; or
c) the precursor guide RNA transcriptional initiation cassette comprises a Pol-III promoter operably linked to the precursor guide RNA.

15. A method for modifying a DNA target sequence in the genome of a plant cell, the method comprising:
a) introducing into a plant cell a Cpf1 endonuclease protein or a plant-optimized polynucleotide encoding said Cpf1 endonuclease protein, and a guide polynucleotide or a recombinant DNA expressing said guide polynucleotide, capable of forming a Cpf1 complex with the Cpf1 endonuclease protein, the guide polynucleotide comprising a variable targeting domain that is substantially complementary to a target sequence in the plant genome; and
b) introducing a polynucleotide modification template comprising at least one region that corresponds to a DNA target sequence adjacent to a PAM sequence recognized by the Cpf1 complex,
wherein said method further comprises incubating said plant cell at a temperature of 28°C to 37°C for a period of at least about 4 hrs.;
further wherein said Cpf1 complex can recognize, bind to, and optionally nick or cleave said target sequence;
wherein said method does not comprise an essentially biological process for the production of plants.

## Patentansprüche

1. Verfahren zur Modifizierung einer Zielsequenz im Genom einer Pflanzenzelle, wobei das Verfahren Folgendes umfasst:
a) Einführen eines Cpf1-Endonuklease-Proteins oder eines pflanzenoptimierten Polynukleotids, das das Cpf1-Endonuklease-Protein codiert, sowie eines Guide-Polynukleotids, das eine variable Targeting-Domäne umfasst, die weitgehend komplementär zu einer Zielsequenz im Pflanzengenom ist, oder einer rekombinanten DNA, die das Guide-Polynukleotid exprimiert, in eine Pflanzenzelle; und
b) Inkubieren der Pflanzenzelle bei einer Temperatur von 28 °C bis 37 °C über einen Zeitraum von mindestens etwa 4 Std.;
wobei das Guide-Polynukleotid und die Cpf1-Endonuklease in der Lage sind, einen Komplex zu bilden, der die Zielsequenz erkennen, daran binden und gegebenenfalls einschneiden oder spalten kann;
wobei das Verfahren kein im Wesentlichen biologisches Verfahren zur Züchtung von Pflanzen umfasst.

2. Verfahren nach Anspruch 1, wobei es sich bei dem pflanzenoptimierten Polynukleotid um eine pflanzenoptimierte mRNA, die das Cpf1-Endonuklease-Protein codiert, oder ein rekombinantes DNA-Konstrukt, das einen Promotor in operativer Verknüpfung mit einem das Cpf1-Endonuklease-Protein codierenden pflanzenoptimierten Polynukleotid umfasst, handelt.

3. Verfahren nach Anspruch 1, ferner umfassend Identifizieren mindestens einer Pflanzenzelle, die eine Modifikation an der Zielsequenz aufweist, wobei die Modifikation an der Zielsequenz aus der Gruppe bestehend aus (i) einer Ersetzung mindestens eines Nukleotids, (ii) einer Deletion mindestens eines Nukleotids, (iii) einer Insertion mindestens eines Nukleotids und (iv) einer beliebigen Kombination von (i) - (iii) ausgewählt ist.

4. Verfahren nach Anspruch 3, wobei die Modifikation an der Zielsequenz mit einer erhöhten Häufigkeit im Vergleich zu einem Kontrollverfahren erfolgt, wobei die Pflanzenzelle unter b) bei einer typischen Pflanzengewebekulturtemperatur von 28 °C inkubiert wird.

5. Verfahren nach Anspruch 4, wobei die erhöhte Häufigkeit im Vergleich zu dem Kontrollverfahren mindestens 2-fach, mindestens 3-fach, mindestens 4-fach, mindestens 5-fach, mindestens 6-fach, mindestens 7-fach, mindestens 8-fach, mindestens 9-fach, mindestens 10-fach, mindestens 10-fach, mindestens 11-fach, mindestens 12-fach, mindestens 13-fach, mindestens 14-fach, mindestens 15-fach, mindestens 30-fach, mindestens 40-fach oder mindestens 50-fach erhöht ist.

6. Verfahren nach Anspruch 1, ferner umfassend Einführen einer Spender-DNA in die Pflanzenzelle, wobei die Spender-DNA ein Polynukleotid von Interesse umfasst.

7. Verfahren nach Anspruch 6, ferner umfassend Identifizieren mindestens einer Pflanzenzelle, die in ihrem Genom das Polynukleotid von Interesse integriert in oder nahe der Zielsequenz umfasst.

8. Verfahren zum Editing einer Nukleotidsequenz im Genom einer Pflanzenzelle, wobei das Verfahren Folgendes umfasst:
a) Einführen eines Cpf1-Endonuklease-Proteins oder eines pflanzenoptimierten Polynukleotids, das das Cpf1-Endonuklease-Protein codiert, sowie eines Guide-Polynukleotids, das eine variable Targeting-Domäne umfasst, die weitgehend komplementär zu einer Zielsequenz im Pflanzengenom ist, und einer Polynukleotidmodifikationsmatrize in eine Pflanzenzelle, wobei die Polynukleotidmodifikationsmatrize mindestens eine Nukleotidmodifikation der Nukleotidsequenz umfasst; und
b) Inkubieren der Pflanzenzelle bei einer Temperatur von 28 °C bis 37 °C über einen Zeitraum von mindestens etwa 4 Std.;
wobei das Guide-Polynukleotid und das Cpf1-Endonuklease-Protein in der Lage sind, einen Komplex zu bilden, der die gesamte oder einen Teil der Zielsequenz erkennen, daran binden und gegebenenfalls einschneiden oder spalten kann;
wobei das Verfahren kein im Wesentlichen biologisches Verfahren zur Züchtung von Pflanzen umfasst.

9. Verfahren nach Anspruch 8, ferner umfassend Identifizieren mindestens einer Pflanzenzelle, die in ihrem Genom die mindestens eine Nukleotidmodifikation der Nukleotidsequenz umfasst.

10. Verfahren nach Anspruch 1 oder 8, wobei das Guide-Polynukleotid aus der Gruppe bestehend aus einem RNA-Polynukleotid, einem DNA-Polynukleotid oder einem RNA-DNA-Polynukleotid ausgewählt ist.

11. Verfahren nach Anspruch 1, wobei es sich bei der Pflanzenzelle um eine Monokotylen-Pflanzenzelle oder eine Dikotylen-Pflanzenzelle handelt.

12. Verfahren nach Anspruch 11, wobei die Pflanzenzelle aus der Gruppe bestehend aus Zellen von Mais, Reis, Sorghum, Roggen, Gerste, Weizen, Hirse, Hafer, Zuckerrohr, Rasengras oder Rutenhirse, Sojabohne, Raps, Luzerne, Sonnenblume, Baumwolle, Tabak, Erdnuss, Kartoffel, Tabak, Arabidopsis und Färberdistel ausgewählt ist.

13. Verfahren zur gleichzeitigen Modifizierung mehrerer Zielsequenzen im Genom einer Pflanzenzelle, wobei das Verfahren Folgendes umfasst:
a) Einführen eines Cpf1-Endonuklease-Proteins oder eines pflanzenoptimierten Polynukleotids, das das Cpf1-Endonuklease-Protein codiert, sowie einer Vorläufer-Guide-RNA-Transkriptionsinitiationskassette, die in der Lage ist, eine einzelne Vorläufer-RNA zu exprimieren, die zu einer Vielzahl einzelner Guide-RNAs prozessiert wird, in die Pflanzenzelle, wobei jede einzelne Guide-RNA eine variable Targeting-Domäne 3' von einem PAM (Protospacer Adjacent Motif) umfasst, wobei die variable Targeting-Domäne komplementär zu einer einzelnen Zielsequenz im Pflanzengenom ist; und
b) Inkubieren der Pflanzenzelle unter (a) bei einer Temperatur von 28 °C bis 37 °C über einen Zeitraum von mindestens etwa 4 Std.;
wobei die einzelnen Guide-RNAs und das Cpf1-Endonuklease-Protein jeweils in der Lage sind, einen Ribonukleotid-Komplex zu bilden, der eine Zielsequenz erkennen, daran binden und gegebenenfalls einschneiden oder spalten kann; wobei das Verfahren kein im Wesentlichen biologisches Verfahren zur Züchtung von Pflanzen umfasst.

14. Verfahren nach Anspruch 13, wobei:
a) die mehreren Zielsequenzen aus mindestens 2, 3, 4, 5, 6, 7, 8, 9, 10, 11, 12, 13, 14, 15, 16, 17, 18, 19, 20 Zielsequenzen bestehen;
b) die Vorläufer-Guide-RNA-Transkriptionsinitiationskassette einen Pol-II-Promotor in operativer Verknüpfung mit der Vorläufer-Guide-RNA umfasst; oder
c) die Vorläufer-Guide-RNA-Transkriptionsinitiationskassette einen Pol-III-Promotor in operativer Verknüpfung mit der Vorläufer-Guide-RNA umfasst.

15. Verfahren zur Modifizierung einer DNA-Zielsequenz im Genom einer Pflanzenzelle, wobei das Verfahren Folgendes umfasst:
a) Einführen eines Cpf1-Endonuklease-Proteins oder eines pflanzenoptimierten Polynukleotids, das das Cpf1-Endonuklease-Protein codiert, sowie eines Guide-Polynukleotids oder einer rekombinanten DNA, die das Guide-Polynukleotid exprimiert, mit der Fähigkeit zur Bildung eines Cpf1-Komplexes mit dem Cpf1-Endonuklease-Protein, wobei das Guide-Polynukleotid eine variable Targeting-Domäne umfasst, die weitgehend komplementär zu einer Zielsequenz im Pflanzengenom ist, in eine Pflanzenzelle; und
b) Einführen einer Polynukleotidmodifikationsmatrize, die mindestens eine Region, die einer DNA-Zielsequenz entspricht, unmittelbar neben einer PAM-Sequenz, die vom Cpf1-Komplex erkannt wird, umfasst,
wobei das Verfahren ferner Inkubieren der Pflanzenzelle bei einer Temperatur von 28 °C bis 37 °C über einen Zeitraum von mindestens etwa 4 Std. umfasst;
weiter wobei der Cpf1-Komplex die Zielsequenz erkennen, daran binden und gegebenenfalls einschneiden oder spalten kann;
wobei das Verfahren kein im Wesentlichen biologisches Verfahren zur Züchtung von Pflanzen umfasst.

## Revendications

1. Procédé de modification d'une séquence cible dans le génome d'une cellule végétale, le procédé comprenant :
a) l'introduction dans une cellule végétale d'une protéine endonucléase Cpf1 ou d'un polynucléotide optimisé pour l'expression dans les plantes codant pour ladite protéine endonucléase Cpf1, et un polynucléotide guide comprenant un domaine de ciblage variable qui est sensiblement complémentaire d'une séquence cible dans le génome végétal ou un ADN recombinant exprimant ledit polynucléotide guide ; et
b) l'incubation de ladite cellule végétale à une température de 28 °C à 37 °C pendant une durée d'au moins environ 4 heures ;
dans lequel ledit polynucléotide guide et ladite endonucléase Cpf1 peuvent former un complexe qui peut reconnaître ou se lier à ladite séquence cible et facultativement couper ou cliver celle-ci ;
ledit procédé ne comprenant pas un processus essentiellement biologique pour la production de plantes.

2. Procédé selon la revendication 1, dans lequel le polynucléotide optimisé pour l'expression dans les plantes est un ARNm optimisé pour l'expression dans les plantes codant pour ladite protéine endonucléase Cpf1 ou une construction d'ADN recombinant comprenant un promoteur lié fonctionnellement à un polynucléotide optimisé pour l'expression dans les plantes codant pour ladite protéine endonucléase Cpf1.

3. Procédé selon la revendication 1, comprenant en outre l'identification d'au moins une cellule végétale qui présente une modification au niveau de ladite séquence cible, dans lequel la modification au niveau de ladite séquence cible est choisie dans le groupe constitué par (i) un remplacement d'au moins un nucléotide, (ii) une délétion d'au moins un nucléotide, (iii) une insertion d'au moins un nucléotide, et (iv) une combinaison quelconque de (i) à (iii).

4. Procédé selon la revendication 3, dans lequel ladite modification au niveau de ladite séquence cible est présente à une fréquence accrue par rapport à un procédé témoin, dans lequel la cellule végétale de b) est incubée à une température typique de culture de tissu végétal de 28°°C.

5. Procédé selon la revendication 4, dans lequel la fréquence accrue par rapport audit procédé témoin est augmentée d'au moins 2 fois, d'au moins 3 fois, au moins 4 fois, au moins 5 fois, au moins 6 fois, au moins 7 fois, au moins 8 fois, au moins 9 fois, au moins 10 fois, au moins 10 fois, au moins 11 fois, au moins 12 fois, au moins 13 fois, au moins 14 fois, au moins 15 fois, au moins 30 fois, au moins 40 fois ou au moins 50 fois.

6. Procédé selon la revendication 1, comprenant en outre l'introduction d'un ADN donneur dans la cellule végétale, dans lequel ledit ADN donneur comprend un polynucléotide d'intérêt.

7. Procédé selon la revendication 6, comprenant en outre l'identification d'au moins une cellule végétale comprenant dans son génome le polynucléotide d'intérêt intégré dans ou à proximité de ladite séquence cible.

8. Procédé d'édition d'une séquence nucléotidique dans le génome d'une cellule végétale, le procédé comprenant :
a) l'introduction dans une cellule végétale d'une protéine endonucléase Cpf1 ou d'un polynucléotide optimisé pour l'expression dans les plantes codant pour ladite protéine endonucléase Cpf1, un polynucléotide guide comprenant un domaine de ciblage variable qui est sensiblement complémentaire d'une séquence cible dans le génome végétal, et une matrice de modification de polynucléotide,
dans lequel ladite matrice de modification de polynucléotide comprend au moins une modification nucléotidique de ladite séquence nucléotidique ; et,
b) l'incubation de ladite cellule végétale à une température de 28 °C à 37 °C pendant une durée d'au moins environ 4 heures ;
dans lequel ledit polynucléotide guide et ladite protéine endonucléase Cpf1 peuvent former un complexe qui peut reconnaître ou se lier à ladite séquence cible et facultativement couper ou cliver celle-ci ;
ledit procédé ne comprenant pas un processus essentiellement biologique pour la production de plantes.

9. Procédé selon la revendication 8, comprenant en outre l'identification d'au moins une cellule végétale comprenant dans son génome ladite ou lesdites modifications nucléotidiques de ladite séquence nucléotidique.

10. Procédé selon la revendication 1 ou 8, dans lequel ledit polynucléotide guide est choisi dans le groupe constitué par un polynucléotide ARN, un polynucléotide ADN ou un polynucléotide ARN-ADN.

11. Procédé selon la revendication 1, dans lequel la cellule végétale est une cellule végétale monocotylédone ou une cellule végétale dicotylédone.

12. Procédé selon la revendication 11, dans lequel la cellule végétale est choisie dans le groupe constitué par une cellule de maïs, riz, sorgho, seigle, orge, blé, millet, avoine, canne à sucre, gazon, ou panic érigé, soja, canola, luzerne, tournesol, coton, tabac, arachide, pomme de terre, tabac, Arabidopsis et carthame.

13. Procédé de modification simultanée de séquences cibles multiples dans le génome d'une cellule végétale, le procédé comprenant :
a) l'introduction dans ladite cellule végétale d'une protéine endonucléase Cpf1, ou d'un polynucléotide optimisé pour l'expression dans les plantes codant pour ladite protéine endonucléase Cpf1, et d'une cassette d'initiation de transcription d'ARN guide précurseur pouvant exprimer un ARN précurseur unique qui est transformé en une pluralité d'ARN guides uniques, dans lequel chaque ARN guide unique comprend un domaine de ciblage variable en 3' d'un motif adjacent au protoespaceur (PAM), dans lequel ledit domaine de ciblage variable est complémentaire d'une séquence cible unique dans le génome végétal ; et
b) l'incubation de ladite cellule végétale de (a) à une température de 28 °C à 37 °C pendant une durée d'au moins environ 4 heures ;
dans lequel chacun dudit ARN guide unique et de ladite protéine endonucléase Cpf1 peut former un complexe ribonucléotidique qui peut reconnaître ou se lier à ladite séquence cible et facultativement couper ou cliver celle-ci ;
ledit procédé ne comprenant pas un processus essentiellement biologique pour la production de plantes.

14. Procédé selon la revendication 13, dans lequel :
a) les séquences cibles multiples comprennent 2, 3, 4, 5, 6, 7, 8, 9, 10, 11, 12, 13, 14, 15, 16, 17, 18, 19, 20 séquences cibles ;
b) la cassette d'initiation de transcription d'ARN guide précurseur comprend un promoteur Pol-II lié fonctionnellement à l'ARN guide précurseur ; ou
c) la cassette d'initiation de transcription d'ARN guide précurseur comprend un promoteur Pol-III lié fonctionnellement à l'ARN guide précurseur.

15. Procédé de modification d'une séquence cible d'ADN dans le génome d'une cellule végétale, le procédé comprenant :
a) l'introduction dans une cellule végétale d'une protéine endonucléase Cpf1 ou d'un polynucléotide optimisé pour l'expression dans les plantes codant pour ladite protéine endonucléase Cpf1, et d'un polynucléotide guide ou d'un ADN recombinant exprimant ledit polynucléotide guide, pouvant former un complexe Cpf1 avec la protéine endonucléase Cpf1, le polynucléotide guide comprenant un domaine de ciblage variable qui est sensiblement complémentaire d'une séquence cible dans le génome végétal ; et
b) l'introduction d'une matrice de modification de polynucléotide comprenant au moins une région qui correspond à une séquence cible d'ADN adjacente à une séquence PAM reconnue par le complexe Cpf1,
ledit procédé comprenant en outre l'incubation de ladite cellule végétale à une température de 28 °C à 37 °C pendant une durée d'au moins environ 4 heures ;
en outre, dans lequel ledit complexe Cpf1 peut reconnaître ou se lier à ladite séquence cible et facultativement couper ou cliver celle-ci ;
ledit procédé ne comprenant pas un processus essentiellement biologique pour la production de plantes.
